# EUROPEAN PATENT SPECIFICATION

(11) **EP 0 668 781 B1**
(45) Date of publication and mention of the grant of the patent: **29.12.1999**
(21) Application number: 92922731.2
(22) Date of filing: 23.10.1992
(51) Int. Cl.: A61K 48/00, A61K 35/12, A61K 39/00, C12N 15/19, C12N 15/24, C12N 15/25, C12N 15/26, C12N 15/90, C12N 15/63

(54) **LYMPHOKINE GENE THERAPY OF CANCER IN COMBINATION WITH TUMOR ANTIGENS**
LYMPHOKINE GENTHERAPIE BEI KREBS IN KOMBINATION MIT TUMORANTIGENEN
THERAPIE GENIQUE DU CANCER PAR LES CYTOKINES EN COMBINAISON AVEC UN ANTIGENE TUMORAL

(30) Priority: 25.10.1991 US 781356; 03.04.1992 US 863641
(43) Date of publication of application: 30.08.1995
(73) Proprietor: SIDNEY KIMMEL CANCER CENTER, San Diego, CA 92121 (US)
(72) Inventor: SOBOL, Robert, E., La Jolla, CA 92037 (US); FRED, H., Gage, La Jolla, CA 92037 (US); ROYSTON, Ivor, La Jolla, CA 92037 (US); FRIEDMAN, Theodore, La Jolla, CA 92037 (US); FAKHRAI, Habib, Oceanside, CA 92056 (US)
(74) Representative: UEXKÜLL & STOLBERG
(86) International application number: US9208999
(87) International publication number: WO9307906

(56) References cited:
- WO-A-92/05262
- IMMUNOLOGY LETTERS, vol. 23,no. 4, February 1990 pages 287-292, BUBENIK J. ET AL.
- IMMUNOLOGY TODAY, vol. 11,no. 6, 1990 pages 196-200, RUSSEL S.J.
- JOURNAL OF CLINICAL ONCOLOGY, vol. 10,no. 2, February 1992 pages 180-199, ROSENBERG S.A.
- Journal of Experimental Medicine, Volume 172, issued October 1990, GANSBACHER et al., "Interleukin 2 Gene Transfer into Tumor Cells Abrogates Tumorigenicity and Induces Protective Immunity", pages 1217-1224, see the entire document.
- Cell, Volume 57, issued 05 May 1989, TEPPER et al., "Murine Interleukin-4 Displays Potent Anti-Tumor Activity in Vivo", pages 503-512, see the entire document.
- Cell, Volume 60, issued 09 February 1990, FEARON et al., "Interleukin-2 Production by Tumor Cells Bypasses T Helper Function in the Generation of an Antitumor Response", pages 397-403, see the entire document.
- Cancer Research, Volume 50, issued 15 August 1990, OGURA et al., "Implantation of Genetically Manipulated Fibroblasts into Mice as Antitumor Alpha-Interferon Therapy", pages 5102-5106, see the entire document.
- Cancer Research, Volume 50, issued 15 December 1990, GANSBACHER et al., "Retroviral Vector-Mediated Interferon Gene Transfer into Tumor Cells Generates Potent and Long Lasting Antitumor Immunity", pages 7820-7825, see the entire document.

## Description

Recent advances in our understanding of the biology of the immune system have lead to the identification of important modulators of immune responses, called cytokines (1-3). Immune system modulators produced by lymphocytes are termed lymphokines, a subset of the cytokines. These agents mediate many of the immune responses involved in anti-tumor immunity. Several of these cytokines have been produced by recombinant DNA methodology and evaluated for their anti-tumor effects. The administration of lymphokines and related immunomodulators has resulted in objective tumor responses in patients with various types of neoplasms (4-7). However, current modes of cytokine administration are frequently associated with toxicities that limit the therapeutic value of these agents.

For example, interleukin-2 (IL-2) is an important lymphokine in the generation of anti-tumor immunity (4). In response to tumor antigens, a subset of lymphocytes termed helper T-cells secrete small quantities of IL-2. This IL-2 acts locally at the site of tumor antigen stimulation to activate cytotoxic T-cells and natural killer cells which mediate systemic tumor cell destruction. Intravenous, intralymphatic and intralesional administration of IL-2 has resulted in clinically significant responses in some cancer patients (4-6). However, severe toxicities (hypotension and adema) limit the dose and efficacy of intravenous and intralymphatic IL-2 administration (5-7). The toxicity of systemically administered lymphokines is not surprising as these agents mediate local cellular interactions and they are normally secreted in only very small quantities.

Additionally, other cytokines, such as interleukin-4 (IL-4), alpha interferon (α-INF) and gamma interferon (γ-INF) have been used to stimulate immune responses to tumor cells. Like IL-2, the current modes of administration have adverse side effects.

To circumvent the toxicity of systemic cytokine administration, several investigators have examined intralesional injection of IL-2. This approach eliminates the toxicity associated with systemic IL-2 administration (8,9,10). However, multiple intralesional injections are required to optimize therapeutic efficacy (9,10). Hence, these injections are impractical for many patients, particularly when tumor sites are not accessible for injection without potential morbidity.

An alternative approach, involving cytokine gene transfer into tumor cells, has resulted in significant anti-tumor immune responses in several animal tumor models (11-14). In these studies, the expression of cytokine gene products following cytokine gene transfer into tumor cells has abrogated the tumorigenicity of the cytokine-secreting tumor cells when implanted into syngeneic hosts. The transfer of genes for IL-2 (11,12) γ-INF (13) or interleukin-4 (IL-4) (14) significantly reduced or eliminated the growth of several different histological types of murine tumors. In the studies employing IL-2 gene transfer, the treated animals also developed systemic anti-tumor immunity and were protected against subsequent tumor challenges with the unmodified parental tumor (11,12). Similar inhibition of tumor growth and protective immunity was also demonstrated when immunizations were performed with a mixture of unmodified parental tumor cells and genetically modified tumor cells engineered to express the IL-2 gene. No toxicity associates with localized lymphokine transgene expression was reported in these animal tumor studies (11-14).

While the above gene-transfer procedure has been shown to provide anti-tumor immunity, it still retains practical difficulties. This approach is limited by the inability to transfer functional cytokine genes into many patients' tumor cells, as most patients' tumors cannot be established to grown in vitro and methods for human in vivo gene transfer are not available.

### SUMMARY OF THE INVENTION

The present invention demonstrates a novel, more practical method of cytokine cancer immunotherapy. In one approach, selected cells from a patient, such as fibroblasts, obtained, for example, from a routine skin biopsy, are genetically modified to express one or more cytokines. Alternatively, patient cells which may normally serve as antigen presenting cells in the immune system such as macrophages, monocytes, and lymphocytes may also be genetically modified to express one or more cytokines. These modified cells are hereafter called cytokine-expressing cells, or CE cells. The CE cells are then mixed with the patient's tumor antigens, for example in the form of irradiated tumor cells, or alternatively in the form of purified natural or recombinant tumor antigen, and employed in immunizations, for example subcutaneously, to induce systemic anti-tumor immunity.

The cytokines are locally expressed at levels sufficient to induce or augment systemic anti-tumor immune responses via local immunization at sites other than active tumor sites. Systemic toxicity related to cytokine administration should not occur because the levels of cytokine secreted by the CE cells should not significantly affect systemic cytokine concentrations.

As the amount of cytokine secreted by the CE cells is sufficient to induce anti-tumor immunity but is too low to produce substantial systemic toxicity, this approach provides the benefit of local cytokine administration. In addition, this novel method obviates the need for intralesional injections, which may produce morbidity. Furthermore, the continuous local expression of cytokine(s) at the sites of immunization may also augment anti-tumor immune responses compared to intermittent cytokine injections. This method also provides the advantage of local immunization with the CE cells, as opposed to cumbersome intravenous infusions. This method also eliminates the need for establishing tumor cell lines in vitro as well as transfer of genes into these tumor cells.

This invention also provides an alternative means of localized expression of cytokines to induce and/or increase immune responses to a patient's tumor through genetic modification of cellular expression of both cytokine(s) and tumor antigen(s). In this embodiment, selected cells from a patient are isolated and transduced with cytokine gene(s) as well as gene(s) coding for tumor antigen(s). The transduced cells are called "carrier cells." Carrier cells can include fibroblasts and cells which may normally serve as antigen presenting cells in the immune system such as macrophages, monocytes, and lymphocytes. Transduced carrier cells actively expressing both the cytokine(s) and the tumor antigen(s) are selected and utilized in local immunizations at a site other than active tumor sites to induce anti-tumor immune responses. As with the CE cells, these carrier cells should not produce substantial systemic toxicities, as the levels of cytokine(s) secreted by the carrier cells should not significantly affect systemic cytokine concentrations. This alternate embodiment is advantageous because it obviates the need to obtain samples of the tumor, which is sometimes difficult. However, carrier cells can be utilized in local immunizations in conjunction with tumor cells, tumor cell homogenates, purified tumor antigens, or recombinant tumor antigens to enhance anti-tumor immunity.

Additionally, this second embodiment retains the same advantages as the first embodiment in that the level of cytokine released by the carrier cells is sufficient to induce anti-tumor immunity but is too low to produce substantial systemic toxicity. In addition, as with the first embodiment, this method obviates the need for intralesional injections, and allows for continuous expression of cytokine(s). This method also eliminates the need for establishing continuous cultures in vitro of tumor cells as well as transfer of genes into these tumor cells, and provides the advantage of local immunization with the carrier cells, as opposed to cumbersome lengthy intravenous infusions.

These approaches may also find application in inducing or augmenting immune responses to other antigens of clinical significance in other areas of medical practice.

### BRIEF DESCRIPTION OF THE DRAWINGS

Figure 1 shows schematic diagrams of retroviral vectors DC/TKIL2, LXSN-IL2, and LNCX-IL2.
Figure 2 shows a mean IL-2 concentration of triplicate supernatant samples measured by ELISA. Supernatants were harvested from overnight cultures of approximately 1.5 x 10⁶ semi-confluent fibroblasts.
Figure 3 shows biological activity of the IL-2 secreted by the transduced fibroblasts was demonstrated by measuring mean ³H-TdR incorporation of an IL-2 dependent T-cell line incubated with triplicate samples of supernatants. Supernatants were harvested from overnight cultures of approximately 1.5 x 10⁶ semi-confluent fibroblasts.
Figure 4 shows comparisons between animals injected with 10⁵ CT26 tumor cells alone (□); 10⁵ CT26 tumor cells and 2 x 10⁶ unmodified BALB/C fibroblasts (■); 10⁵ CT26 tumor cells and 2 x 10⁶ IL-2 transduced BALB/C fibroblasts (●); and 10⁵ CT26 tumor cells and 1 x 10⁶ transduced BALB/C fibroblasts (○). Tumor measurements are the mean products of the cross-sectional diameter of the tumors from four animals in each treatment group. The (*) indicates statistically significant difference (P < 0.05) in tumor growth curves.

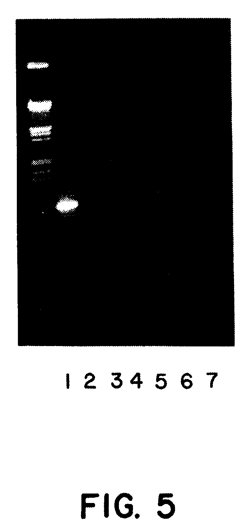

Figure 5 shows PCR analysis of neomycin phosphotransferase DNA sequences. Lane 1 - positive control pLXSN-RI-IL2. Lanes 2 through 4 tests genomic DNA; Lanes 5 and 6 ovary genomic DNA; Lane 7 negative control, no DNA. Identical results were obtained with liver, spleen and lung genomic DNA (data not shown).
Figure 6 shows the effect of IL-2 modified fibroblasts on tumor establishment and development using 2 x 10⁶ fibroblasts mixed with 5 x 10⁴ CT26 tumor cells concentrating on the rate of tumor growth.
Figure 7 shows the effect of IL-2 modified fibroblasts on tumor establishment and development using 2 x 10⁶ fibroblasts mixed with 5 x 10⁴ CT26 tumor cells concentrating on the time of tumor onset for the individual animal in each treatment group.
Figure 8 shows the effect of IL-2 modified fibroblasts on tumor establishment and development using 2 x 10⁶ fibroblasts mixed with 1 x 10⁵ CT26 tumor cells concentrating on the rate of tumor growth.
Figure 9 shows the effect of IL-2 modified fibroblasts on tumor establishment and development using 2 x 10⁶ fibroblasts mixed with 1 x 10⁵ CT26 tumor cells concentrating on the time of tumor onset for the individual animal in each treatment group.
Figure 10 shows the effect of IL-2 modified cells on tumor establishment and development using 2 x 10⁶ DCTK-IL2-modified CT26 tumor cells mixed with 1 x 10⁵ unmodified CT26 compared to 2 x 10⁶ DCTK-IL2-modified fibroblasts mixed with 1 x 10⁵ CT26 concentrating on the rate of tumor growth.
Figure 11 shows the effect of IL-2 modified cells on tumor establishment and development using 2 x 10⁶ DCTK-IL2-modified CT26 tumor cells mixed with 1 x 10⁵ unmodified CT26 compared to 2 x 10⁶ DCTK-IL2-modified fibroblasts mixed with 1 x 10⁵ CT26 concentrating on the time of tumor onset for the individual animal in each treatment group.
Figure 12 shows the effect of IL-2 modified fibroblasts on induction of systemic anti-tumor immunity and the rate of tumor growth. Mice were immunized with 2 x 10⁶ fibroblasts mixed with 2.5 x 10⁵ irradiated CT26 tumor cells 7 days prior to challenge with 5 x 10⁴ fresh tumor cells.
Figure 13 shows the effect of IL-2 modified fibroblasts on induction of systemic anti-tumor immunity and the time of tumor onset for the individual animal in each treatment group. Mice were immunized with 2 x 10⁶ fibroblasts mixed with 2.5 x 10⁵ irradiated CT26 tumor cells 7 days prior to challenge with 5 x 10⁴ fresh tumor cells.
Figure 14 shows the effect of IL-2 modified fibroblasts on induction of systemic anti-tumor immunity and the rate of tumor growth. Mice were immunized with 2 x 10⁶ fibroblasts mixed with 2.5 x 10⁵ irradiated CT26 tumor cells 14 days prior to challenge with 5 x 10⁴ fresh tumor cells.
Figure 15 shows the effect of IL-2 modified fibroblasts on induction of systemic anti-tumor immunity and the time of tumor onset for the individual animal in each treatment group. Mice were immunized with 2 x 10⁶ fibroblasts mixed with 2.5 x 10⁵ irradiated CT26 tumor cells 14 days prior to challenge with 5 x 10⁴ fresh tumor cells.

### DETAILED DESCRIPTION

A novel method of tumor immunotherapy is described comprising the genetic modification of cells resulting in the secretion of cytokine gene products to stimulate a patient's immune response to tumor antigens. "Gene" is defined herein to be a nucleotide sequence encoding the desired protein. In one embodiment, autologous fibroblasts genetically modified to secrete at least one cytokine gene product are utilized to immunize the patient in a formulation with tumor antigens at a site other than an active tumor site. In another embodiment, cells genetically modified to express at least one tumor antigen gene product and to secrete at least one cytokine gene product are utilized in formulation to immunize the patient at a site other than an active tumor site. Cytokines are preferably expressed in cells which efficiently secrete these proteins into the surrounding milieu. Fibroblasts are an example of such cells. Fibroblasts or other cells can be genetically modified to express and secrete one or more cytokines, as described later in this specification.

Tumor antigens can be provided by several methods, including, but not limited to the following: 1) CE cells can be transduced with gene(s) coding for tumor antigens. These "carrier cells" are then utilized in patient immunizations. 2) Cloned gene sequences coding for appropriate tumor antigens can be transferred into cells such as fibroblasts or antigen-presenting cells. These cells are then mixed with CE or carrier cells to immunize the patient. 3) Tumor antigens can be cloned in bacteria or other types of cells by recombinant procedures. These antigens are then purified and employed an immunization with CE and/or carrier cells. 4) Tumor antigens can be purified from tumor cells and used, along with CE or carrier cells, to immunize the patient. 5) Tumor cells may be irradiated or mechanically disrupted and mixed with CE and/or carrier cells for patient immunizations.

This invention encompasses the following steps:
(A) isolation of appropriate cells for generation of CE cells or carrier cells; (B) isolation of cytokine genes or isolation of cytokine genes and tumor antigen genes, as well as appropriate marker and/or suicide genes; (C) transfer of the genes from (B) to produce the CE cells or carrier cells; (D) preparation of immunological samples of the patient's tumor antigens or other suitable tumor antigens for immunization with CE or carrier cells; (E) inactivation of the malignant potential of tumor cells if they are used as a source of tumor antigens for immunization; and (F) preparation of samples for immunization. Following are several embodiments contemplated by the inventors. However, it is understood that any means known by those in the art to accomplish these steps will be usable in this invention.

### (A) Isolation of Cells to Generate CE and Carrier Cells

Cells to be utilized as CE cells and carrier cells can be selected from a variety of locations in the patient's body. For example, skin punch biopsies provide a readily available source of fibroblasts for use in generating CE cells, with a minimal amount of intrusion to the patient. alternatively, these fibroblasts can be obtained from the tumor sample itself. Cells of hematopoietic origin may be obtained by venipuncture, bone marrow aspiration, lymph node biopsies, or from tumor samples. Other appropriate cells for the generation of CE or carrier cells can be isolated by means known in the art. Non-autologous cells similarly selected and processed can also be used.

### (B) Isolation of Genes

Numerous cytokine genes have been cloned and are available for use in this protocol. The genes for IL-2, γ-INF and other cytokines are readily available (1-5, 11-14). Cloned genes of the appropriate tumor antigens are isolated according to means known in the art.

Selectable marker genes such as neomycin resistance (Neo^{R}) are readily available. Incorporation of a selectable marker gene(s) allows for the selection of cells that have successfully received and express the desired genes. Other selectable markers known to those in the art of gene transfer may also be utilized to generate CE cells or carrier cells expressing the desired transgenes.

"Suicide" genes can be incorporated into the CE cells or carrier cells to allow for selective inducible killing after stimulation of the immune response. A gene such as the herpes simplex virus thymidine kinase gene (TK) can be used to create an inducible destruction of the CE cells or carrier cells. When the CE cells or carrier cells are no longer useful, a drug such as acyclovir or gancyclovir can be administered. Either of these drugs will selectively kill cells expressing TK, thus eliminating the implanted transduced cells. Additionally, a suicide gene may be a gene coding for a non-secreted cytotoxic polypeptide attached to an inducible promoter. When destruction of the CE or carrier cells is desired, the appropriate inducer of the promoter is administered so that the suicide gene is induced to produce cytotoxic polypeptide which subsequently kills the CE or carrier cell. However, destruction of the CE or carrier cells may not be required.

Genes coding for tumor antigen(s) of interest can be cloned by recombinant methods. The coding sequence of an antigen expressed by multiple tumors may be utilized for many individual patients.

### (C) Transfer of Genes

Numerous methods are available for transferring genes into cultured cells (15). For example, the appropriate genes can be inserted into vectors such as plasmids or retroviruses and transferred into the cells. Electroporation, lipofection and a variety of other methods are known in the field and can be implemented.

One method for gene transfer is a method similar to that employed in previous human gene transfer studies, where tumor infiltrating lymphocytes (TILs) were modified by retroviral gene transduction and administered to cancer patients (16). In this Phase I safety study of retroviral mediated gene transfer, TILs were genetically modified to express the Neomycin resistance (Neo^{R}) gene. Following intravenous infusion, polymerase chain reaction analyses consistently found genetically modified cells in the circulation for as long as two months after administration. No infectious retroviruses were identified in these patients and no side effects due to gene transfer were noted in any patients (16). These retroviral vectors have been altered to prevent viral replication by the deletion of viral gag, pol and env genes.

When retroviruses are used for gene transfer, replication competent retroviruses may theoretically develop by recombination between the retroviral vector and viral gene sequences in the packaging cell line utilized to produce the retroviral vector. We will use packaging cell lines in which the production of replication competent virus by recombination has been reduced or eliminated. Hence, all retroviral vector supernatants used to infect patient cells will be screened for replication competent virus by standard assays such as PCR and reverse transcriptase assays (16). Furthermore, exposure to replication competent virus may not be harmful. In studies of subhuman primates injected with a large inoculum of replication competent murine retrovirus, the retrovirus was cleared by the primate immune system (17). No clinical illnesses or sequelae resulting from replication competent virus have been observed three years after exposure. In summary, it is not expected that patients will be exposed to replication competent murine retrovirus and it appears that such exposure may not be deleterious (17).

### (D) Preparation of Immunological Samples of the Patient's Tumor Antigens or Purified Recombinant Tumor Antigens

Tumor cells bearing tumor associated antigens are isolated from the patient. These cells can derive either from solid tumors or from leukemic tumors. For solid tumors, single-cell suspensions can be made by mechanical separation and washing of biopsy tissue (18).

Hematopoietic tumors may be isolated from peripheral blood or bone marrow by standard methods (19).

A second variant is the use of homogenates of tumor cells. Such homogenates would contain tumor antigens available for recognition by the patient's immune system upon stimulation by this invention. Either unfractionated cell homogenates, made, for example, by mechanical disruption or by freezing and thawing the cells, or fractions of homogenates preferably with concentrated levels of tumor antigens, can be used.

Likewise, purified tumor antigens, obtained for example by immunoprecipitation or recombinant DNA methods, could be used. Purified antigens would then be utilized for immunizations together with the CE cells and/or carrier cells described above to induce or enhance the patient's immune response to these antigens.

In the embodiments employing carrier cells, tumor antigens are available through their expression by the carrier cells. These carrier cells can be injected alone or in conjunction with other tumor antigen preparations or CE cells. Likewise, when CE cells are used, purified recombinant tumor antigen, produced by methods known in the art, can be used.

If autologous tumor cells are not readily available, heterologous tumor cells, their homogenates, their purified antigens, or carrier cells expressing such antigens could be used.

### (E) Inactivation of Tumor Cells

When viable tumor cells are utilized in immunizations as a source of tumor antigens, the tumor cells can be inactivated so that they do not grow in the patient. Inactivation can be accomplished by several methods. the cells can be irradiated prior to immunization (18). This irradiation will be at a level which will prevent their replication. Such viable cells can then present their tumor antigens to the patient's immune system, but cannot multiply to create new tumors.

Alternatively, tumor cells that can be cultured may be transduced with a suicide gene. As described above, a gene such as the herpes simplex thymidine kinase (TK) gene can be transferred into tumor cells to induce their destruction by administration of acyclovir or gancyclovir. After immunization, the TK expressing tumor cells can present their tumor antigens, and are capable of proliferation. After a period of time during which the patients's immune response is stimulated, the cells can be selectively killed. This approach might allow longer viability of the tumor cells utilized for immunizations, which may be advantageous in the induction or augmentation of anti-tumor immunity.

### (F) Preparation of Samples for Immunization

CE cells and/or carrier cells and tumor cells, and/or homogenates of tumor cells and/or purified tumor antigen(s), are combined for patient immunization. Approximately 10⁷ tumor cells will be required. If homogenates of tumor cells or purified or non-purified fractions of tumor antigens are used, the tumor dose can be adjusted based on the normal number of tumor antigens usually present on 10⁷ intact tumor cells. The tumor preparation should be mixed with numbers of CE or carrier cells sufficient to secrete cytokine levels that induce anti-tumor immunity (11-12) without producing substantial systemic toxicity which would interfere with therapy.

The cytokines should be produced by the CE cells or the carrier cells at levels sufficient to induce or augment immune response but low enough to avoid substantial systemic toxicity. This prevents side effects created by previous methods' administration of greater than physiological levels of the cytokines.

These mixtures, as well as carrier cells that are utilized alone, will be formulated for injection in any manner known in the art acceptable for immunization. Because it is important that at least the CE cells and carrier cells remain viable, the formulations must be compatible with cell survival. Formulations can be injected subcutaneously, intramuscularly, or in any manner acceptable for immunization.

Contaminants in the preparation which may focus the immune response on undesired antigens should be removed prior to the immunizations.

The following examples are provided for illustration of several embodiments of the invention and should not be interpreted as limiting the scope of the invention.

### EXAMPLE I

### IMMUNIZATION WITH FIBROBLASTS EXPRESSING IL-2 MIXED WITH IRRADIATED TUMOR CELLS

### 1) Isolation of Autologous Fibroblasts for Use in Generating IL-2 Secreting CE Cells

Skin punch biopsies will be obtained from each patient under sterile conditions. The biopsy tissue will be minced and placed in RPMI 1640 media containing 10% fetal calf serum (or similar media) to establish growth of the skin fibroblasts in culture. The cultured fibroblasts will be utilized to generate IL-2 secreting CE cells by retroviral mediated IL-2 gene transfer.

### 2) Retroviral Vector Preparation and Generation of IL-2 Secreting CE Cells

The cultured skin fibroblasts will then be infected with a retroviral vector containing the IL-2 and Neomycin resistance (Neo^{R}) genes. An N2 vector containing the Neo^{R} gene will be used, and has been previously utilized by a number of investigators for in vitro and in vivo work, including investigations with human subjects (16). The IL-2 vector will be generated from an N2-derived vector, LLRNL, developed and described by Friedmann and his colleagues (20). It will be made by replacement of the luciferase gene of LLRNL with a full-length cDNA encoding human IL-2. Retroviral vector free of contaminating replication-competent virus is produced by transfection of vector plasmid constructions into the helper-free packaging cell line PA317. Before infection of patients' cells, the vector will have been shown to be free of helper virus. In the event that helper virus is detected, the vector will be produced in the GP + envAM12 packaging cell line in which the viral gag and pol genes are separated from the env, further reducing the likelihood of helper virus production.

### 3) Transduction Protocol

The cultured primary fibroblasts will be incubated with supernatant from the packaging cell line as described (20). Supernatant from these cells will be tested for adventitious agents and replication competent virus as described (16) and outlined in Table 1. The fibroblasts are washed and then grown in culture media containing G418, (a neomycin analogue) to select for transduced cells expressing the Neo^{R} gene. The G418-resistant cells will be tested for expression of the IL-2 gene by measuring the concentration of IL-2 in the culture supernatant by an enzyme linked immunosorbent assay (ELISA) (12). G418-resilient cells expressing IL-2 will be stored at -70°C until required for subsequent use in immunizations.

**Table 1**

| Adventitious Agents and Safety Testing | |
|---|---|
| 1. | Sterility |
| 2. | Mycoplasma |
| 3. | General Safety |
| 4. | Viral Testing |
| | LCM Virus |
| | Thymic agent |
| | S+/L- eco |
| | S+/L-xeno |
| | S+/L- ampho |
| | 3T3 amplification |
| | MRC-5/Vero |

### 4) Preparation of Irradiated Tumor Cells

Tumors obtained form clinically indicated surgical resections or from superficial lymph node or skin metastases will be minced into 2-3 mm pieces and treated with collagenase and DNAse to facilitate separation of the tumor into a single cell suspension. The collected cells will be centrifuged and washed in RPMI 1640 media and then cryopreserved in a solution containing 10% dimethyl sulphoxide and 50% fetal calf serum in RPMI 1640 media. The cells will be stored in liquid nitrogen until the time of administration. Prior to their use in subcutaneous immunizations, the cells will be thawed, washed in media free of immunogenic contaminants, and irradiated with 4,000 rads per minute for a total of 20,000 rads in a cesium irradiator.

### 5) Patient Selection

Patients will have a histologically confirmed diagnosis of cancer. Patients with tumors that must be resected for therapeutic purposes or with tumors readily accessible for biopsy are most appropriate for this embodiment of the invention.

### 6) Pretreatment Evaluation

The following pretreatment evaluations will be performed:
1) History and physical examination including a description and quantification of disease activity.
2) Performance Status Assessment
   - 0 =: Normal, no symptoms
   - 1 =: Restricted, but ambulatory
   - 2 =: Up greater than 50% of waking hours, capable of self-care
   - 3 =: Greater than 50% of waking hours confined to bed or chair, limited self-care
   - 4 =: Bedridden
3) Pretreatment Laboratory;
   CBC with differential, platelet count, PT, PTT, glucose, BUN, creatinine, electrolytes, SGOT, SGPT, LDH, alkaline phosphatase, bilirubin, uric acid, calcium, total protein albumin.
4) Other Analyses:
   Urinalysis
   CH₅₀, C₃ and C₄ serum complement levels Immunophenotyping of peripheral blood B cell and T cell subsets
   Assays for detectable replication-competent virus in peripheral blood cells
   PCR assays of peripheral blood leukocytes for Neo^{R}, IL-2 and viral env
5) Other Pretreatment Evaluation:
   Chest X-ray and other diagnostic studies including computerized tomography (CT), magnetic resonance imaging (MRI) or radionuclide scans may be performed to document and quantify the extent of disease activity.

Follow-up evaluations of these assessments at regular intervals during the course of therapy (approximately every 1 to 3 months) will be useful in determining response to therapy and potential toxicity, permitting adjustments in the number of immunizations administered.

### 7) Restrictions on Concurrent Therapy

For optimal effects of this treatment, patients should receive no concurrent therapy which is known to suppress the immune system.

### 8) Final Formulation

Each patient will receive subcutaneous immunizations with a mixture if irradiated tumor cells and autologous fibroblast CE cells genetically modified to secrete IL-2. Approximately 10⁷ tumor cells will be mixed with 10⁷ fibroblasts known to secrete at least 20 units/ml of IL-2 in tissue culture when semi-confluent (12). The irradiated tumor cells and genetically modified fibroblasts will be placed in a final volume of 0.2 ml normal saline for immunization.

### 9) Dose Adjustments

At least two subcutaneous immunizations will be administered, two weeks apart, with irradiated tumor cells and autologous fibroblasts genetically modified to secrete IL-2. If no toxicity is observed, subsequent booster immunizations may be administered periodically (at least one week apart) to optimize the anti-tumor immune response.

### J) Treatment of Potential Toxicity

Toxic side effects are not expected to result from these immunizations. However, potential side effects of these immunizations are treatable in the following manner:

If massive tumor cell lysis results, any resulting uric acid nephropathy, adult respiratory distress syndrome, disseminated intravascular coagulation or hyperkalemia will be treated using standard methods.

Local toxicity at the sites of immunization will be treated with either topical steroids and/or surgical excision of the injection site as deemed appropriate.

Hypersensitivity reactions such as chills, fever and/or rash will be treated symptomatically with antipyretics and antihistamines. Patients should not be treated prophylactically. Should arthralgias, lymphadenopathy or renal dysfunction occur, treatment with corticosteroids and/or antihistamines will be instituted. Anaphylaxis will be treated by standard means such as administration of epinephrine, fluids, and steroids.

### EXAMPLE II

### A. Retroviral IL-2 Gene Transfer and Expression in Fibroblasts

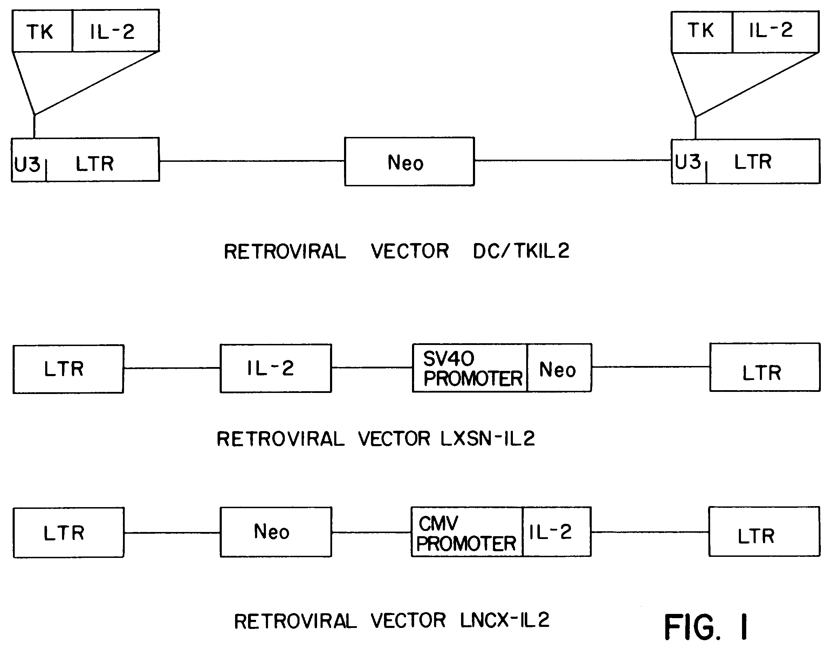

Retroviral vectors were employed to transfer and express IL-2 and neomycin phosphotransferase genes in murine and primary human fibroblasts. The retroviral vector DC/TKIL2 produced by Gilboa and co-workers (Gansbacher, et al., J. Exp. Med. 172:1217-1223, 1990, which is incorporated herein by reference) was utilized to transduce murine fibroblasts for application in an animal tumor model (see Section B below). Human fibroblasts were transduced with the retroviral vector LXSN-RI-IL2. Schematic diagrams of the structure of these retroviral vectors are provided in Figure 1. A more complete description of the LXSN-RI-IL2 vector, including its nucleotide sequence, is provided in Example III and in Tables 2, 3 and 4.

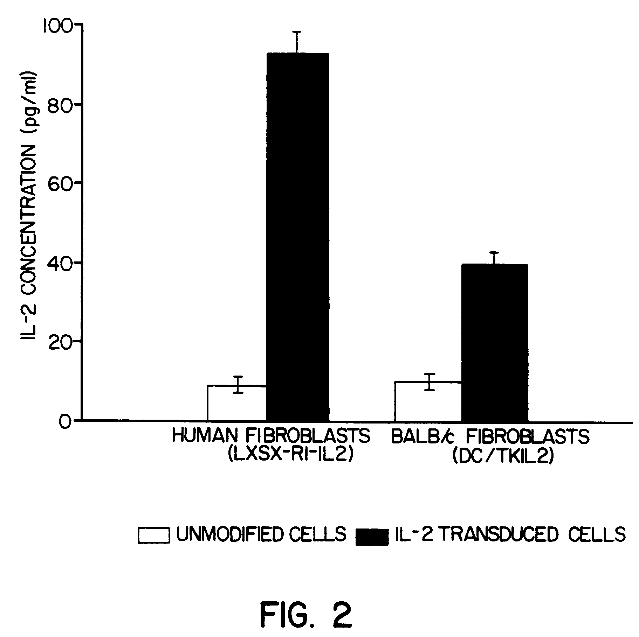

Following infection with the described vectors and selection for 2-3 weeks in growth media containing the neomycin analogue G418, Balb/c and human embryonic fibroblast culture supernatants were harvested and tested for IL-2 by an enzyme-linked immunosorbent assay (ELISA). Figure 2 depicts the levels of IL-2 secreted by the transduced fibroblasts.

These results can be confirmed using negative control fibroblasts infected with an N2-derived retroviral vector expressing an irrelevant gene such as luciferase or β-galactosidase and studies with adult human fibroblasts.

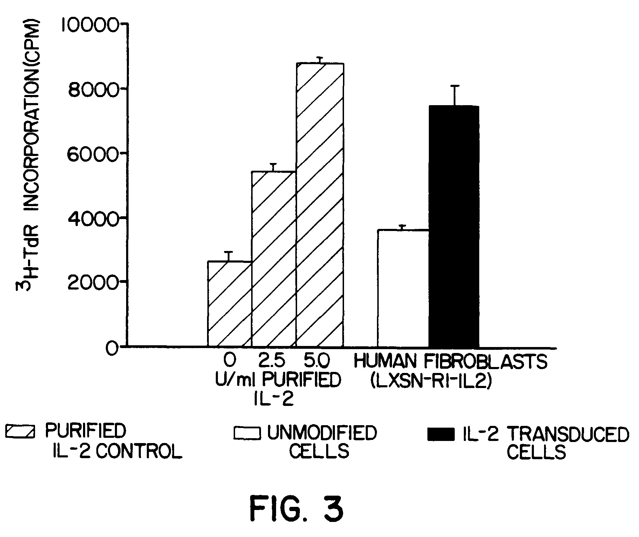

Biological activity of the IL-2 expressed by the transduced human fibroblasts was confirmed by a cell proliferation bioassay employing an IL-2 dependent T cell line. In this assay, supernatant from the transduced fibroblasts and control unmodified fibroblasts were incubated with the IL-2 dependent T cell line CTLL-2. Incorporation of ³H-thymidine was measured as an indicator of cell proliferation and IL-2 activity (Figure 3).

### B. Efficacy of Transduced Fibroblasts in an Animal Tumor Model

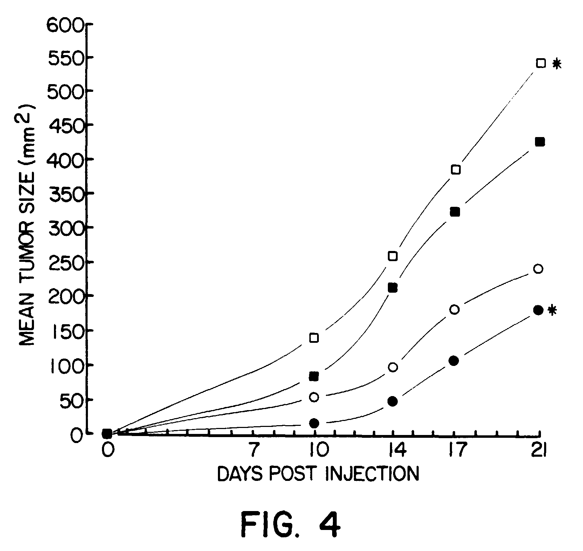

The efficacy of fibroblasts genetically modified to secrete IL-2 was tested in an animal model of colorectal carcinoma. In these studies, the Balb/c CT26 tumor cell line was injected subcutaneously with Balb/c fibroblasts transduced to express IL-2. Control groups included animals injected with 1) a mixture of CT26 tumor cells and unmodified fibroblasts; 2) CT26 tumor cells without fibroblasts and 3) transduced fibroblasts alone. No tumors were detected in 3/8 animals treated with transduced fibroblasts and CT26 cells. In contrast, all untreated control animals (8/8) injected with CT26 tumor cells developed palpable tumors. No tumors were detected in the animals inoculated with transduced fibroblasts without CT26 tumor cells. The mean CT26 tumor size in Balb/c mice injected with the IL-2 secreting fibroblasts was considerably smaller compared to the control groups (Figure 4). A multivariate non-parametric statistical procedure (Koziol, et al., Biometries 37:383-390, 1981 and Koziol, et al., Computer Prog. Biomed. 19:69-74, 1984, which is incorporated herein by reference) was utilized to evaluate differences in tumor growth among the treatment groups. The tumor growth curves for the four treatment groups presented in Figure 4 were significantly different (p=0.048). Subsequent comparisons between treatment groups revealed a significant difference (p < 0.05) in tumor growth between animals injected with CT26 tumor cells alone and animals treated with 2 x 10⁶ transduced fibroblasts and CT26 tumor cells (Figure 4).

### EXAMPLE III

### A. Project Overview

Lymphokine gene therapy of cancer will be evaluated in cancer patients who have failed conventional therapy. An N2-derived vector containing the neomycin phosphotransferase gene will be used. This vector has been employed by a number of investigators for in vitro and in vivo studies including recently approved investigations with human subjects (Rosenberg et al., N. Eng. J. Med., 323:570-578, 1990). The lymphokine vectors used in this investigation will be generated from the N2-derived vector, LXSN, developed and described by Miller et al., Mol. Cell Biol. 6:2895, 1986 and Miller et al., BioTechniques 7:980, 1989, which are incorporated herein by reference. The vector LXSN-RI-IL2 contains human IL-2 cDNA under the control of the retroviral 5' LTR promoter and the neomycin phosphotransferase gene under the control of the SV40 promoter (see Figure 1). The normal human IL-2 leader sequence has been replaced with a chimeric sequence containing rat insulin and human IL-2 leader sequences (see Tables 2, 3 and 4). This chimeric leader sequence enhances IL-2 gene expression.

To construct the LXSN-RI-IL2 vector, the bacterial plasmid pBC12/CMV/IL2 (Cullen, B.R., DNA 7:645-650, 1988, which is incorporated herein by reference) containing the full-length IL-2 cDNA and chimeric leader sequence was digested with HindIII and the ends were blunted using Klenow polymerase. IL-2 cDNA was subsequently released from the plasmid by digestion with BamHI. The IL-2 fragment was purified by electrophoresis in a 1% agarose gel and the appropriate band was extracted utilizing a glass powder method. Briefly, the gel slice was dissolved in 4M NaI at 55°. After cooling to room temperature, 4 µl of oxidized silica solution (BIO-101, La Jolla, CA) was added to adsorb the DNA. The silica was ythen washed with a cold solution of 50% ethanol containing 0.1 M NaCl in TE buffer. The DNA was eluted from the silica by heating at 55° in distilled H₂O. The purified IL-2 cDNA was then directionally ligated into the HpaI-BamHI cloning sites of the pLXSN vector. A more complete description of the pLXSN-RI-IL2 vector and its partial nucleotide sequence are provided in Tables 2, 3, 4, 5 and 6.

**Table 2**

| Description of the LXSN-RI-IL2 from position 1 to 6365 | |
|---|---|
| Bases | Description |
| 1-589 | Moloney murine sarcoma virus 5' LTR |
| 659-1458 | The sequence of die extended packaging signal |
| 1469-2151 | IL-2 cDNA with chimeric leader sequence |
| 1469-1718 | IL-2 chimeric leader sequence |
| 1647-1718 | coding region of the signal peptide |
| 1719-2151 | Mature IL-2 coding sequence |
| 2158-2159 | Mo mu sarcoma virus end/SV 40 start |
| 2159-2503 | Simian virus 40 early promoter |
| 2521-2522 | Simian virus DNA end/Tn5 DNA start |
| 2557-3351 | Neomycin phosphotransferase |
| 3370-3371 | Tn5 DNA end/Moloney murine leukemia virus start |
| 3411-4004 | Moloney murine leukemia virus 3' LTR |
| 4073-4074 | Moloney murine leukemia DNA end/pBR322 DNA start |
| 4074-6365 | Plasmid backbone |

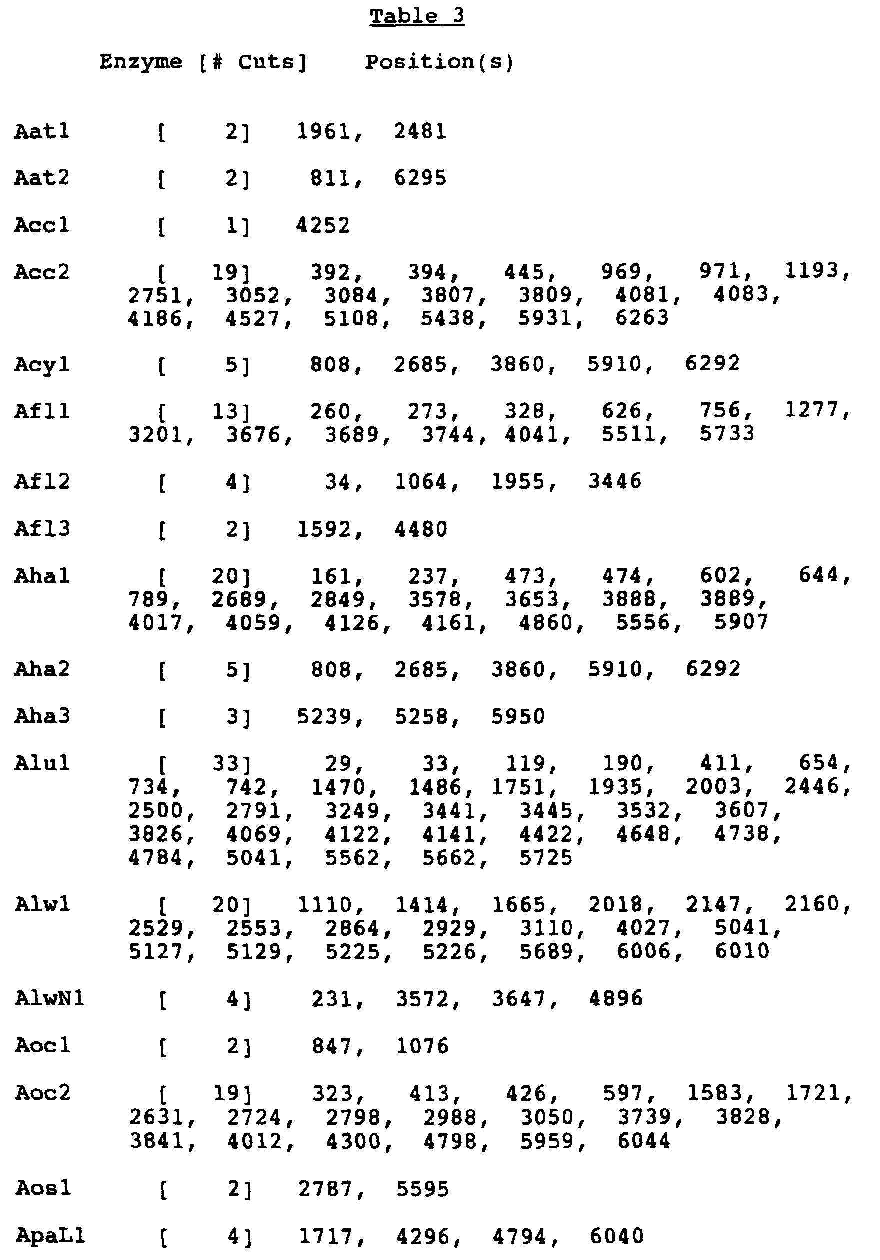

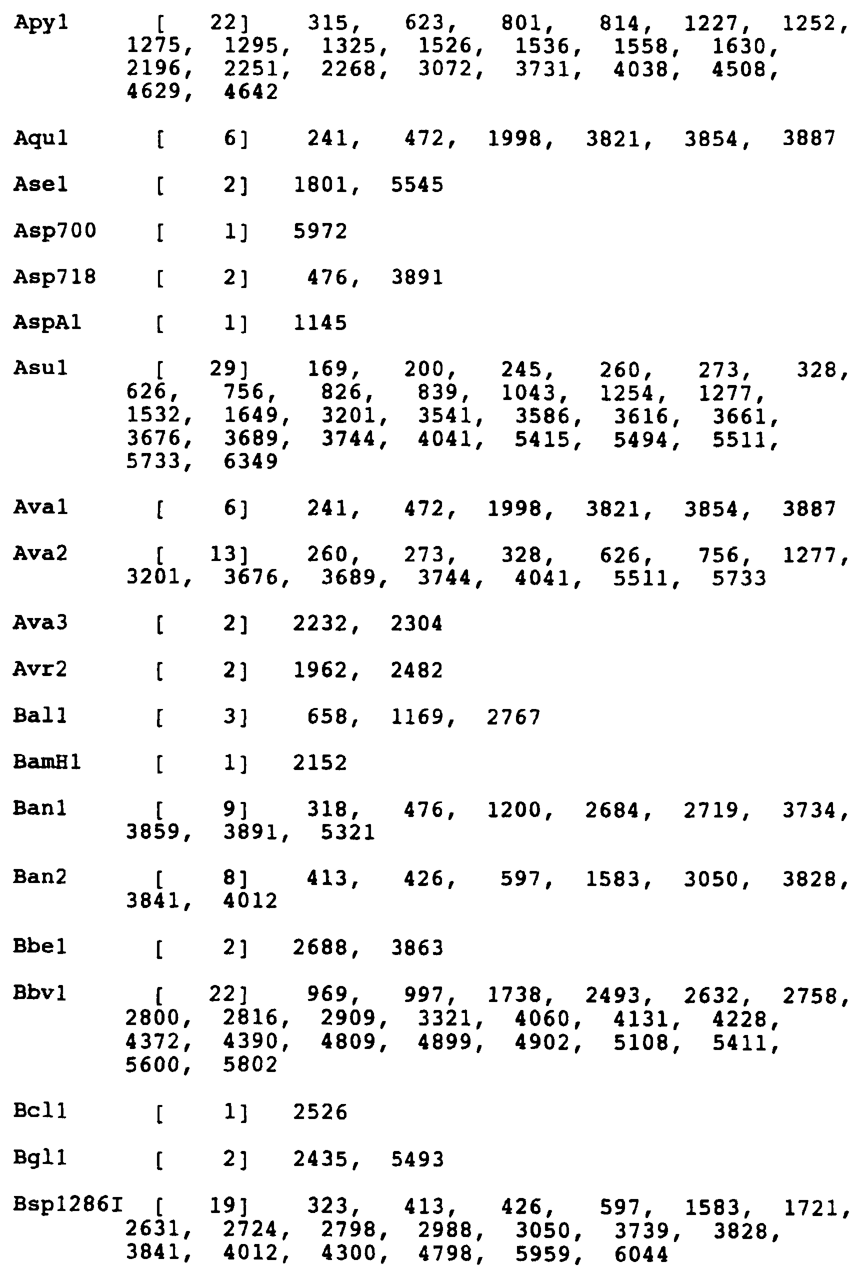

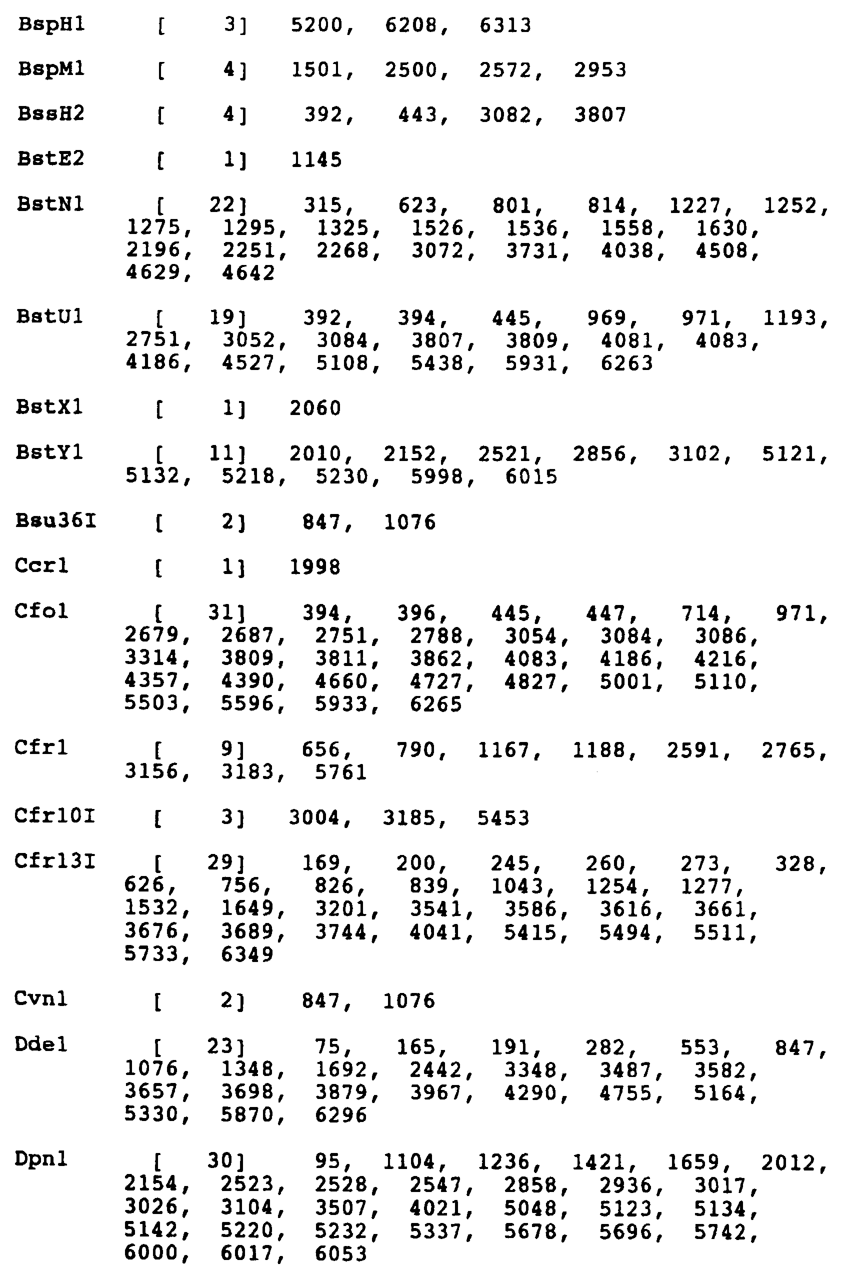

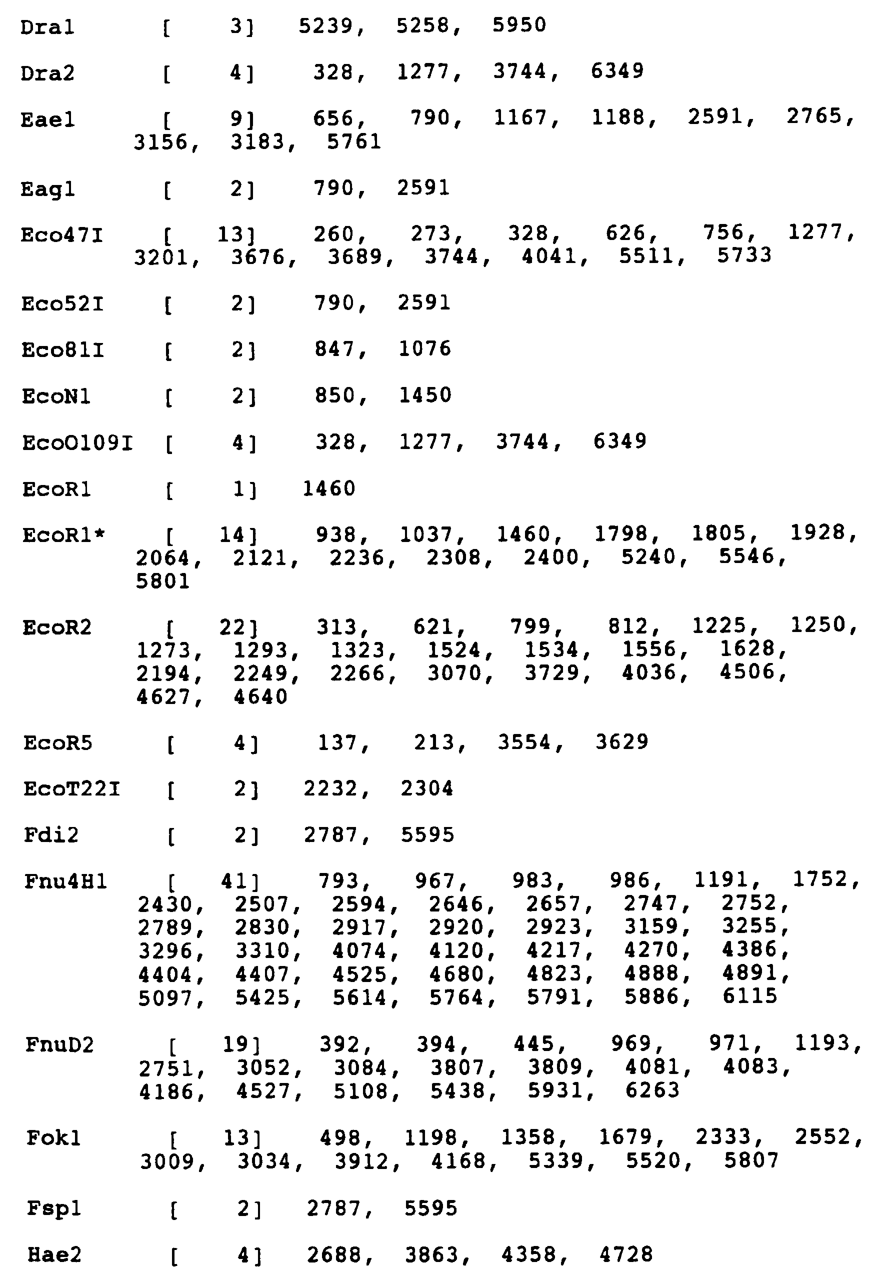

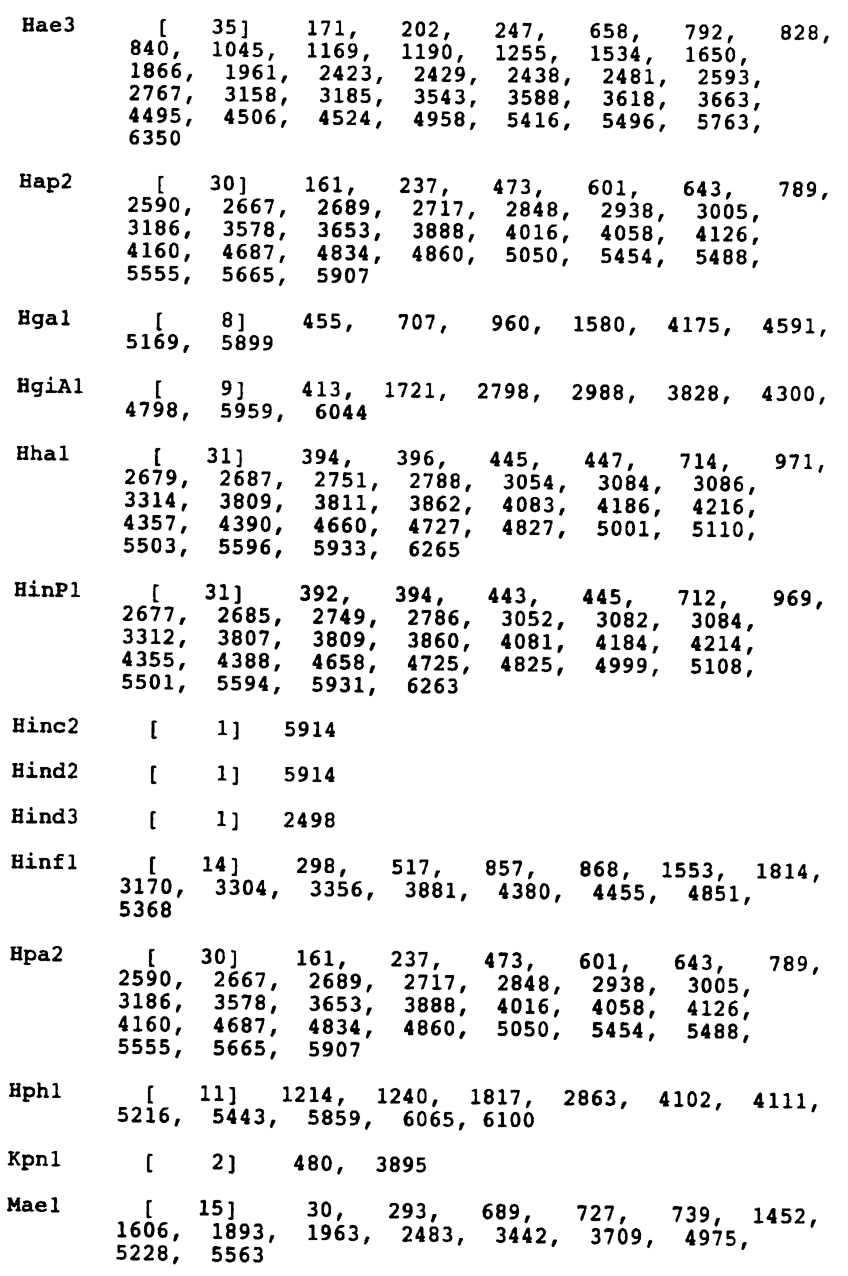

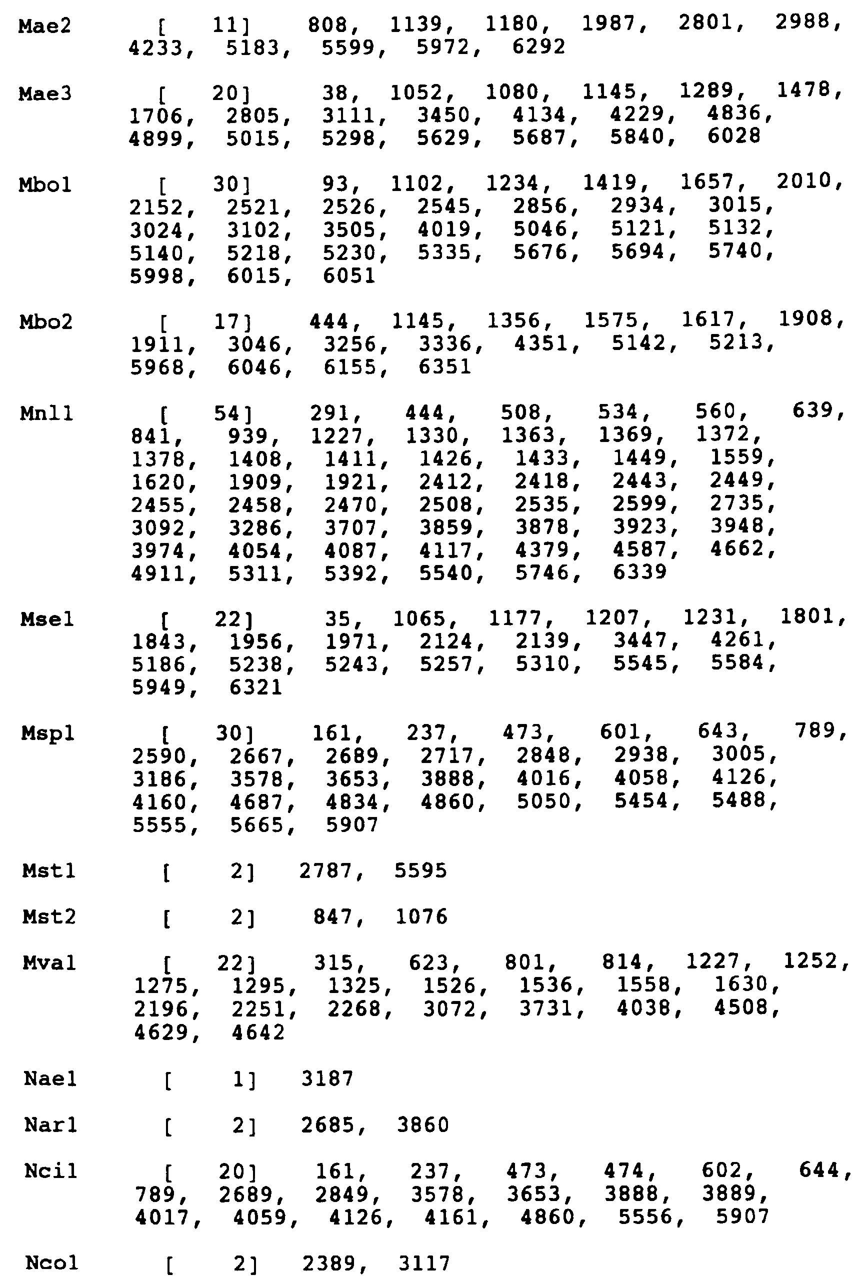

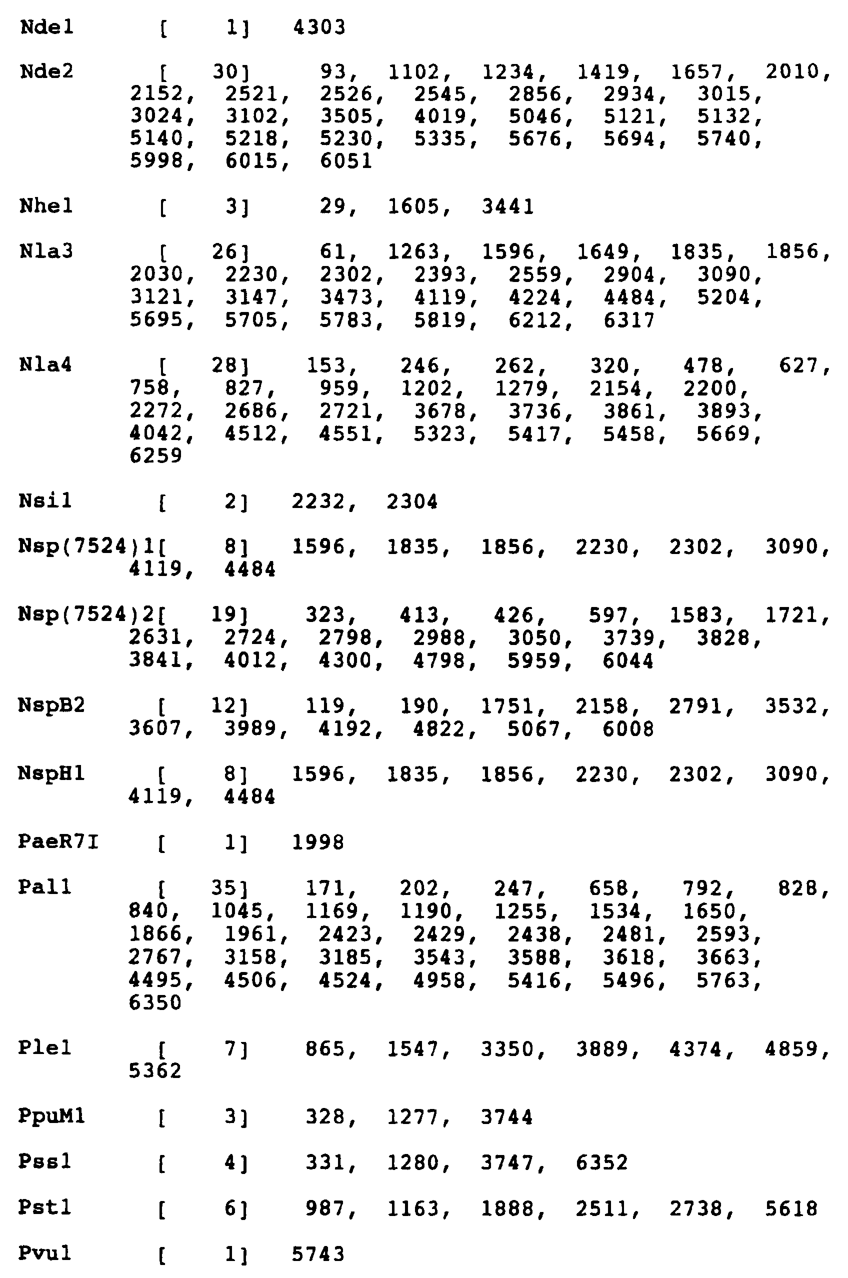

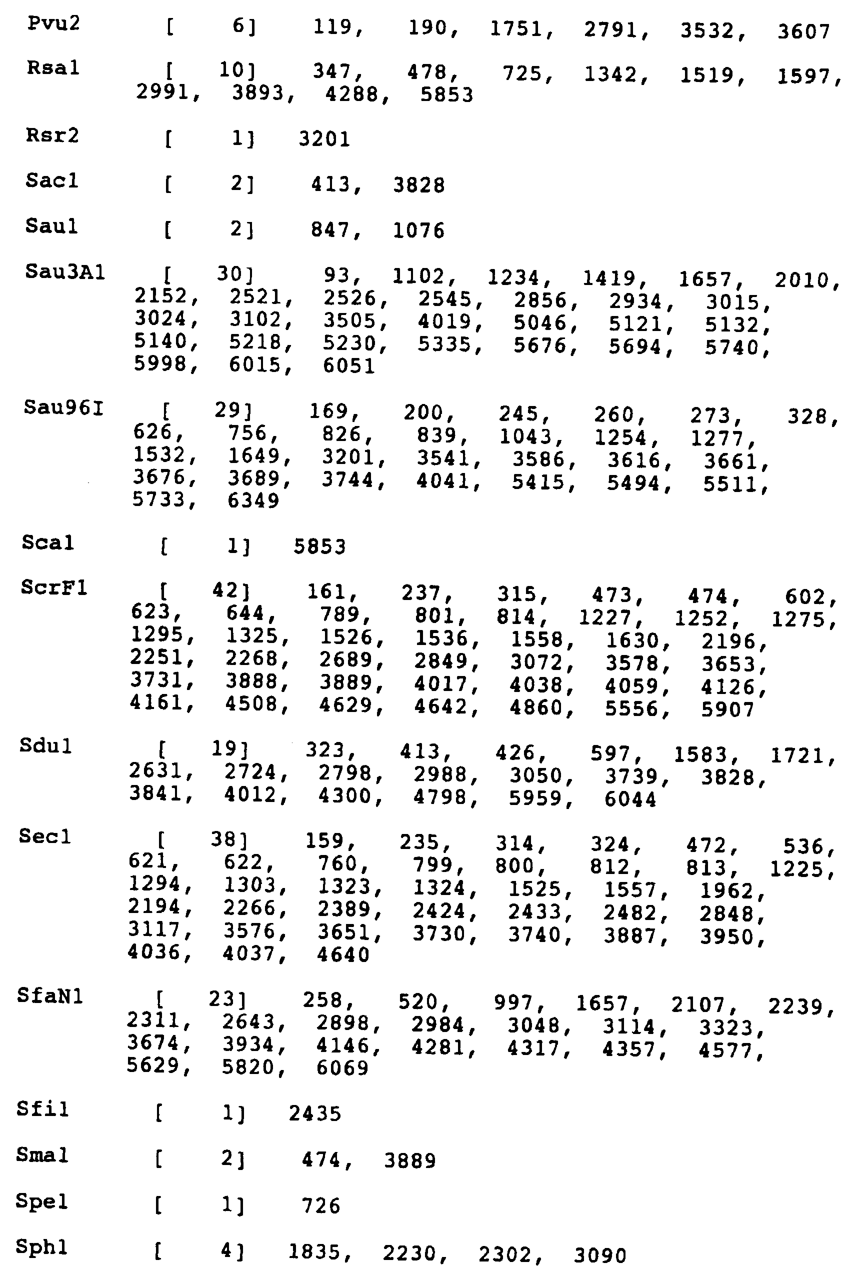

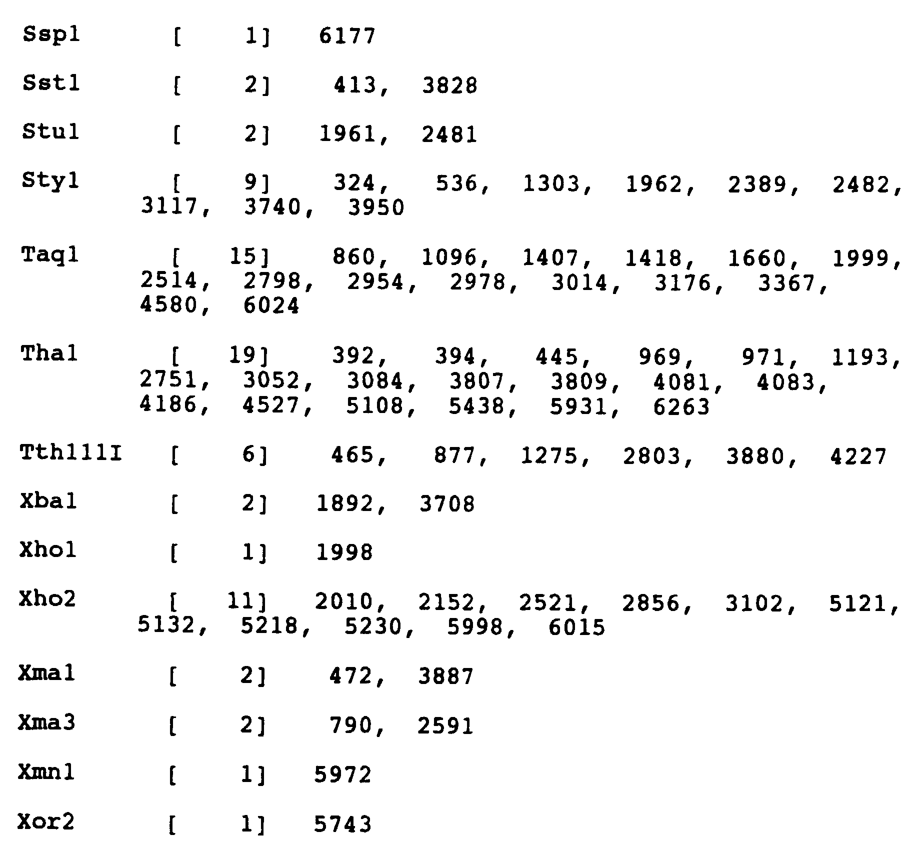

**Table 4**

| Enzymes which do not cut LXSNRII.L2: | | | | |
|---|---|---|---|---|
| Acc3 | Bgl2 | Cla1 | Hpa1 | Nru1 |
| SnaB1 | | | | |
| Apa1 | Bsm1 | Dra3 | Mlu1 | PflM1 |
| Spl1 | | | | |
| Asu2 | BspM2 | Eco47III | Mro1 | Sac2 |
| Sst2 | | | | |
| Ban3 | BstB1 | Esp1 | Not1 | Sal1 |

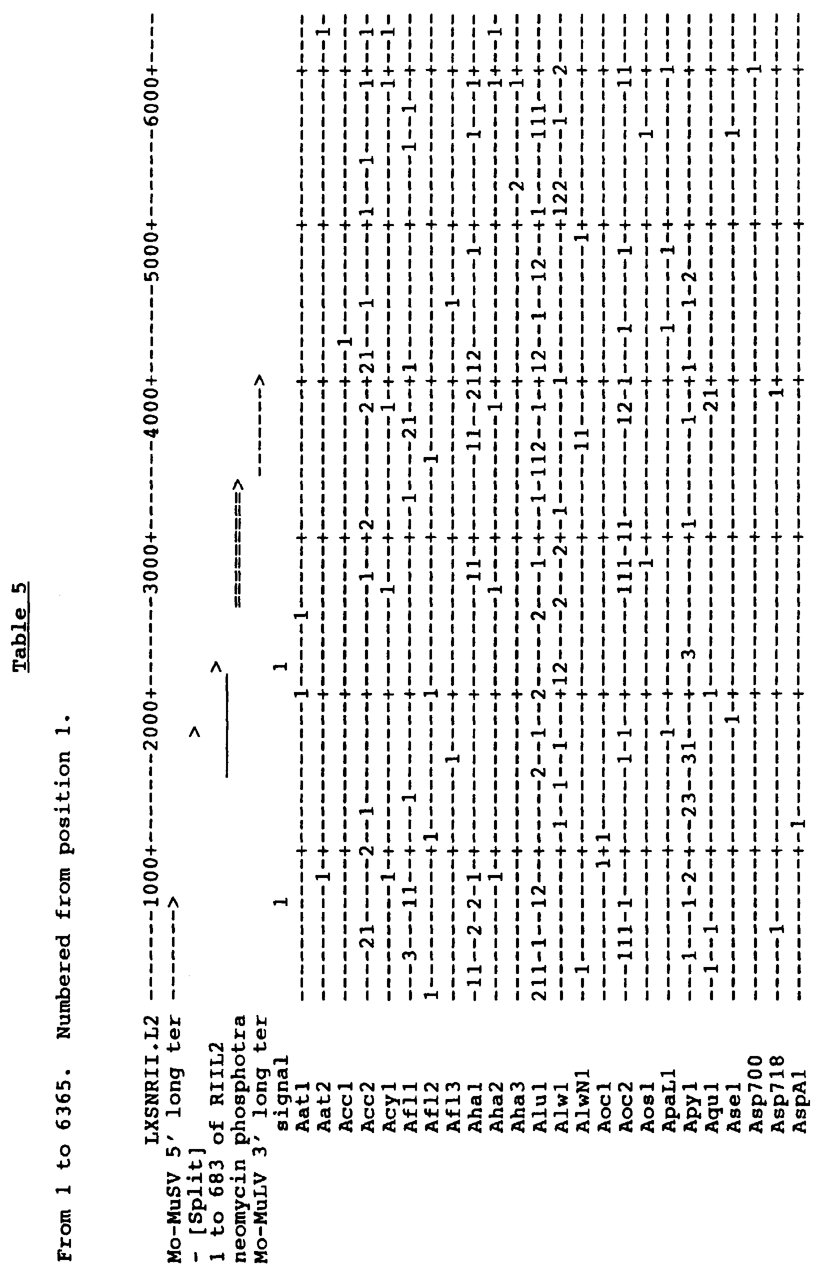

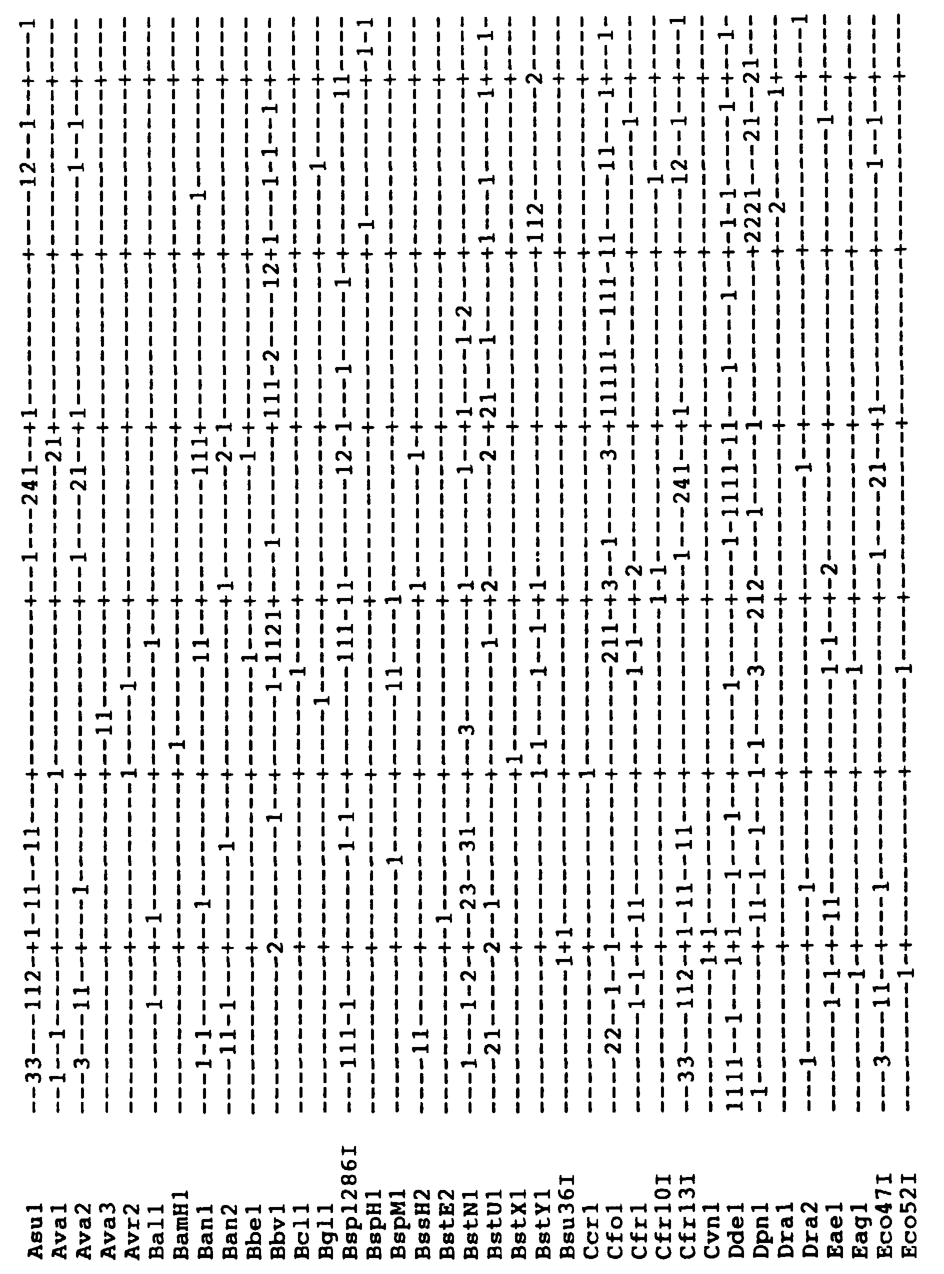

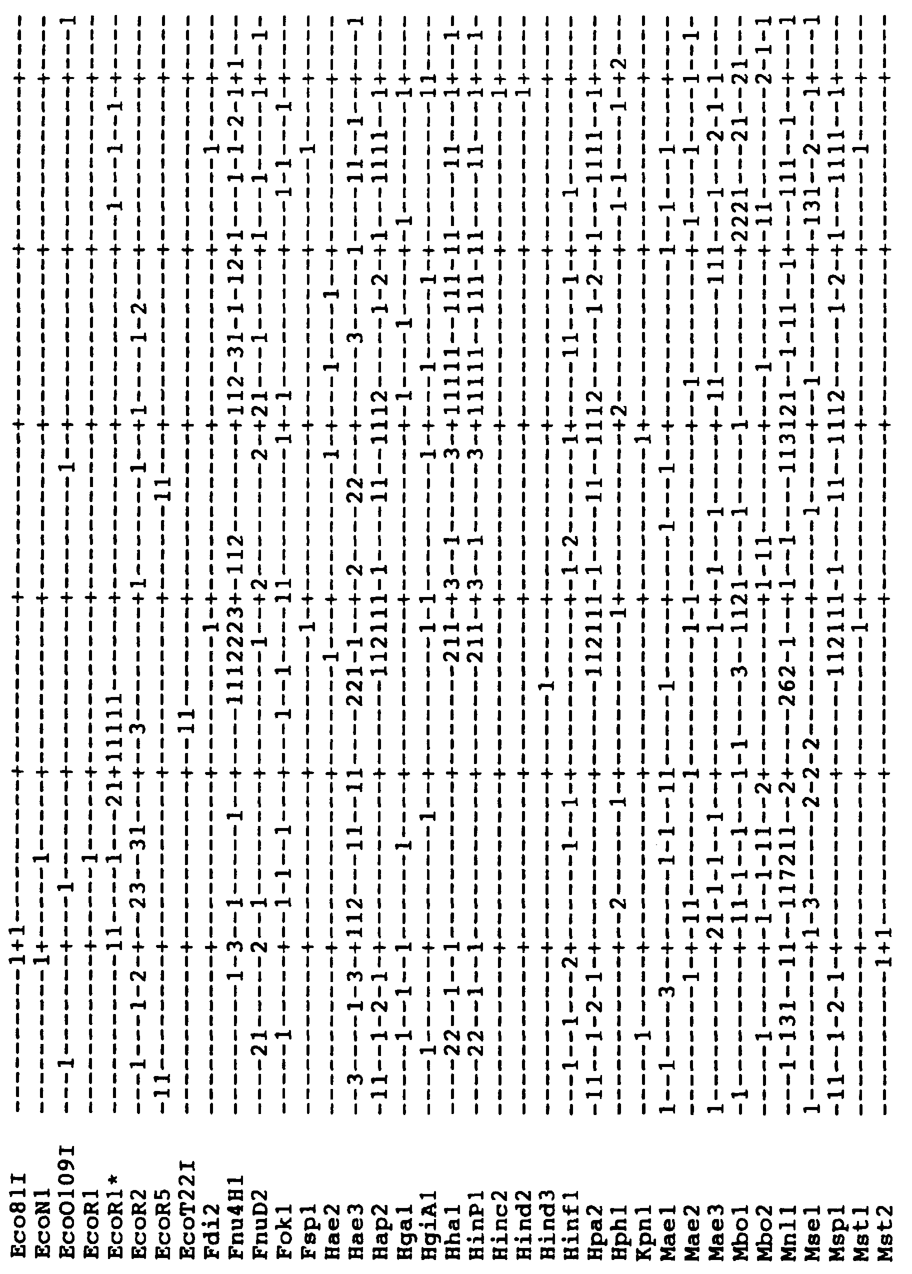

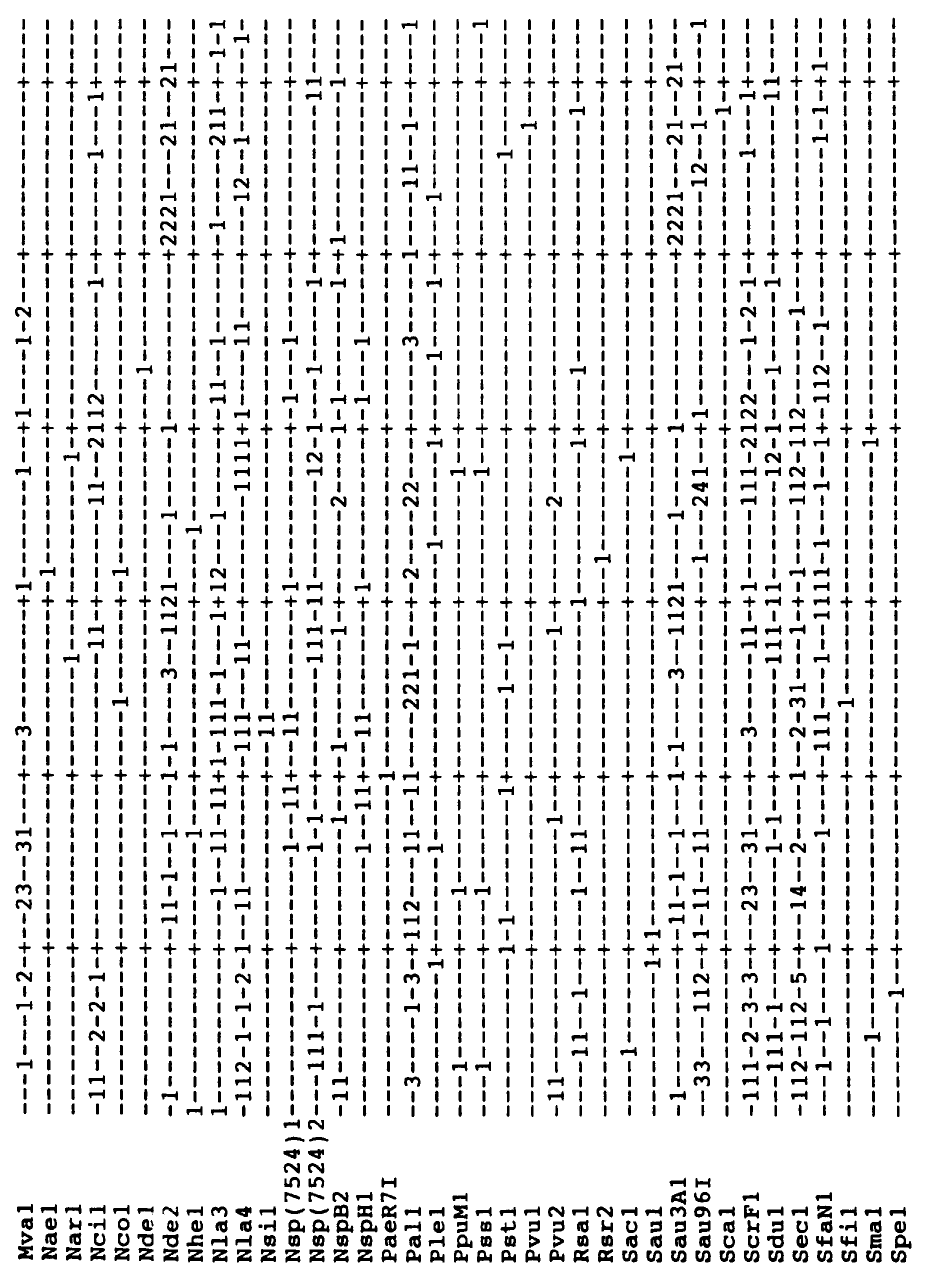

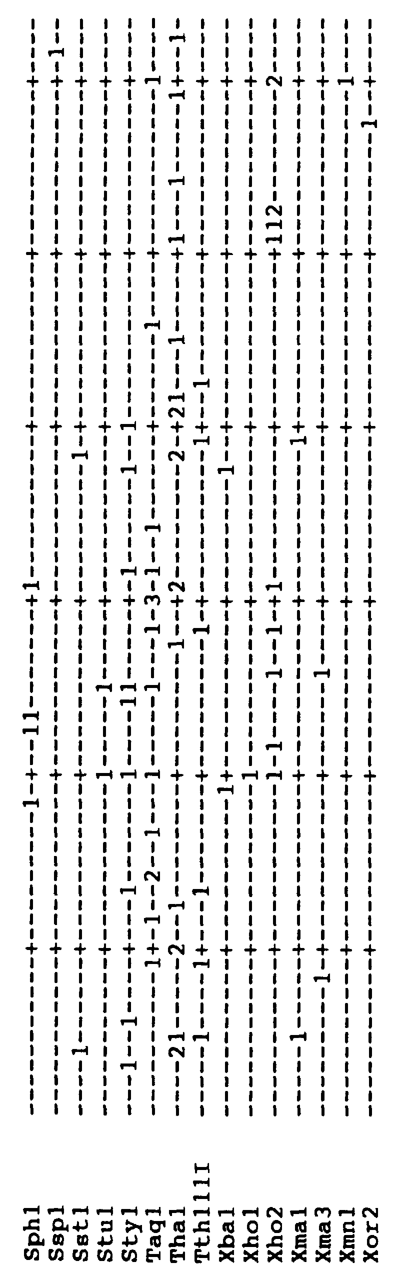

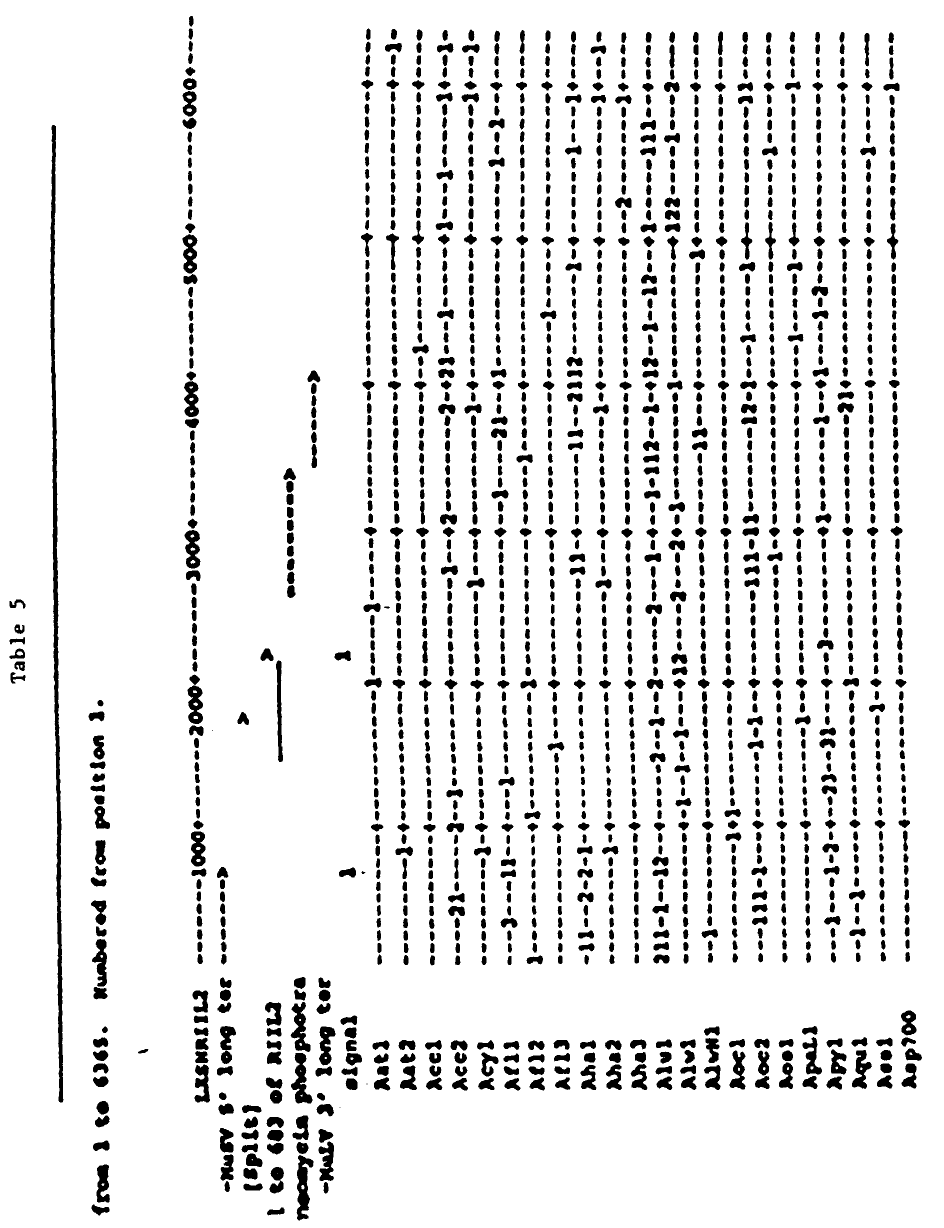

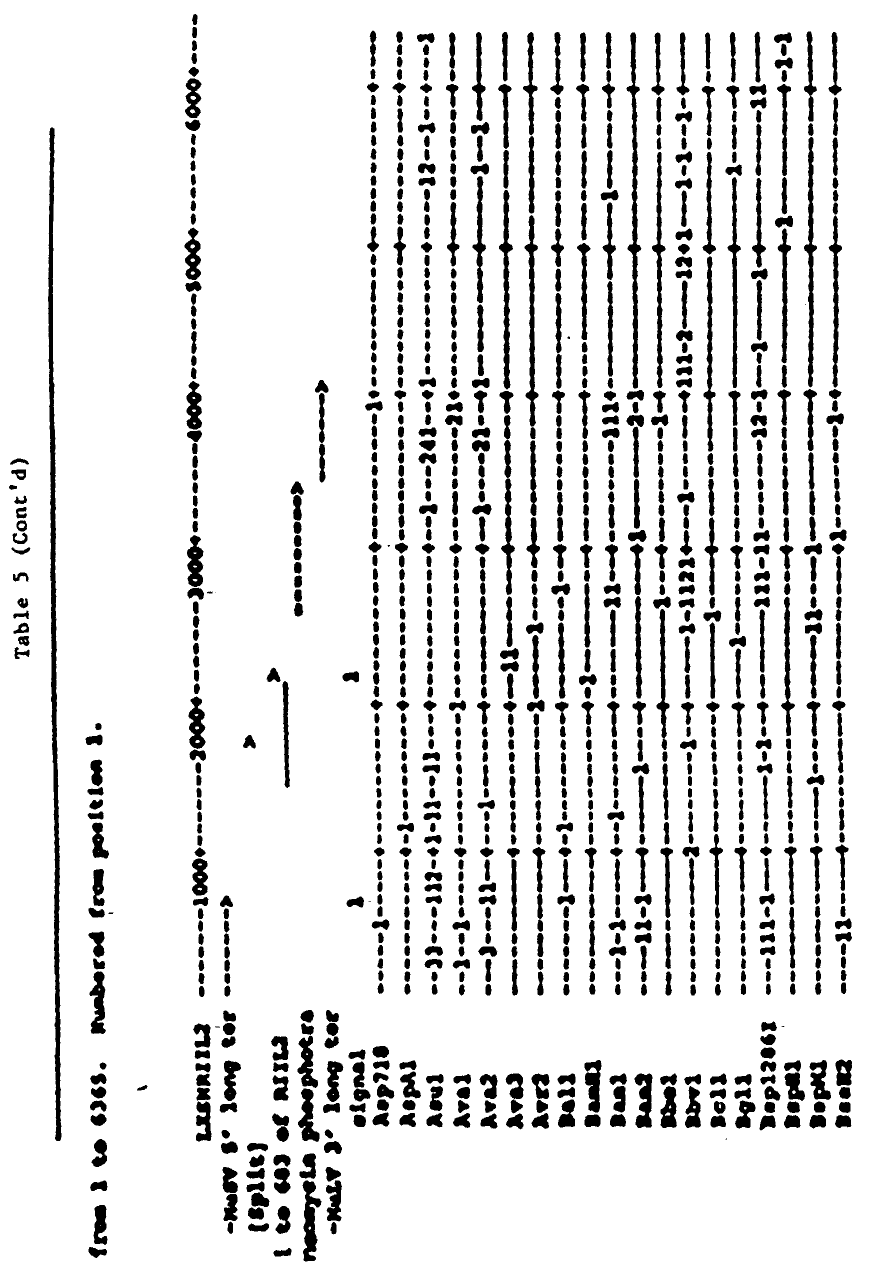

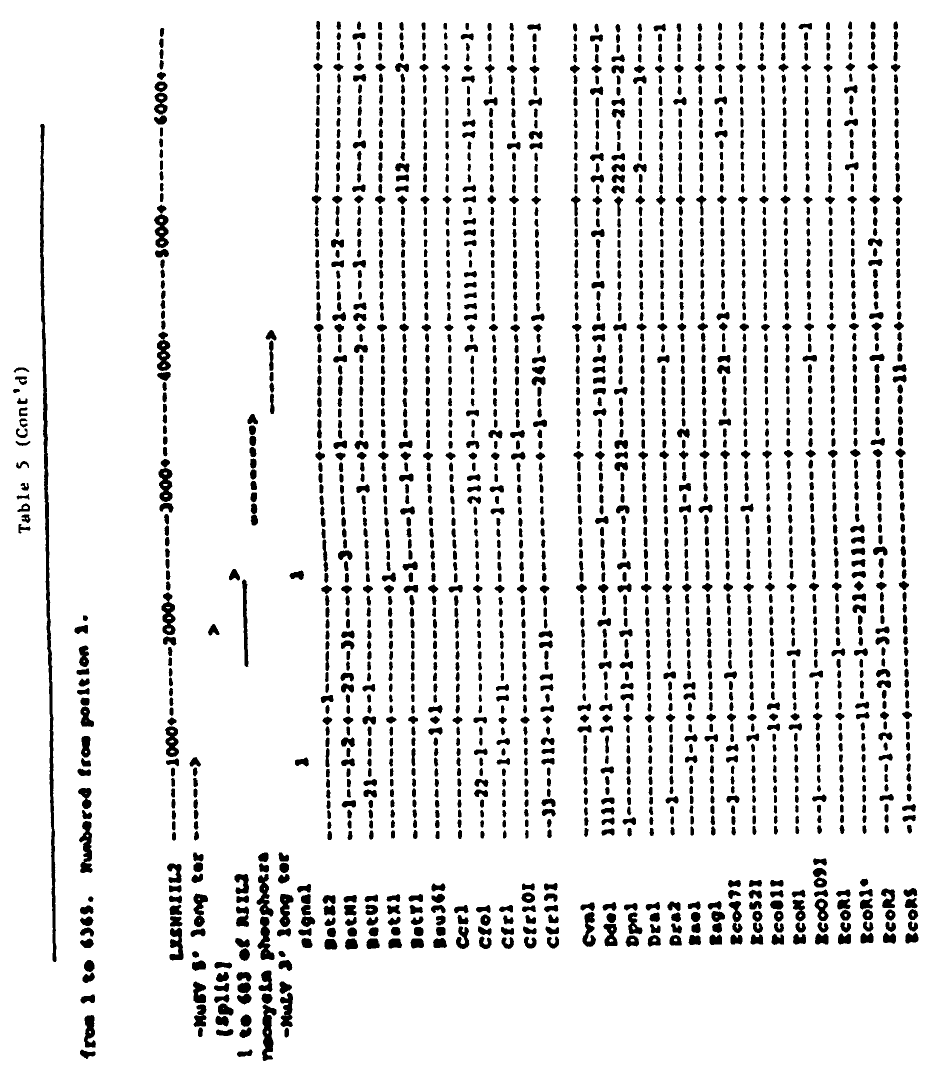

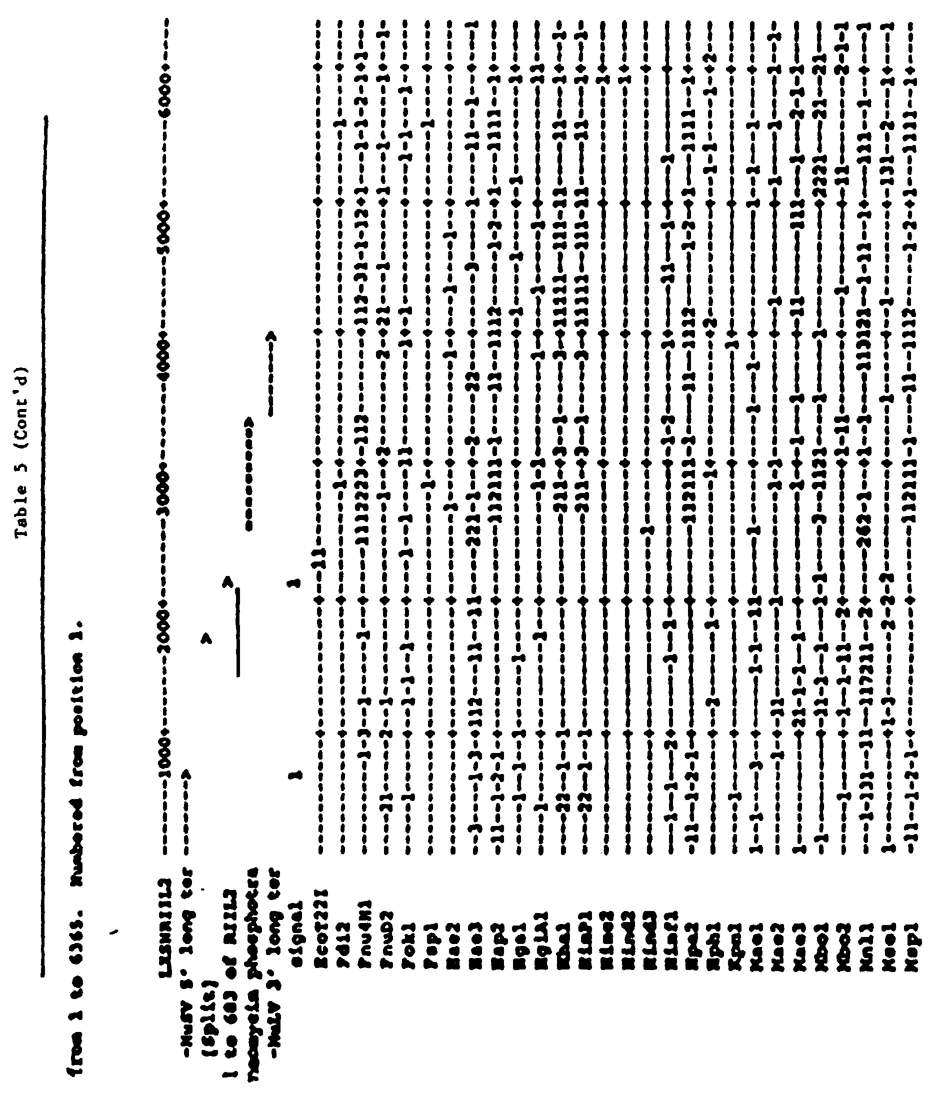

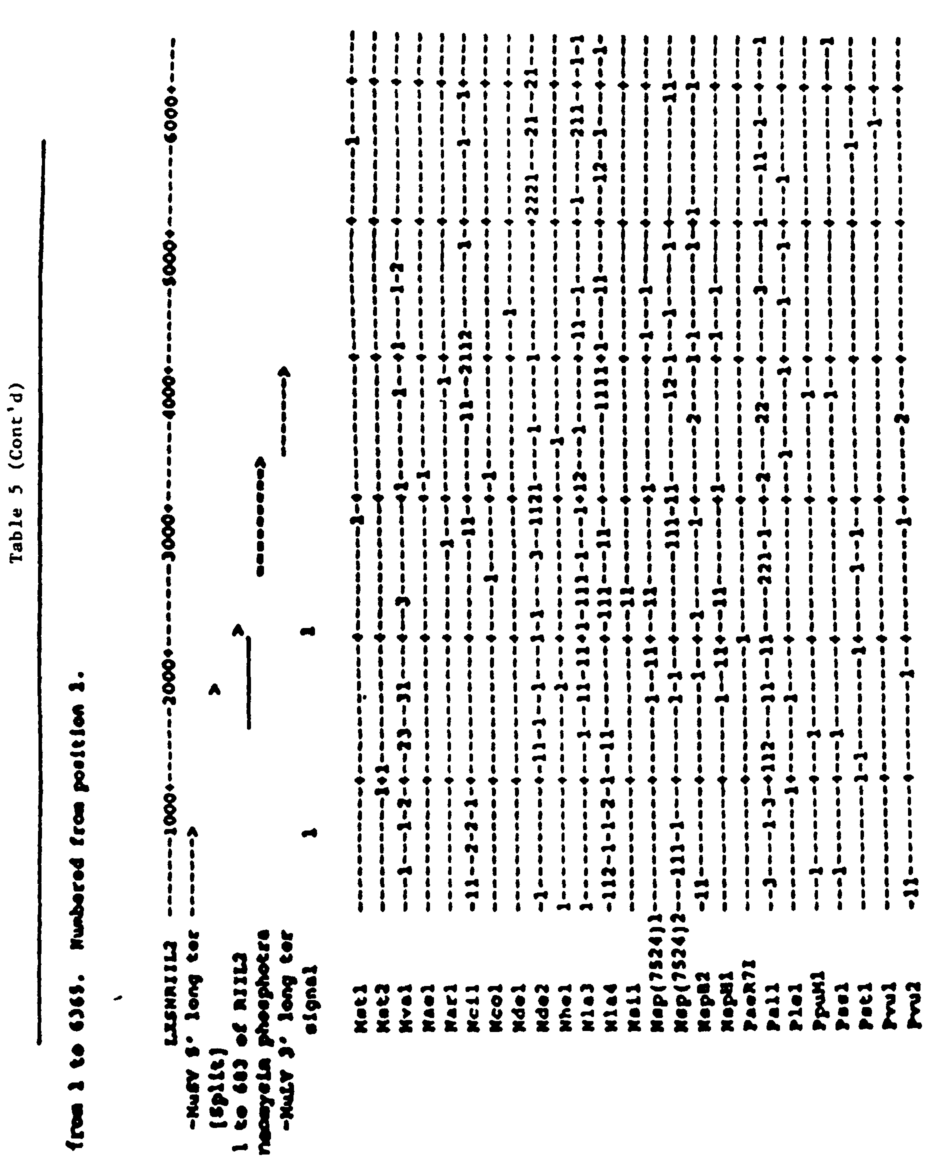

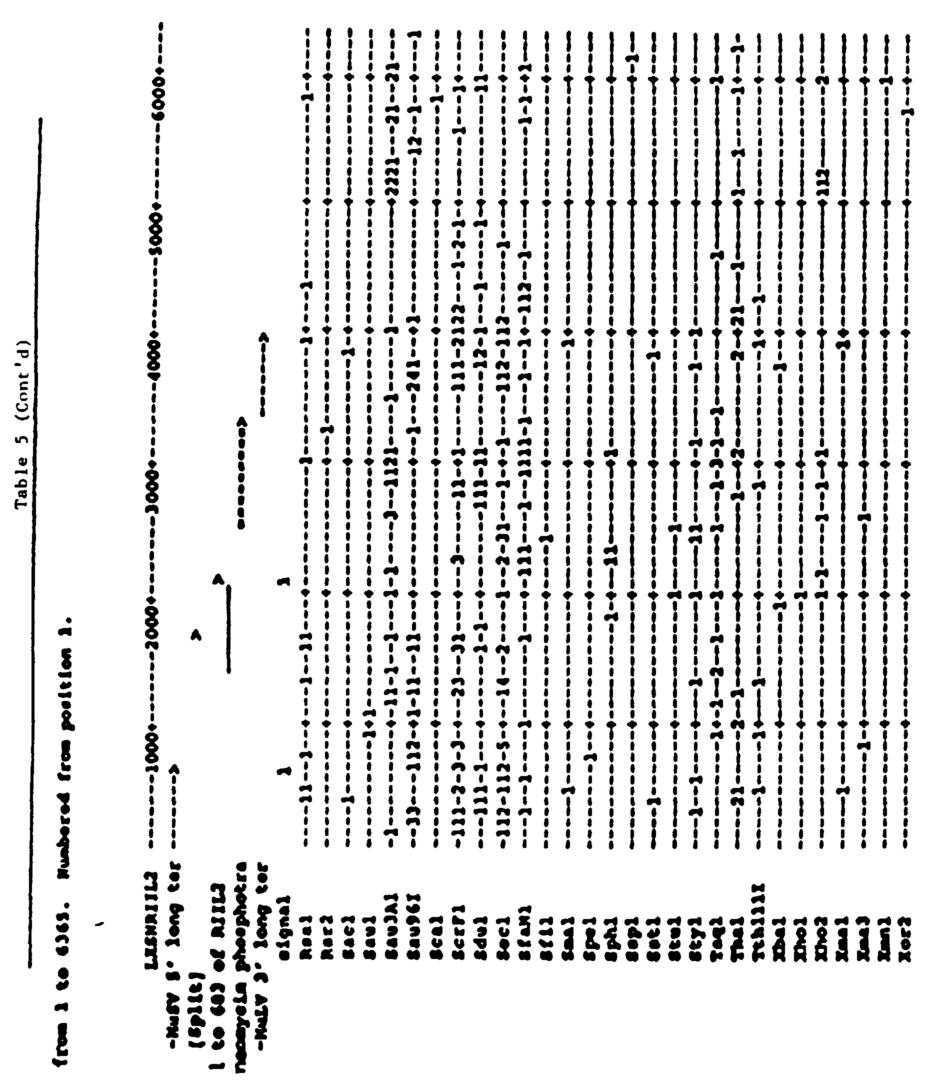

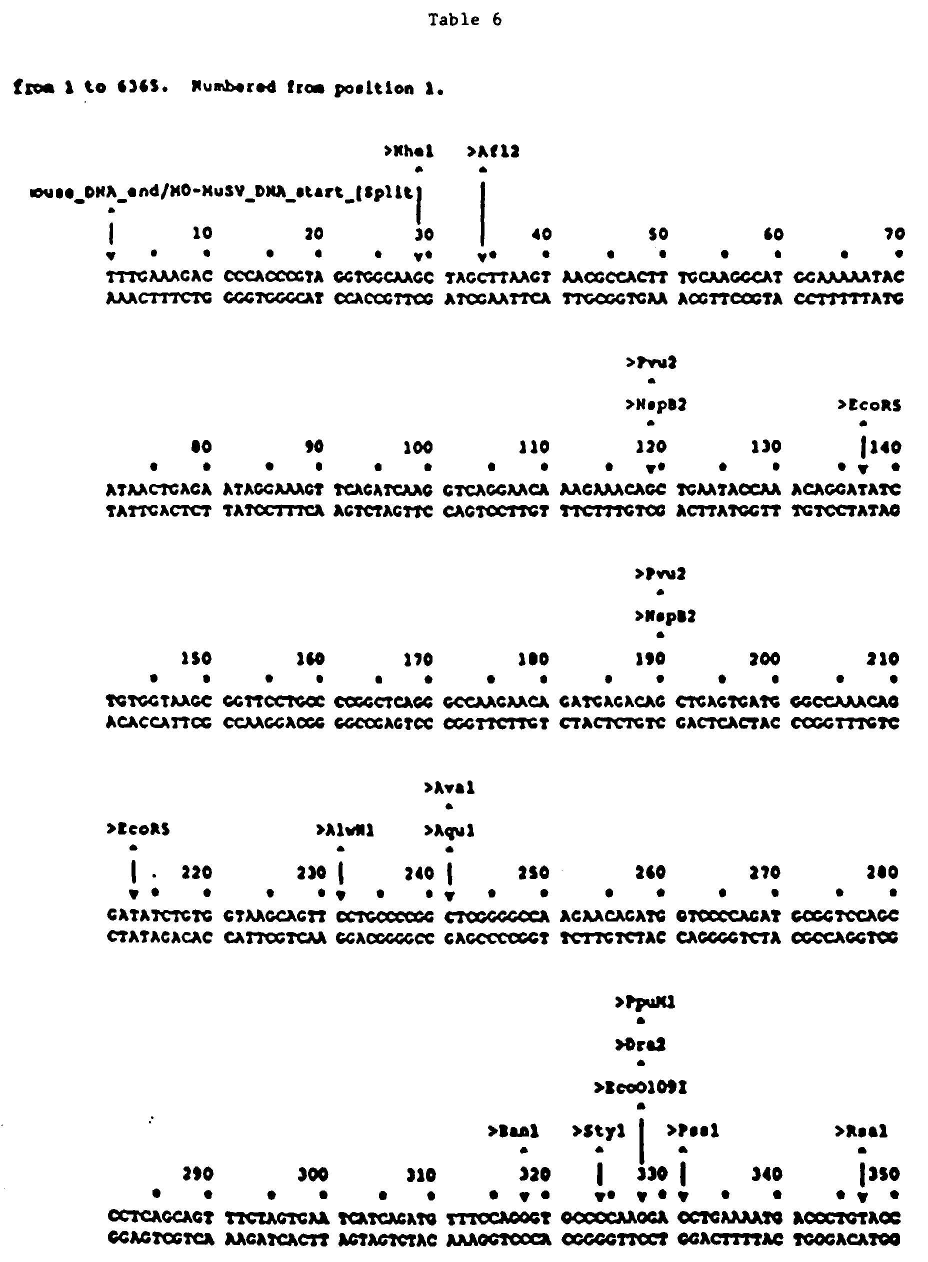

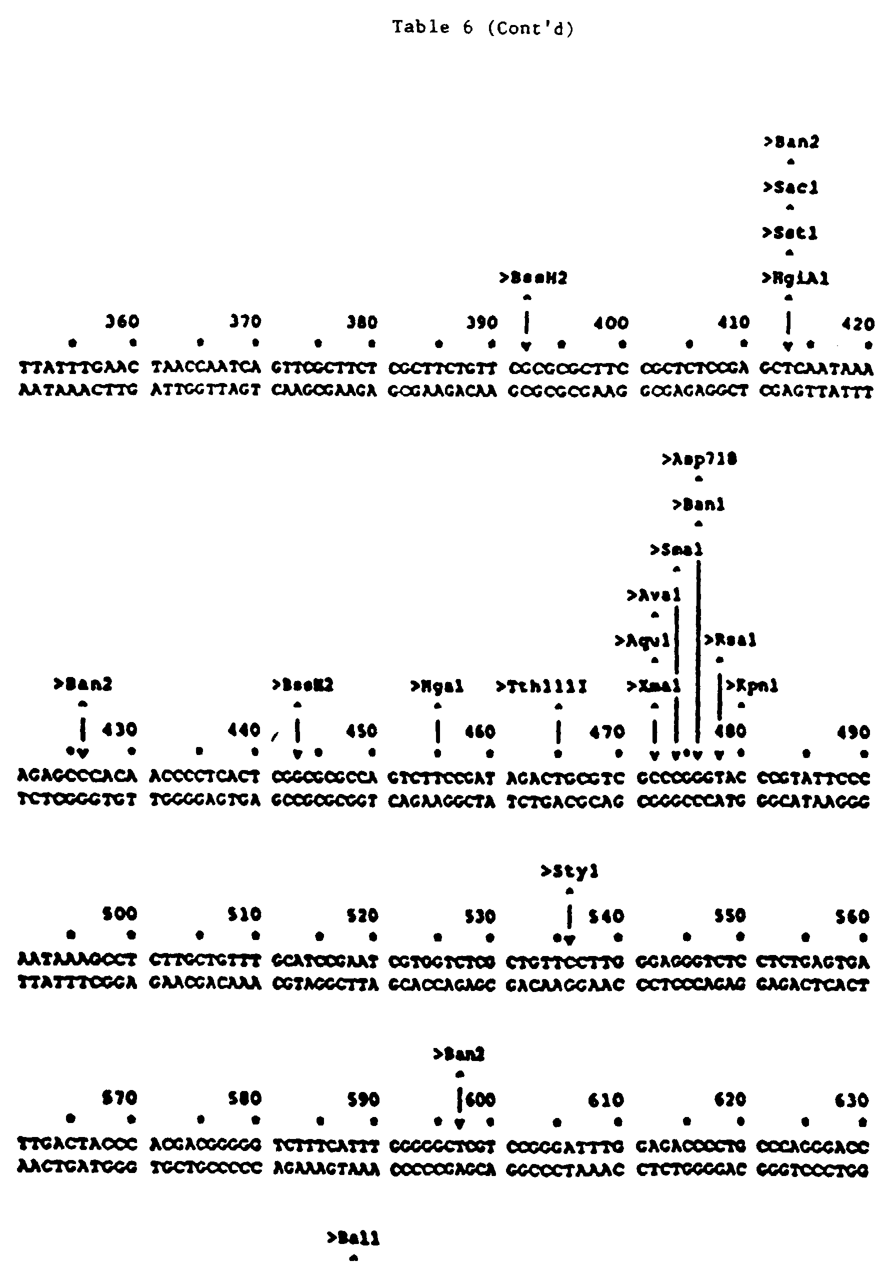

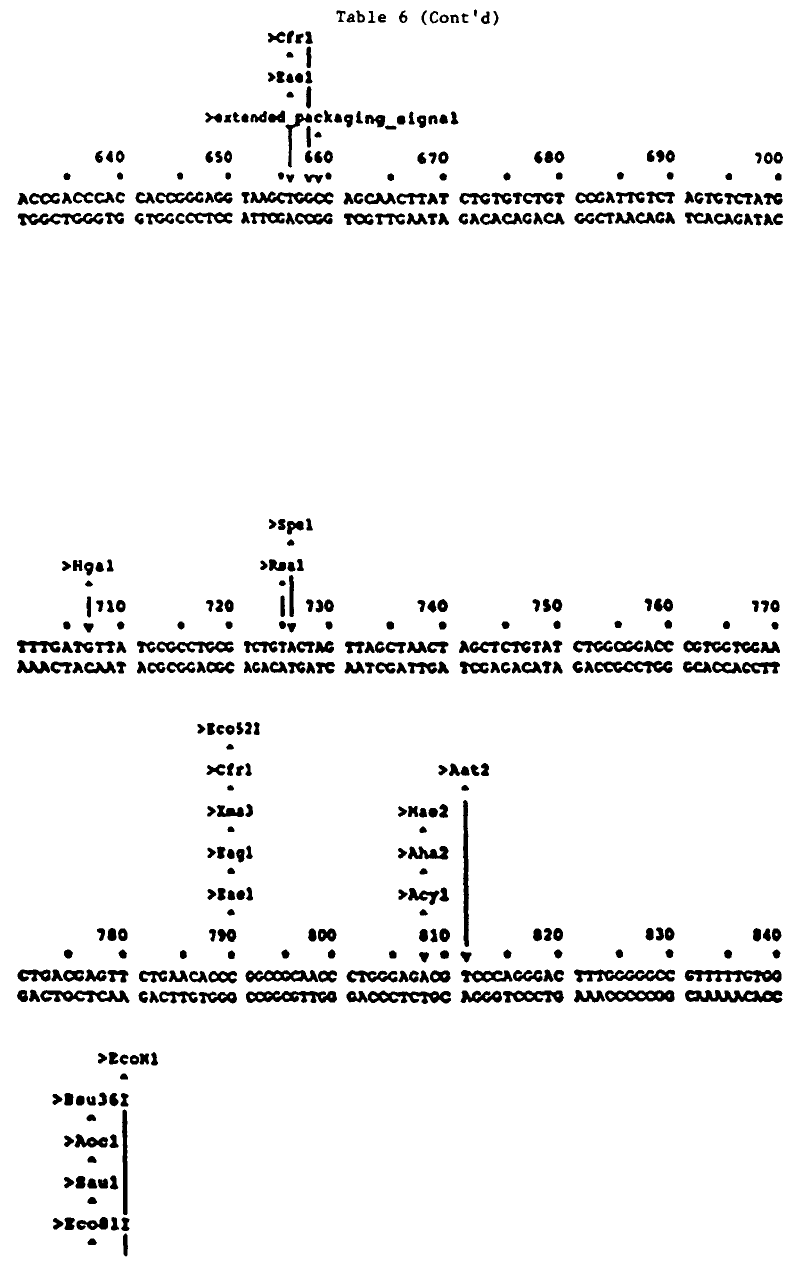

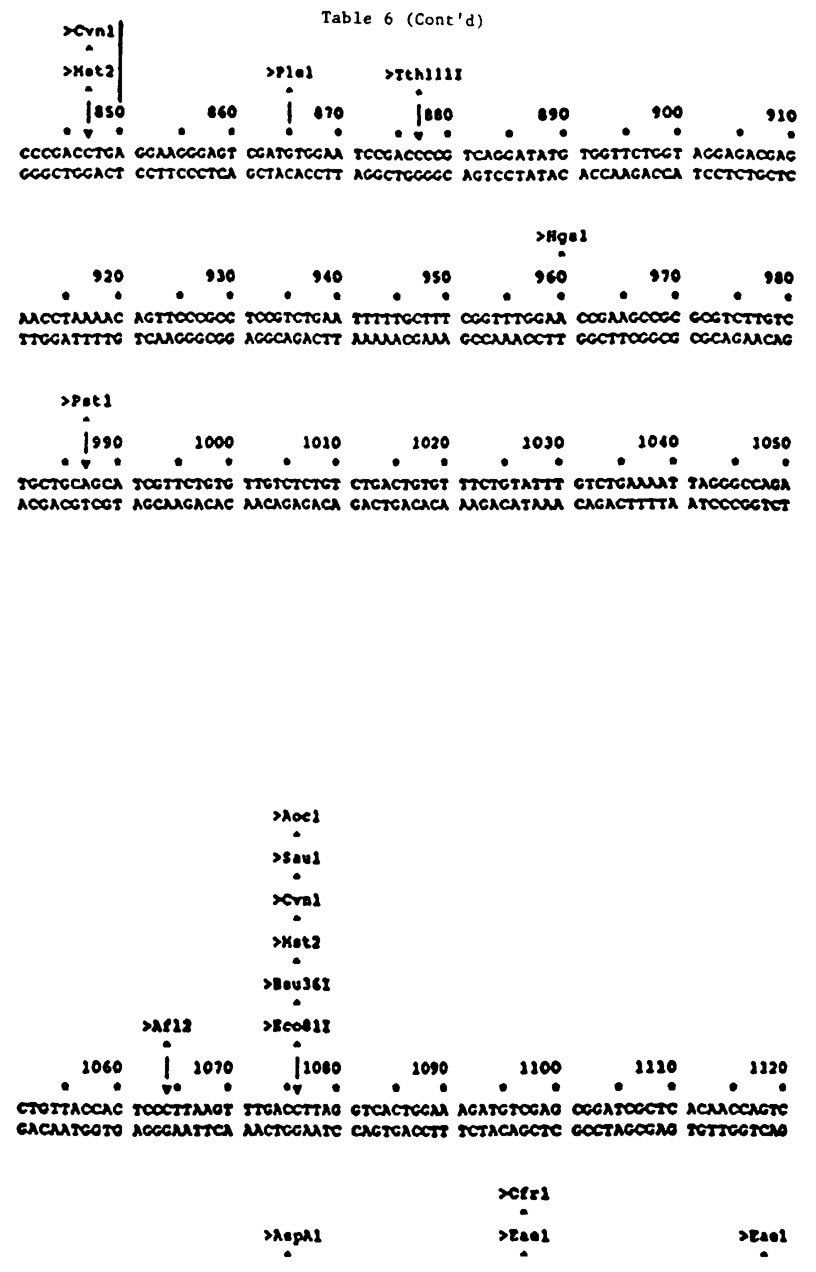

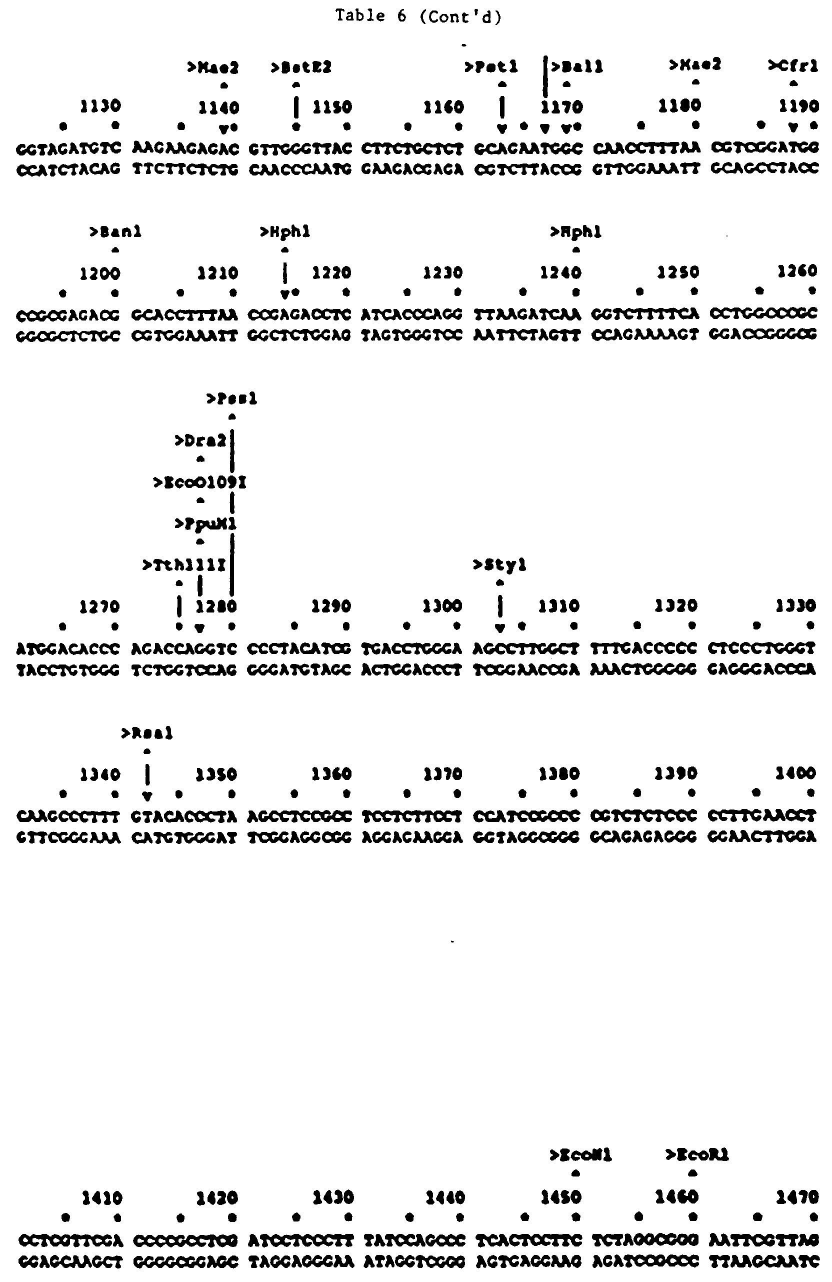

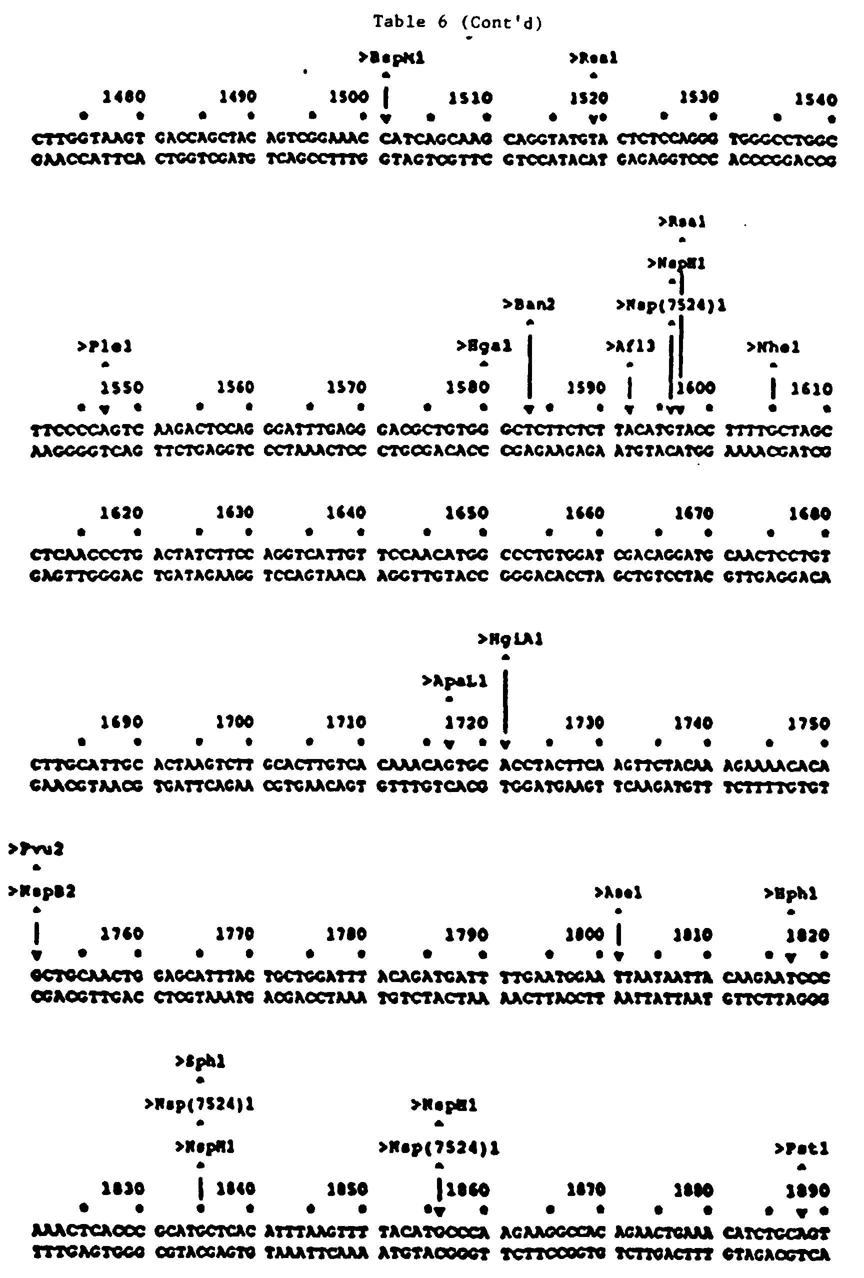

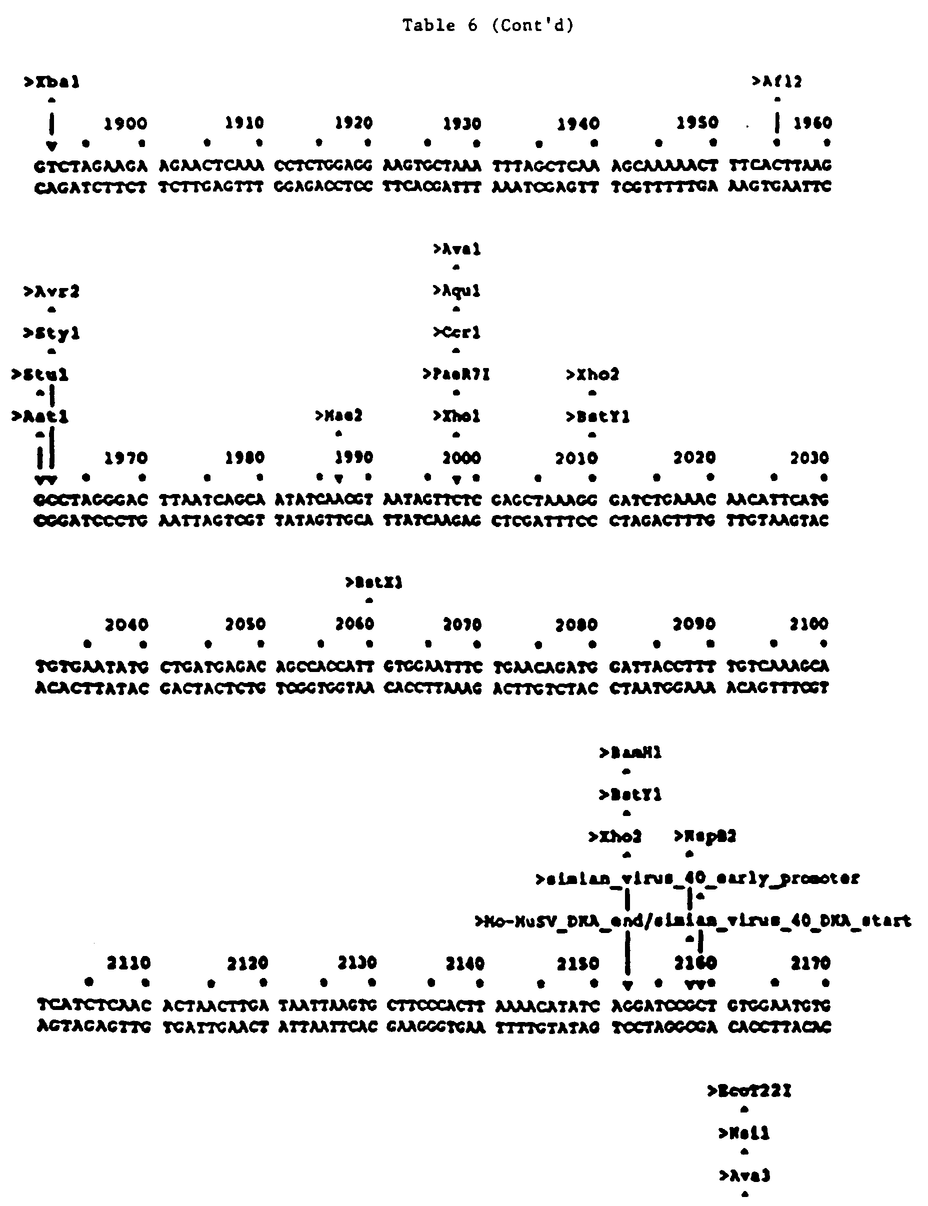

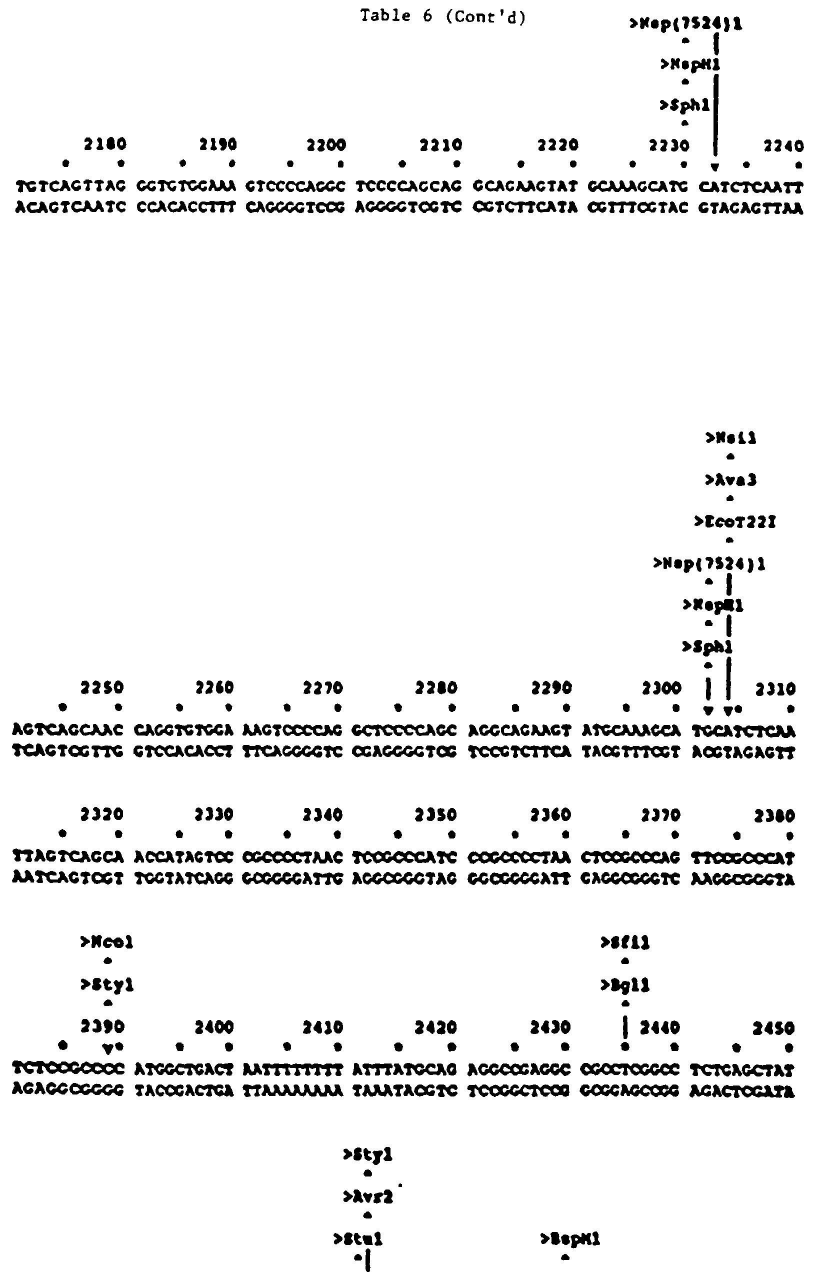

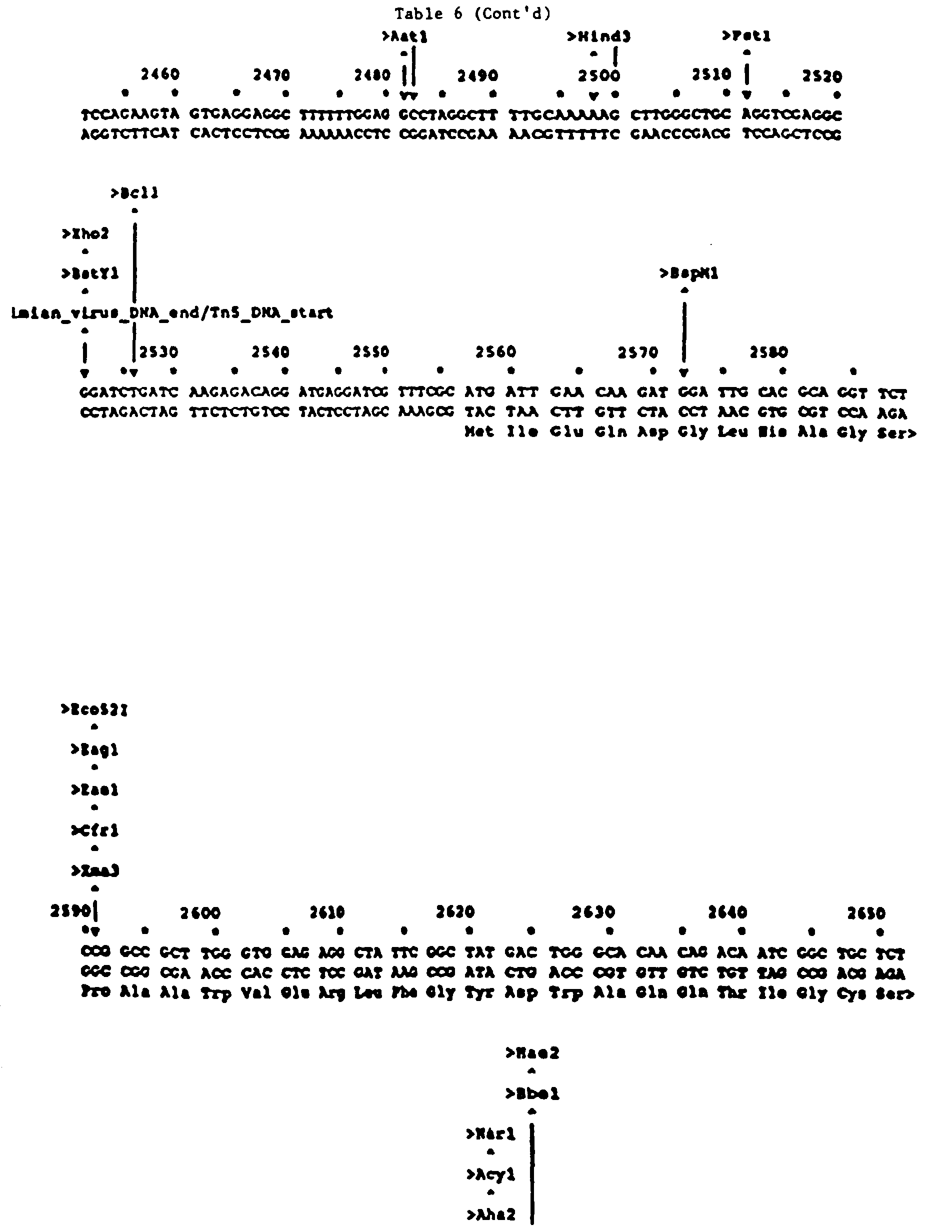

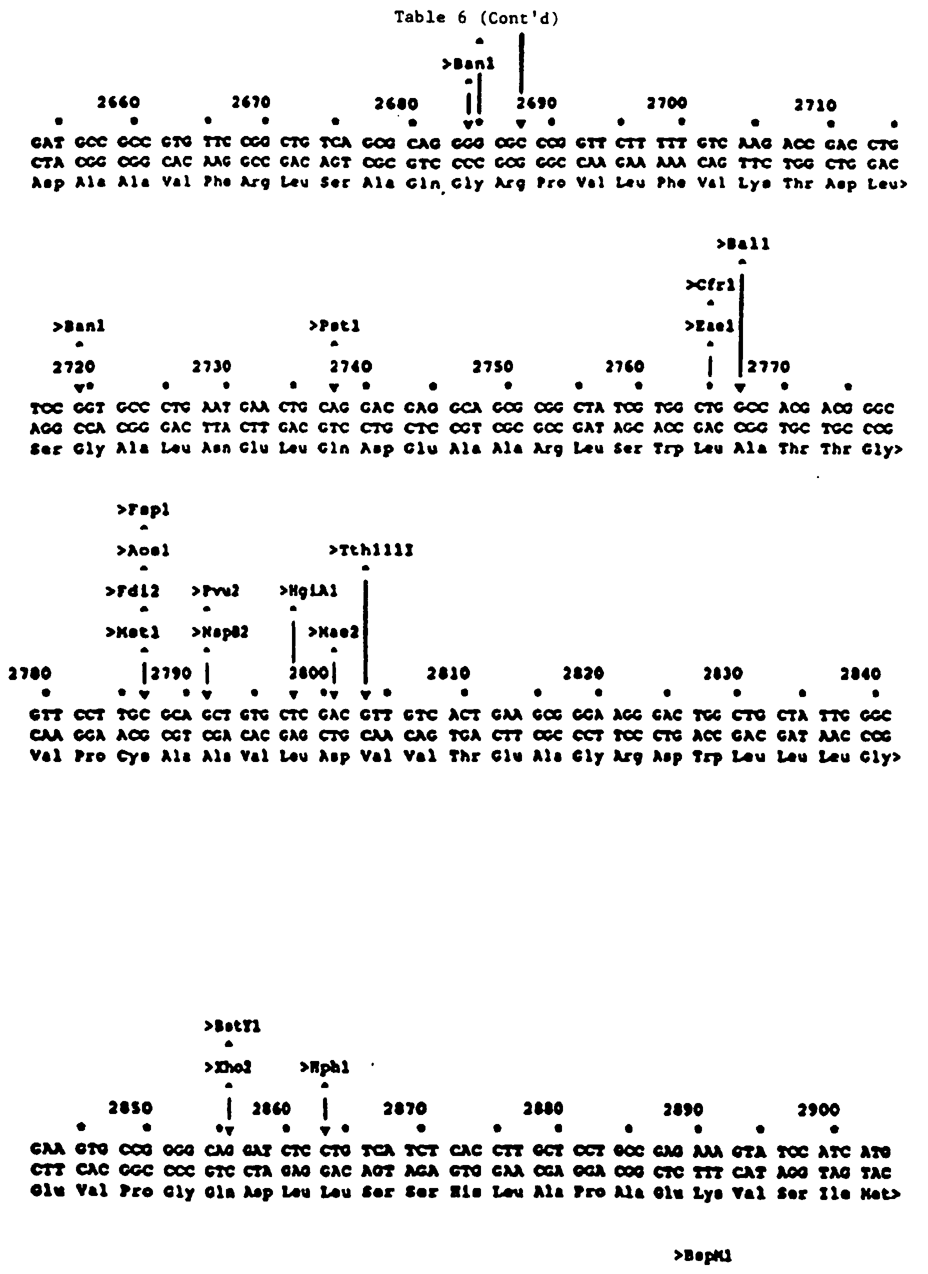

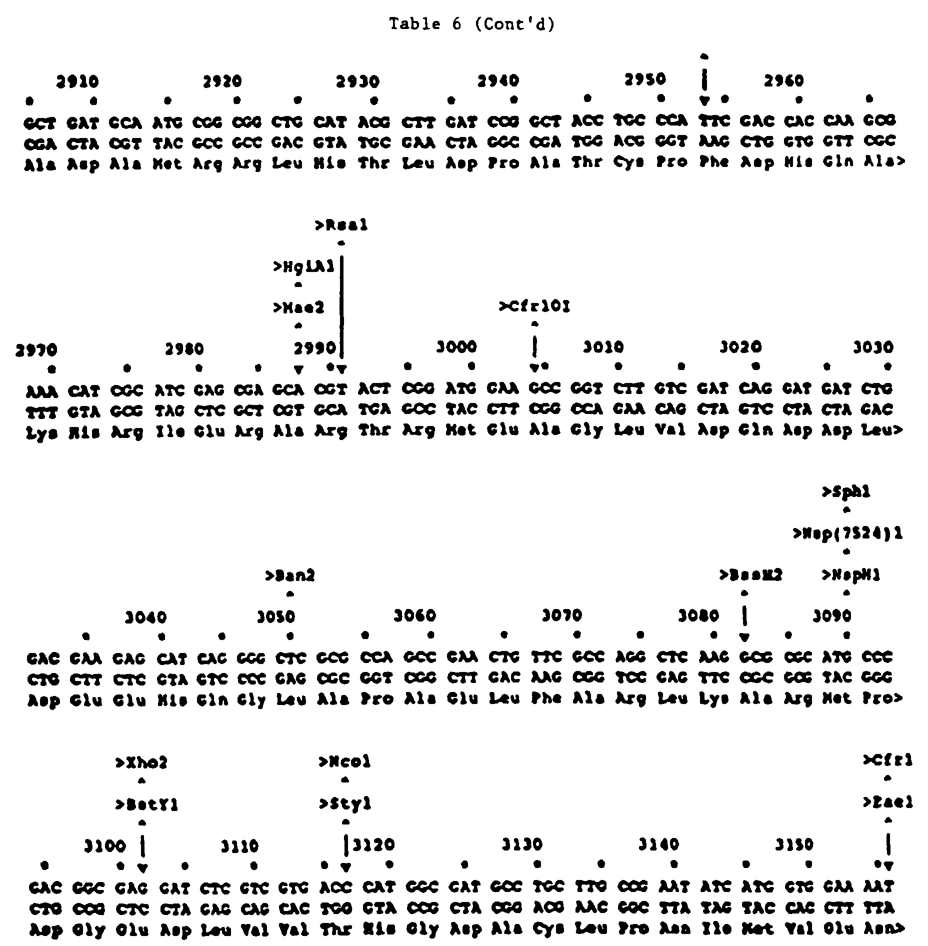

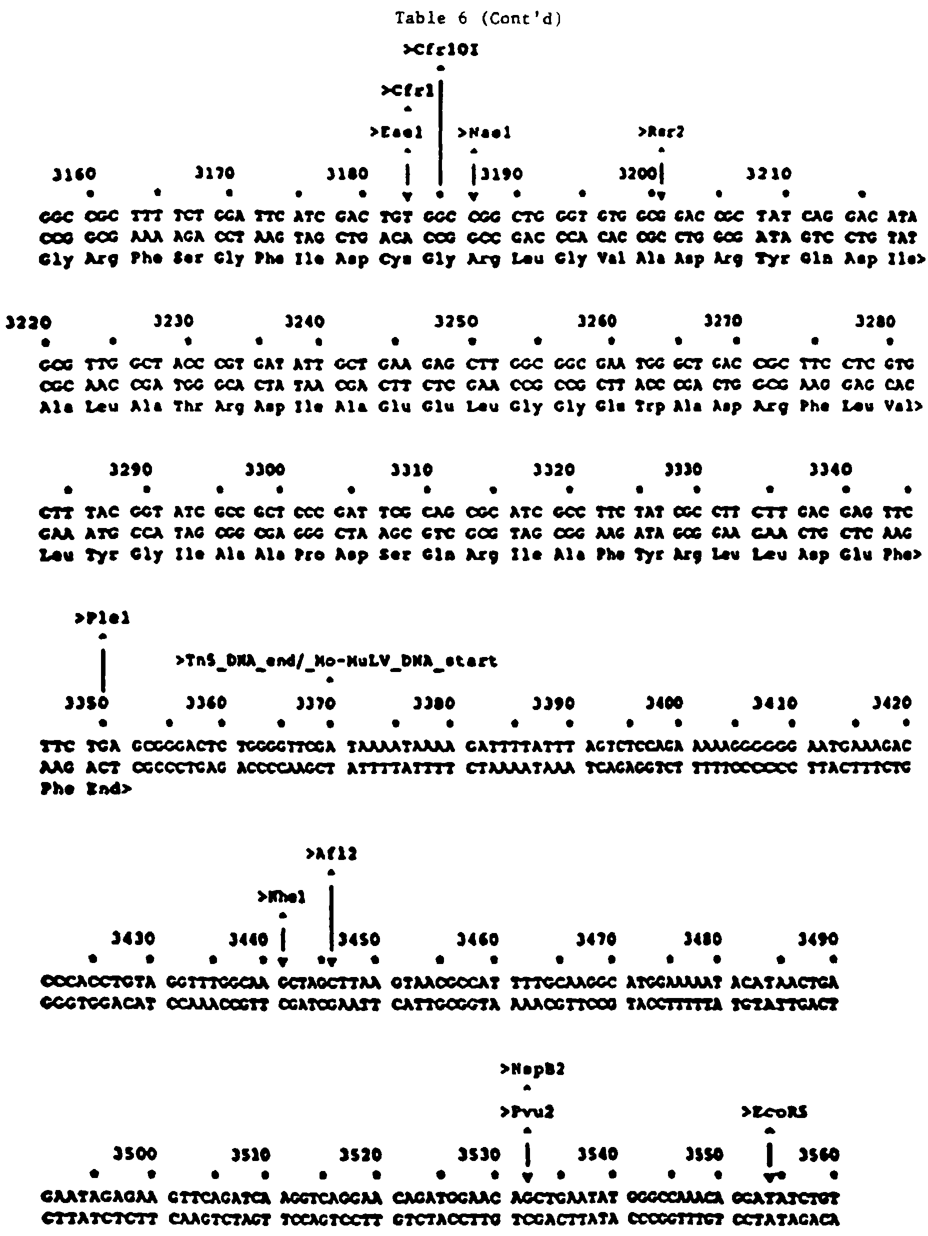

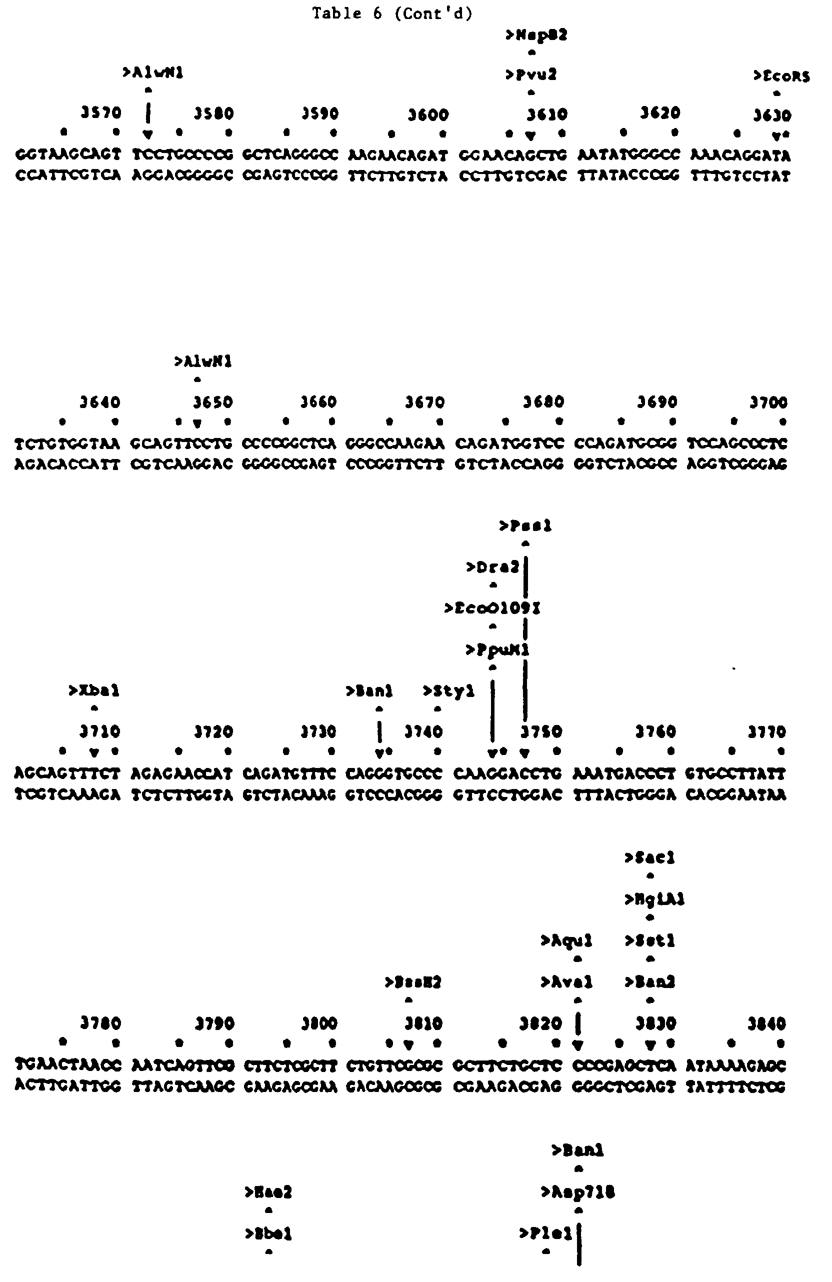

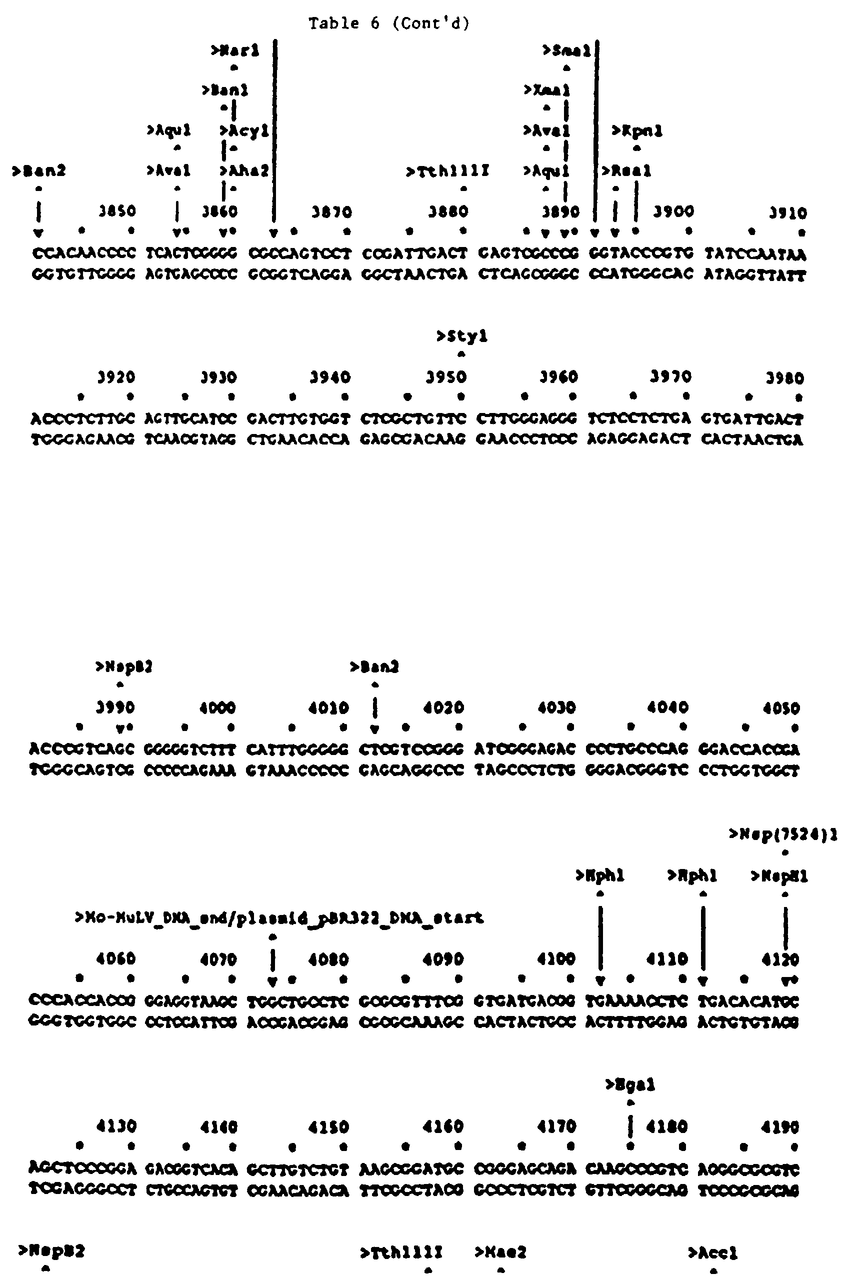

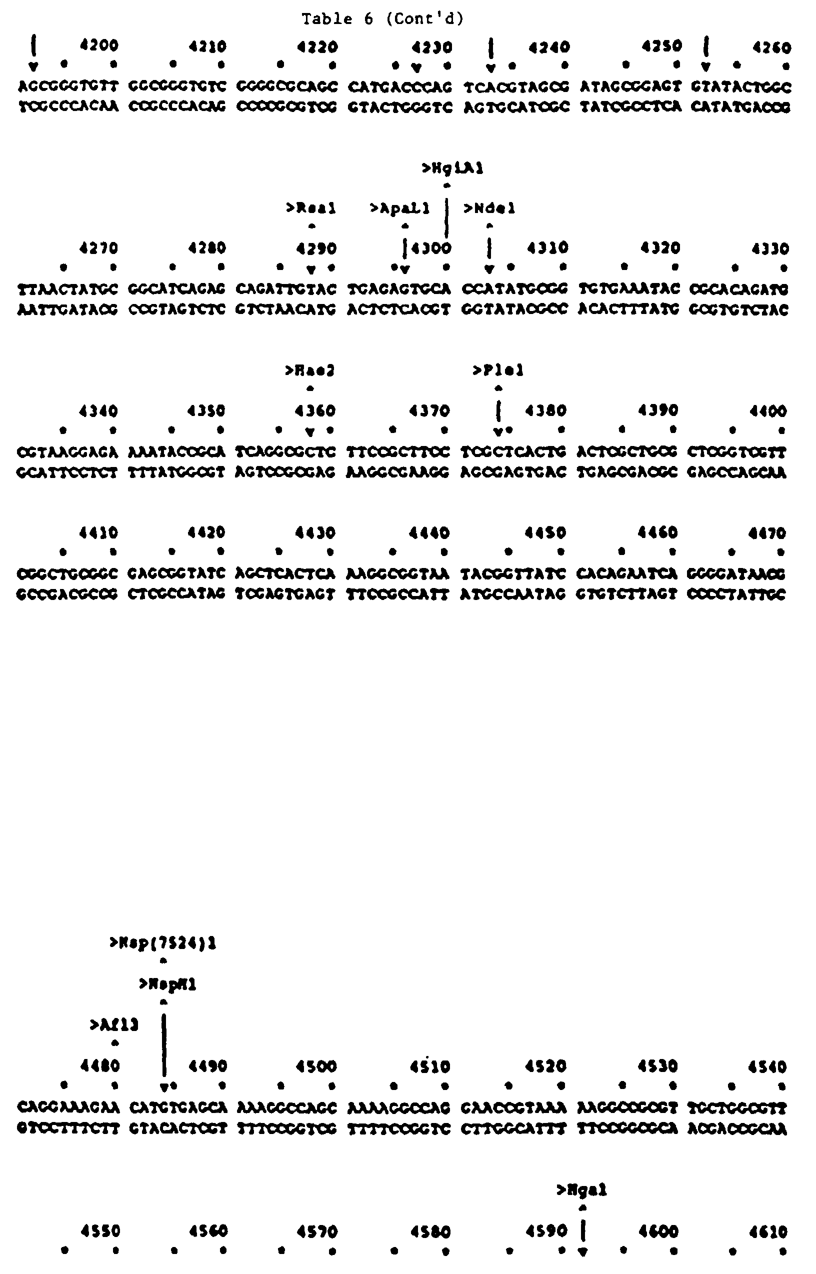

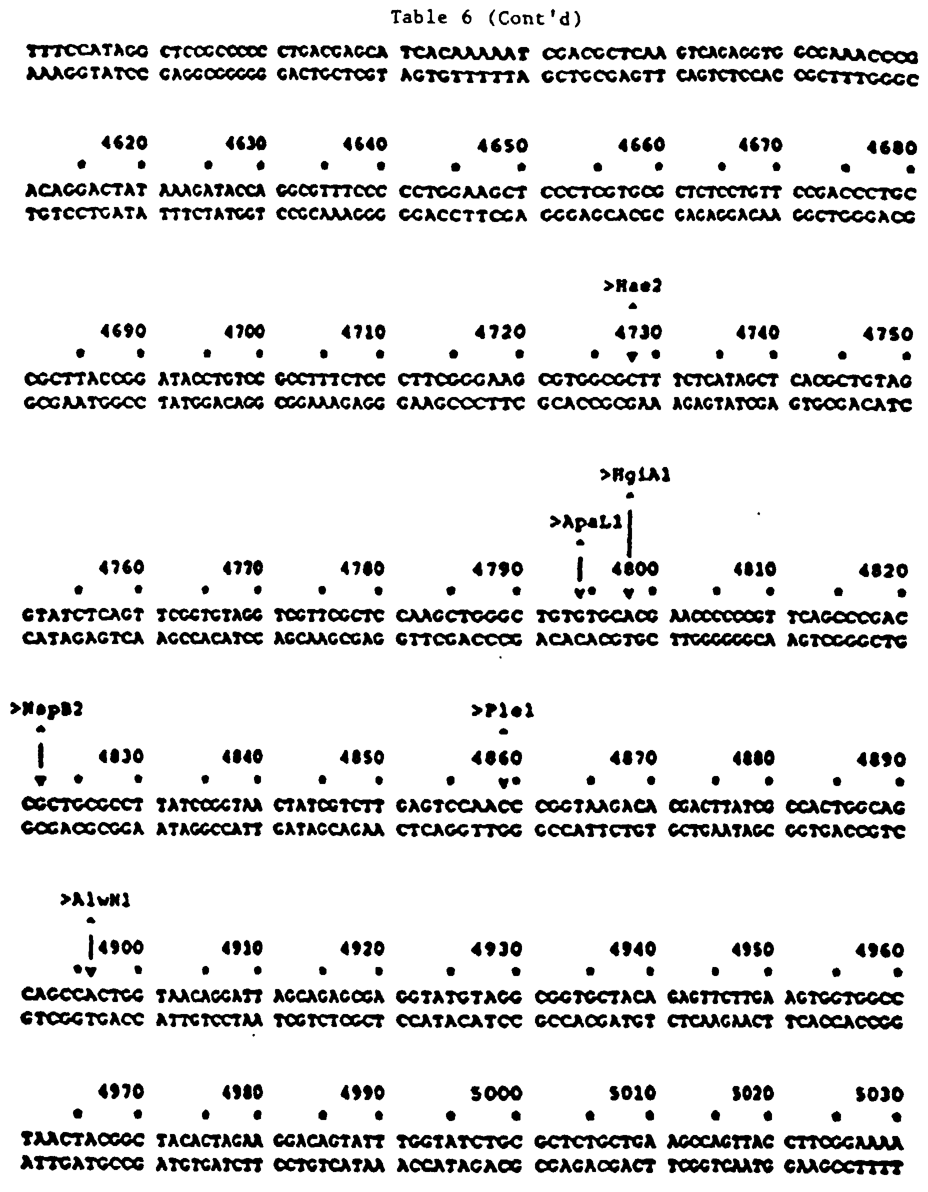

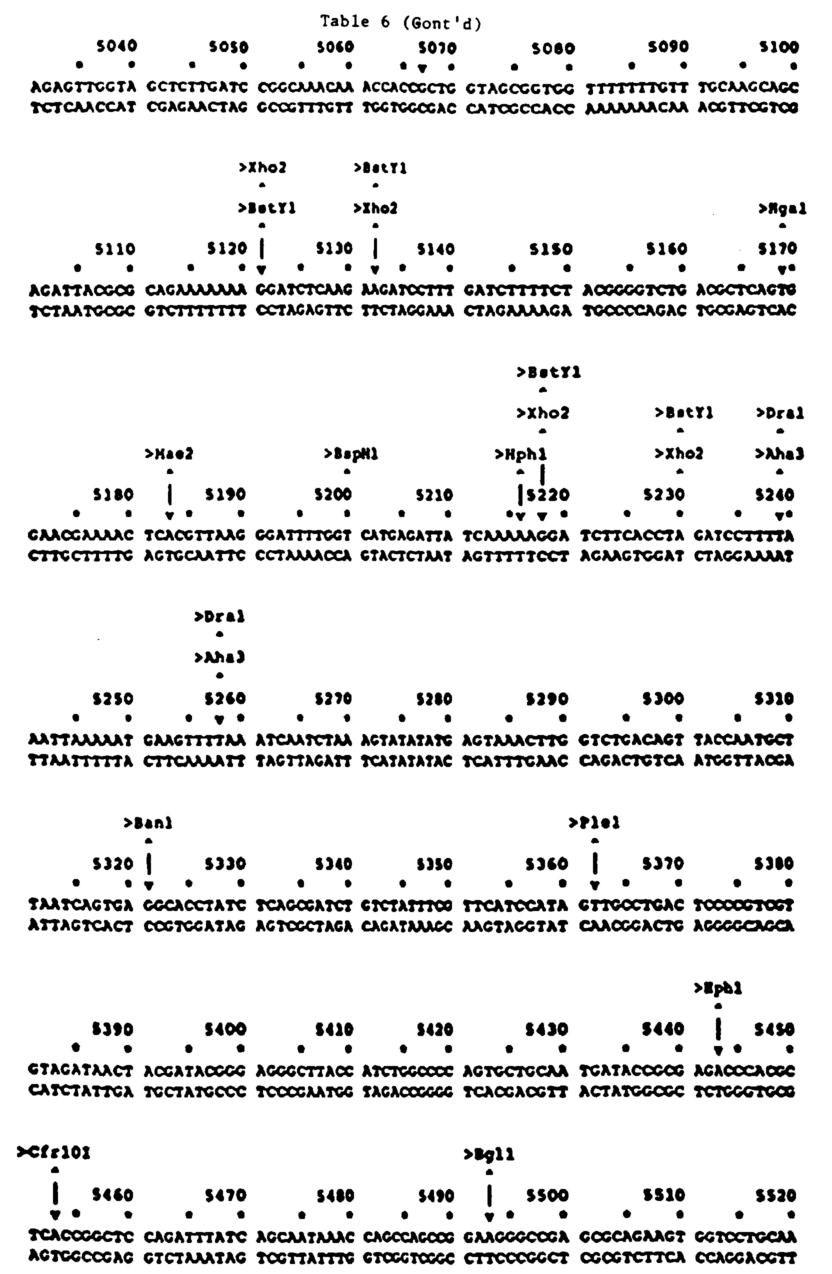

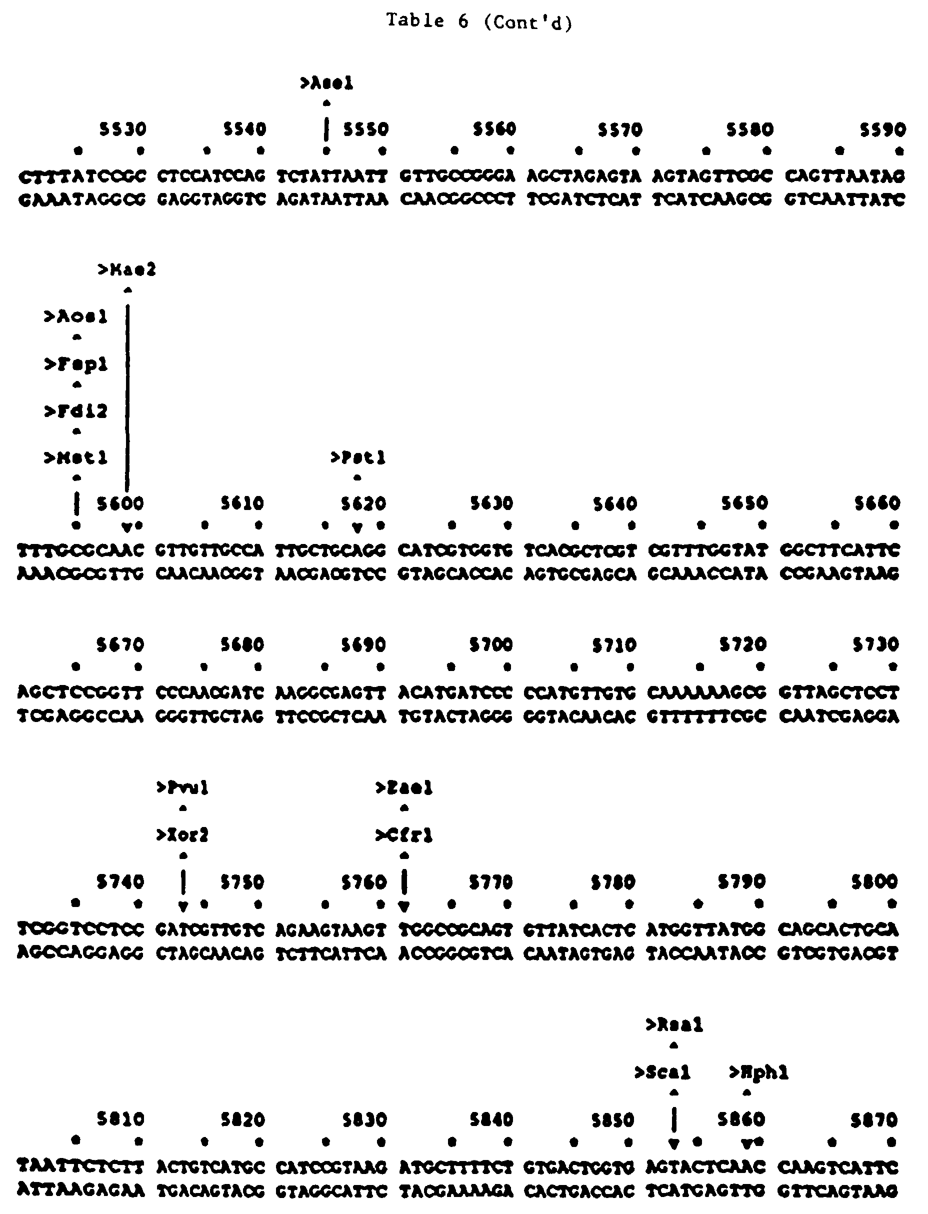

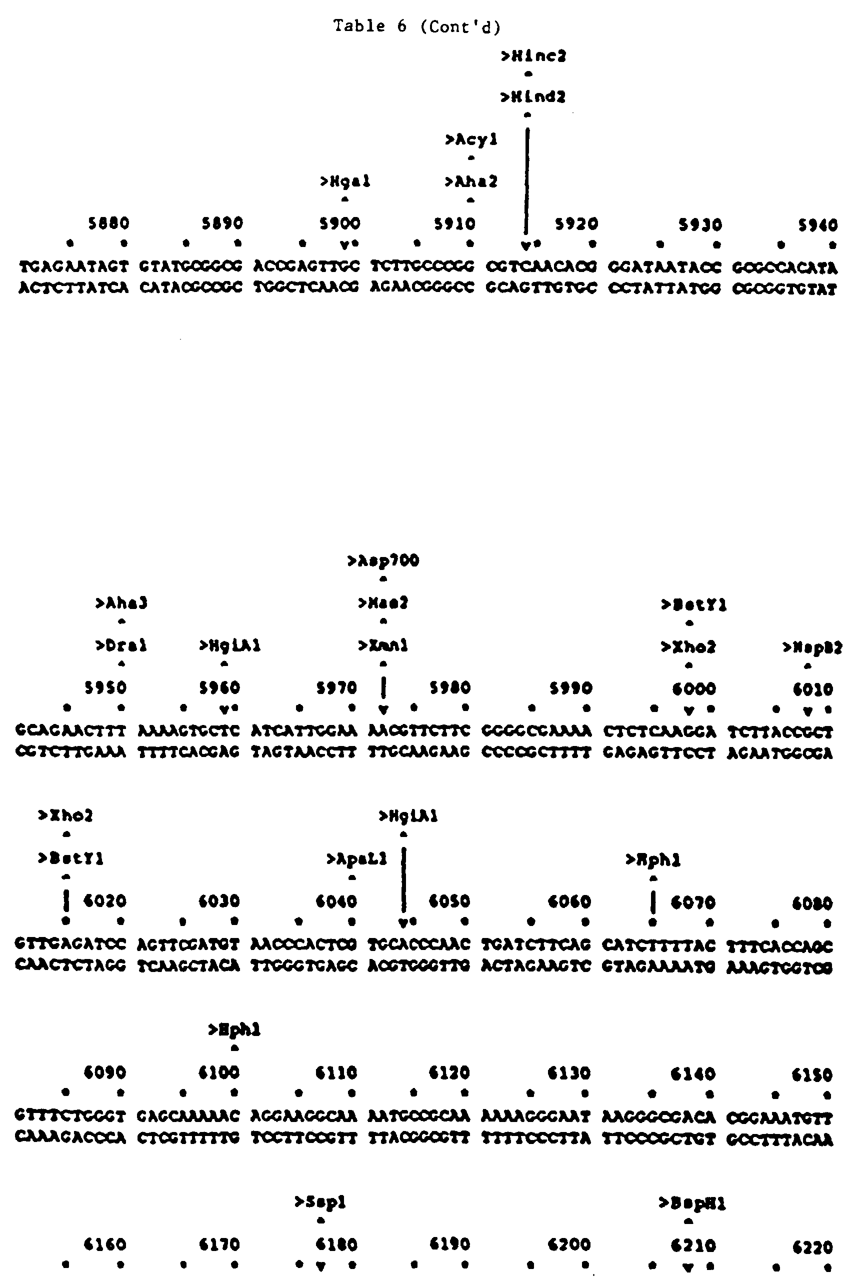

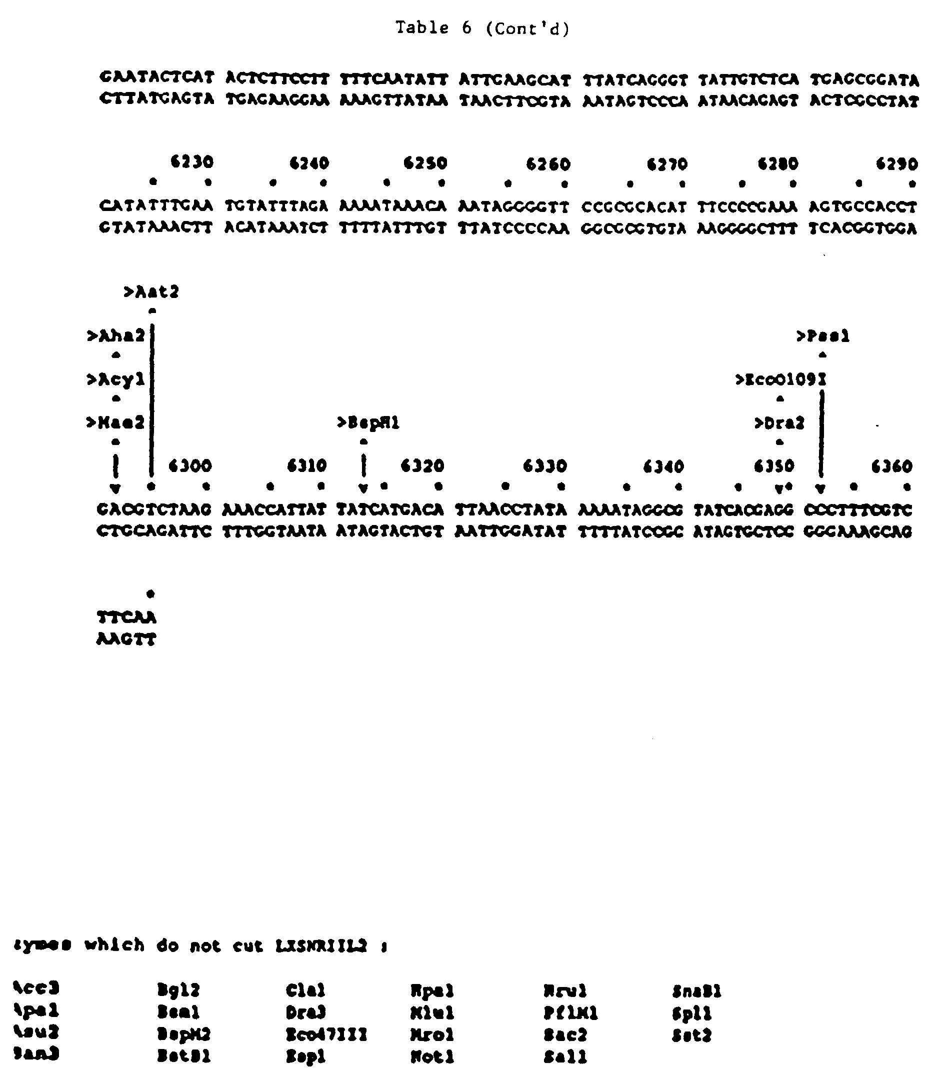

To generate the LXSN-RI-IL2 retroviral vector, 10 micrograms of pLXSN-RI-IL2 DNA was transfected into the ecotropic packaging cell line PE501 by standard calcium phosphate precipitation methods (Miller et al., Mol. Cell Biol. 6:2895, 1986). The transfected PE501 cell line was grown in DMEM medium with 10% FCS. The medium was changed after 24 hours and supernatant harvested 24 hours later to infect the amphotropic packaging cell line PA317 as described (Miller et al., Mol. Cell Biol. 6:2895, 1986 and Miller et al., BioTechniques 7:980, 1989). The infected PA317 cells were harvested by trypsinization 24 hours later and replated 1:20 in DMEM containing 10% FCS and the neomycin analogue G418 (400 µg/ml). The cells were grown at 37°C in 7% CO₂ atmosphere. The selection medium was changed every 5 days until colonies appeared. On day 14, twenty colonies were selected, expanded and tested for viral production by standard methods (Xu et al., Virology 171:331-341, 1989). Briefly, supernatants were harvested from confluent culture dishes, passed through a .45 µm filter, diluted with DMEM with 10% FCS and utilized to infect NIH 3T3 cells in the presence of 8 µg/ml polybrene. After 24 hours, the infected NIH 3T3 cells were grown in culture medium that contained the neomycin analogue G418. After 12-14 days, the colonies were stained, counted and the viral titer calculated as described (Xu et al., Virology 171:331-341, 1989).

Colonies with the highest viral titers (>10⁴ infectious units/ml) were tested for IL-2 expression by Northern blot analyses. Colonies with the highest viral titers and documented IL-2 expression were cryopreserved and will be utilized as stock cultures to produce the LXSN-RI-IL2 retroviral vector trial.

### EXAMPLE IV

### RETROVIRAL VECTOR CONSTRUCTION AND CYTOKINE EXPRESSION

To increase IL-2 production by transduced cell lines, vectors were used containing different promoters to drive IL-2 expression, and a human IL-2 cDNA was directionally sub-cloned into the insulin secretory signal peptide (17). The IL-2 cDNA was directionally sub-cloned into the parental plasmids of the LXSN (LTR promoter) and LNCX (CMV promoter) vectors (gifts of Dr. A.D. Miller) (18). The newly constructed vectors (Figure 1), designated as LXSN-IL2 and LNCX-IL2, were packaged in the PA317 cell line for production of retroviral supernatant. As a control, the high level expressing, double copy vector DC/TKIL-2 vector (thymidine kinase promoter) (a gift of Dr. E. Gilboa) was used for comparison.

These vectors were used to transduce a number of murine and human, primary and established cell lines. Pools of transduced cells were selected and expanded in DMEM medium, containing 10% fetal bovine serum (FBS) and 400 µg/ml of active G-418, a neomycin analogue. The results of expression studies in the MCR9 and Balb/c 3T3 cell lines are presented in Table 7.

**Table 7**

| Comparison of IL-2 expression by fibroblasts transduced with different IL-2 vectors. | | | |
|---|---|---|---|
| Fibroblast | Vector | ng IL-2 | Units IL-2 |
| | | per 10⁶ cells per day | |
| Murine | LNCX (Control) | 0.4 ±50% | <1 |
| | LNCX-IL2 | 33.7 ±11% | 67 |
| | LXSN-IL2 | 6.6 ± 6% | 13 |
| | DC/TKIL-2 | 1.9 ± 5% | 4 |
| Human | LXSN (Control) | 0.7 ±29% | 1 |
| | LNCX-IL2 | 159.5 ±17% | 319 |
| | LXSN-IL2 | 25.5 ±15% | 51 |
| | DC/TKIL-2 | 3.0 ±10% | 6 |

### EXAMPLE V

### FIBROBLAST CULTURE AND CONDITIONS FOR RETROVIRAL TRANSDUCTION

The culture conditions for the growth of primary fibroblasts retroviral transduction were optimized. Primary fibroblasts were successfully cultured. The optimal conditions enable the growth of approximately 3-4 x 10⁶ primary fibroblasts from a 12 mm² skin biopsy in approximately 4-6 weeks. Retroviral infection, G418 selection, and expansion of the genetically modified fibroblasts takes an additional 4-6 weeks.

Exploring the conditions for genetic modification of primary fibroblasts suggests that optimal transduction may be obtained by the following procedure: The fibroblasts are synchronized in G1 phase by serum starvation, followed by stimulation with medium containing 15% fetal bovine serum 15 hours prior to transduction. The cells are then subjected to 2 cycles of retrovirus infection, each cycle lasting approximately 3 hours. The cells are refed with fresh media overnight, and then selection in G418 is initiated the next day. This method is capable of transducing 5-15% of the fibroblasts in a culture, depending on the multiplicity of infection.

This procedure was used to transduce a large number of primary and established fibroblasts. As an example, Table 8 compares the expression levels of IL-2 in fibroblast lines transduced with LXSN-IL2.

**Table 8**

| Expression of IL-2 by fibroblasts transduced with LXSN-IL2. | | | | |
|---|---|---|---|---|
| Fibroblast Line | Species | Origin | no IL-2 | Units IL-2 |
| | | | per 10⁶ cells per day | |
| Balb/c 3T3 | Murine | Transformed | 6.6 ± 6% | 13 |
| MCR9 | Human | Embryonic | 25.5 ±15% | 51 |
| NHDF 313 | Human | Skin | 25.0 ±10% | 50 |
| GT1 | Human | Skin | 15.0 ± 5% | 30 |

These results indicate that the IL-2 expression levels in established, embryonic, and primary fibroblast cultures are similar. Comparison of these data with Table 7 suggest that IL-2 expression is affected more by factors such as different promoters than by the fibroblast line used. Similarly, changes in culture conditions can have important effects on IL-2 expression. Table 9 shows that transduced GT1 cells, a primary human fibroblast culture expressed 15-fold more IL-2 under 100 µg/ml G418 selection than under 25 µg/mi G418 selection. Several other primary fibroblast lines have also been transduced with our vectors and are currently growing under G418 selection.

**Table 9**

| Effect of G418 concentration on IL-2 expression by GT1 cells transduced with LXSN-IL2. | |
|---|---|
| Selection dose of G418 | ng IL-2 secreted |
| | per 10⁶ cells per day* |
| 25 µg/ml | 1.0 ± 10% |
| 50 µg/ml | 3.0 ± 6% |
| 100 µg/ml | 15.0 ± 5% |

| | |
|---|---|
| ^{*}After three weeks of G418 selection. | |

### EXAMPLE VI

### COMPARISON OF IL-2 EXPRESSION LEVELS INDUCED PERIPHERAL BLOOD LYMPHOCYTES AND GENETICALLY MODIFIED FIBROBLASTS

In order to compare the production of IL-2 by genetically modified fibroblasts to that achieved by stimulating normal human peripheral blood lymphocytes (nPBL) in vitro, nPBL were isolated by Ficol-Paque density centrifugation, and cultured in the presence of allogeneic nPBL (mixed lymphocyte culture, MLC) or 2 µM calcium ionophore (CI) (A23187) free acid) plus 17 nM phorbol 12-myristate 13-acetate (PMA). The results of this experiment, present in Table 10, indicate that the level of IL-2 expression in the PMA/CI stimulated normal T cell population was 2 ng/10⁶ cells/24 hours. This is equivalent to IL-2 expression by Balb/c 3T3 fibroblasts transduced with DC/TKIL-2 (Table 7), our least productive vector. The level of IL-2 expression in the MLC was 130 pg/10⁶ cells/24 hours. This was lower than the PMA/CI stimulated culture, presumably because PMA/CI induced a nonspecific response while MLC resulted in specific Th stimulation. When the estimated percentage of antigen-specific Th in the MLC-stimulated population is taken into consideration, the level of IL-2 expression per stimulated T cell becomes equivalent for both methods.

**Table 10**

| Levels of IL-2 secretion by different cells. | |
|---|---|
| Cells | pg IL-2 secreted |
| | per 10⁶ cells per day |
| Lymphocytes: | |
| Control (non-activated) | 5 ± 50% |
| PMA + Calcium Ionophore | 2,000 ± 6% |
| Mixed lymphocyte culture | 130 ± 90% |

| Transduced fibroblasts: | |
|---|---|
| MCR9-LXSN-IL2 | 24,000 ± 5% |
| MCR9-LNCX-IL2 | 162,000 ± 20% |
| MCR9-DC/TKIL-2 | 10,000 ± 6% |

### EXAMPLE VII

### FIBROBLAST MEDIATED CYTOKINE GENE THERAPY IN MURINE TUMOR MODELS

Two experimental protocols were used to study the efficacy of fibroblast-mediated cytokine gene therapy on induction of anti-tumor immunity. The first protocol was designed to test the effects of genetically modified fibroblasts on tumor implantation, while the second protocol was designed to induce a systemic anti-tumor immunity. The results of each experiment are presented with two figures and one table. In the first figure, the rate of tumor growth for each treatment group is presented as the mean tumor size in the group over time. In the second figure, a Kaplan-Meier curve presents the time of tumor onset for the individual animals in each treatment group. The number of animals, the number and percentage of tumor free animals, and the tumor size distribution patterns for each experiment are presented in a table.

### EXAMPLE VII(a)

### EFFECT OF FIBROBLAST MEDIATED CYTOKINE GENE THERAPY ON TUMOR IMPLANTATION

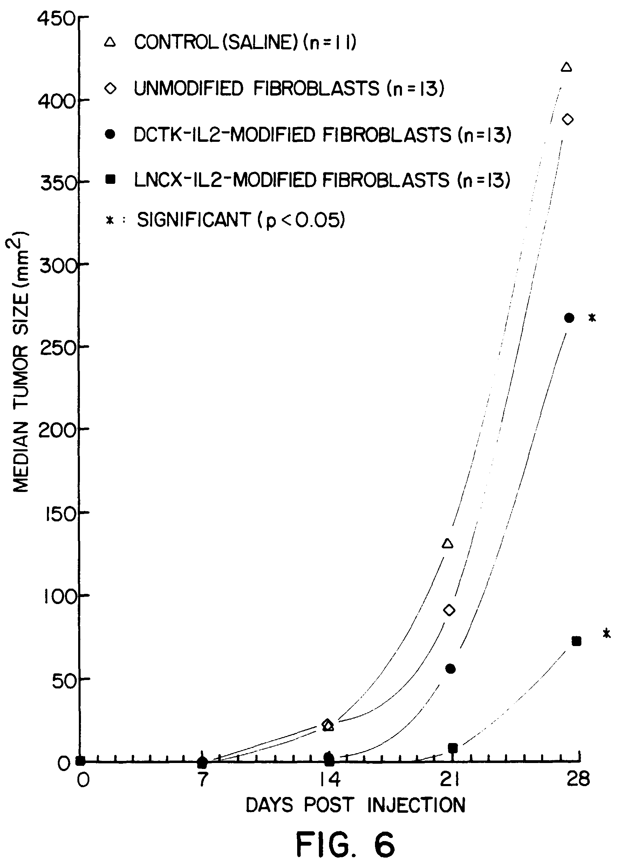

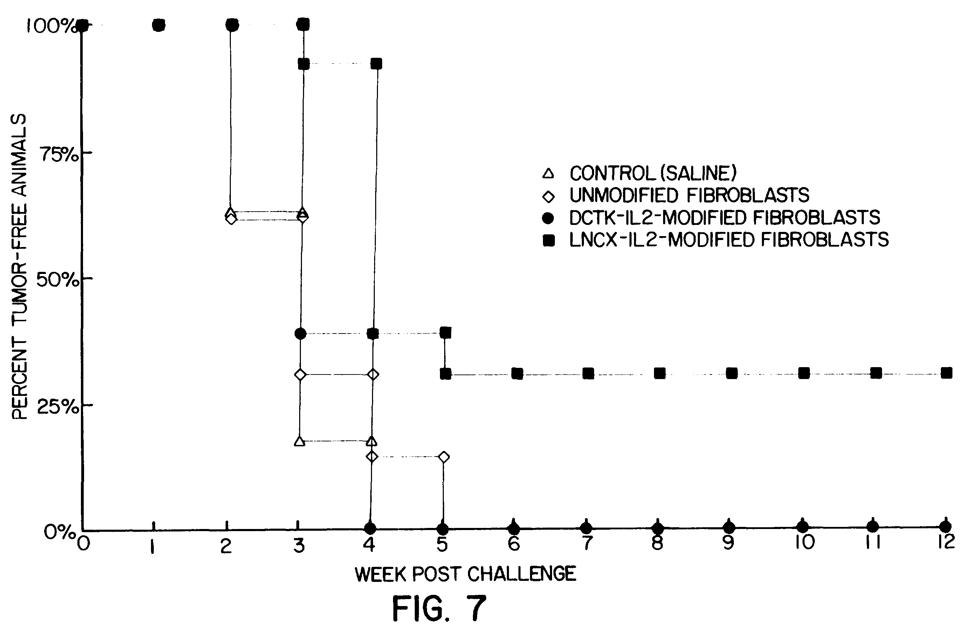

Mice were injected subcutaneously with mixtures of 5 x 10⁴ CT26 cells and 2 x 10⁶ fibroblasts genetically modified by different retroviral vectors to express IL-2. In the control arms injected with tumor cells only, or with tumor cells mixed with unmodified fibroblasts, 31 of 33 animals (94%) developed tumors by 4 weeks (Figures 6 and 7, Table 9). In contrast, 22 out of the 34 animals (65%) receiving fibroblast mediated cytokine gene therapy were tumor free at 3 weeks, and 5 animals (18%) remain tumor free after 12 weeks. Those animals that received fibroblast mediated IL-2 therapy and developed tumor were characterized by a delayed onset and rate of tumor growth.

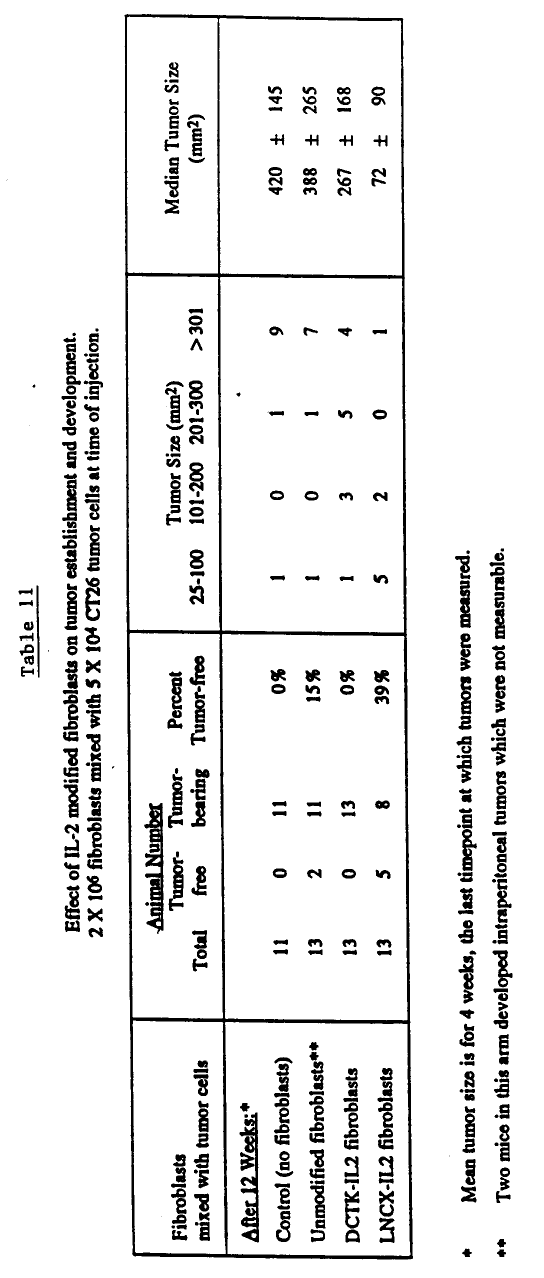

After 3 weeks the mean tumor size (measured as the product of the longest and widest tumor axes) in the control group of mice was 128 mm², compared to 68 and 7 mm² in groups of mice injected with tumor cells mixed with fibroblasts transduced with DC/TKIL-2 or LNCX-IL2, respectively. This resulted in a highly significant difference (corrected x² = 18.69, p = 0.001) between the IL-2 treated animals compared to the mice treated with CT26 alone or CT26 mixed with unmodified fibroblasts. After four weeks the equivalent measurements were 373,300 and 72 mm² (Table 11). It is notable that LNCX-IL2, the highest expressing vector caused substantially greater inhibition of tumorigenicity than the lower expressing vector DC/TKIL-2. A multivariate non-parametric statistical procedure (19,20), utilized to evaluate differences in tumor growth, demonstrated that after 4 weeks the differences between the growth curves for the four groups presented in Figure 2 were highly significant (p < 0.001). Subsequent comparisons between the control arm and animals that received tumor cells mixed with IL-2 transduced fibroblasts revealed a significant difference (P < 0.05). The differences between the animals injected with tumor cells alone, and those injected with tumor cells plus unmodified fibroblasts were not significant, while the differences between animals receiving low IL-2 expressing fibroblast, and those receiving high IL-2 expressing fibroblasts was significant (P = 0.05).

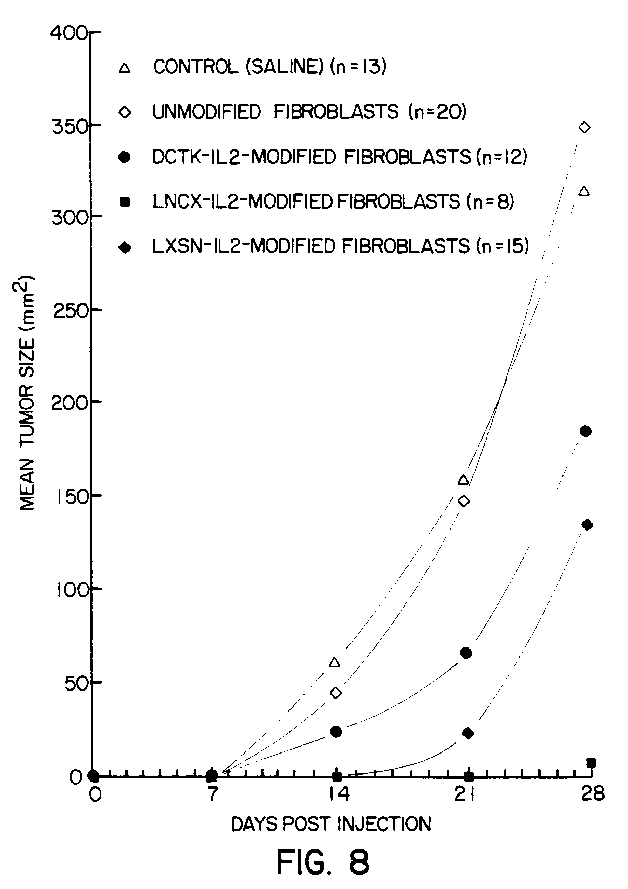

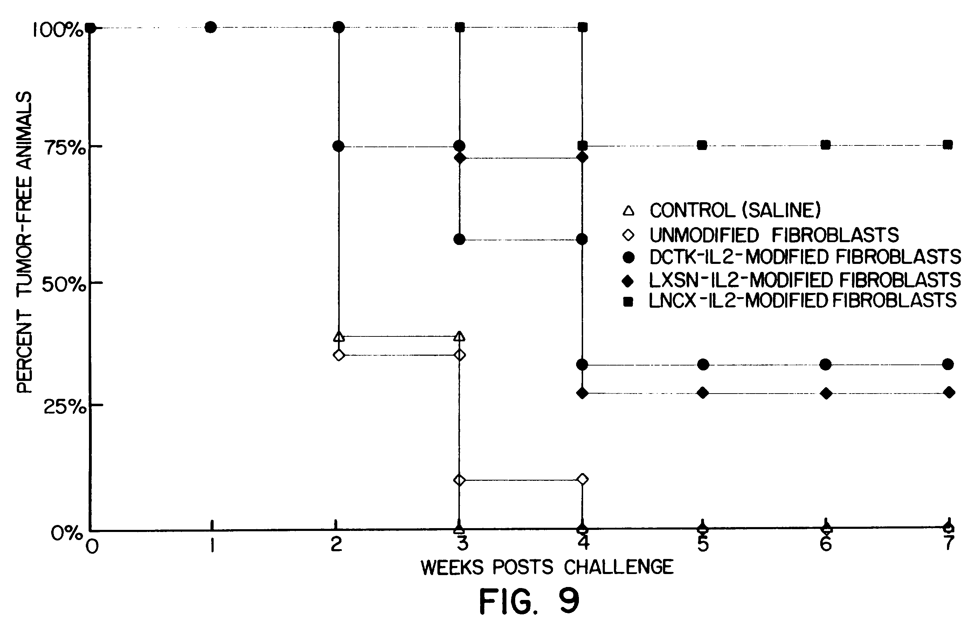

When mice were injected with 2 x 10⁶ modified fibroblasts mixed with 1 x 10⁵ live tumor cells the results became more striking (see Figures 8 and 9, and Table 12). All the control animals developed tumors after 4 weeks whereas 33% and 27% of the animals treated with fibroblasts modified with the DCTK-IL2 or LXSN-IL2 vectors (respectively) remain tumor free after 7 weeks (the experiment is ongoing). More dramatically, 75% of the animals treated with fibroblasts modified with the highest IL-2 producing vector, LNCX-IL2, remain tumor free after 7 weeks. These data clearly demonstrate the importance of an initial high dose of IL-2 to prevent tumor establishment.

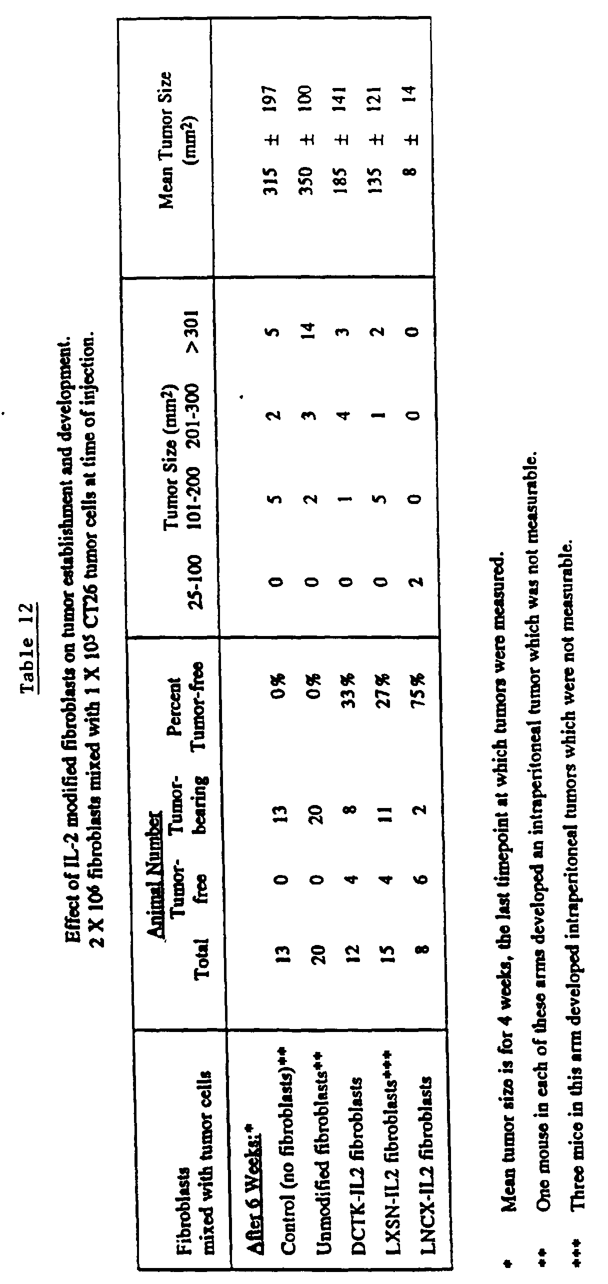

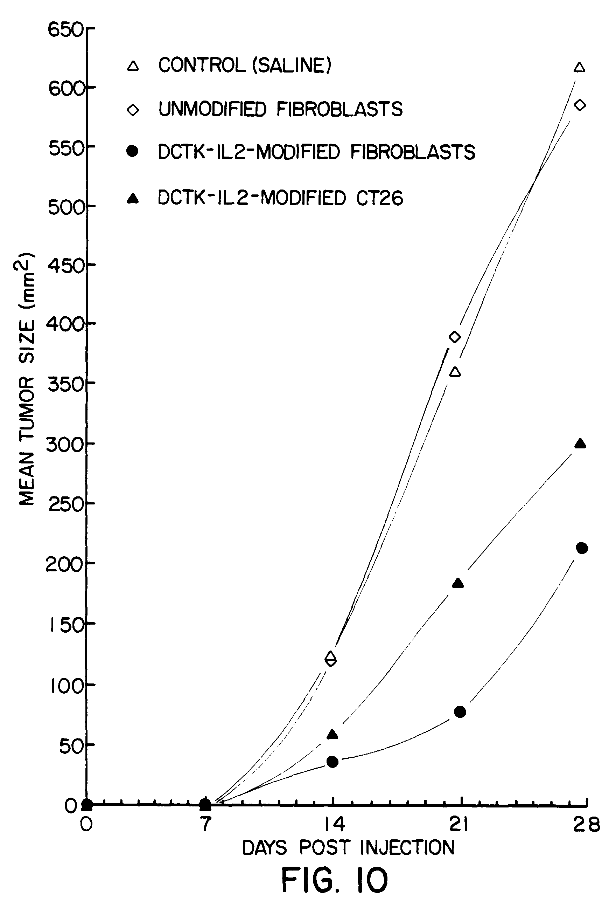

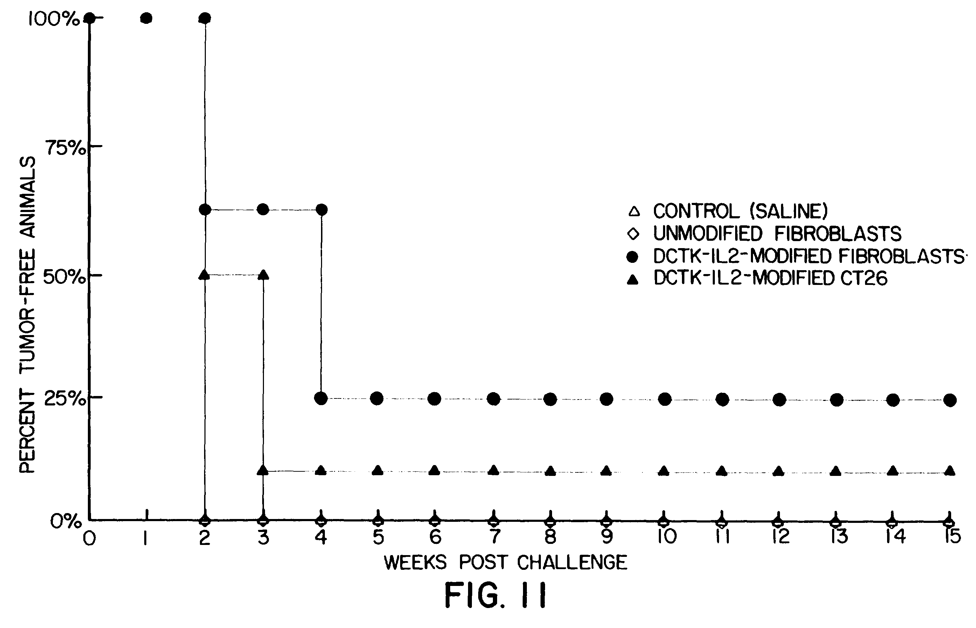

As an additional control, mice were injected with CT26 cells genetically modified to express IL-2 (results not shown). Injection of up to 1 x 10⁶ IL-2 expressing tumor cells into Balb/c mice failed to produce tumors. Injection of higher numbers however, resulted in some animals developing tumors with delayed onset. These data confirm the results reported in the literature (1). In order to compare the efficacy of IL-2 producing fibroblasts to IL-2 producing tumor cells, we mixed 2 x 10⁶ CT26 tumor cells modified with the DCTK-IL2 vector with 1 x 10⁵ unmodified tumor cells. Figures 10 and 11, and Table 13 show that DCTK-IL2 modified tumor cells are somewhat effective in preventing tumor development. Four weeks after injection, the mean tumor size for the treatment arm is 303 mm², compared to 620 mm² for the control arm. After 22 weeks, one animal (10%) remains tumor free, compared to none in the control arms. Data for animals treated under the same conditions with DCTK-IL2 modified fibroblasts in a separate experiment are included for comparison purposes. This comparison suggests that DCTK-IL2 modified tumor cells have an effect on tumor establishment similar to that of DCTK-IL2 modified fibroblasts.

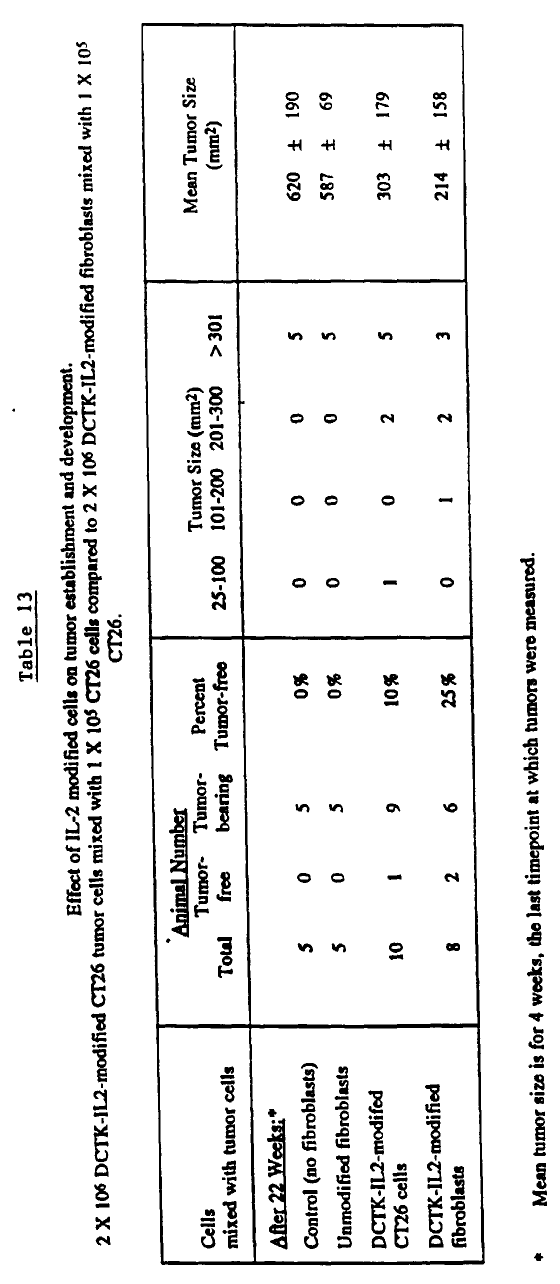

### EXAMPLE VII(b)

### EFFECT OF FIBROBLAST MEDIATE CYTOKINE GENE THERAPY ON SYSTEMIC ANTI-TUMOR IMMUNITY

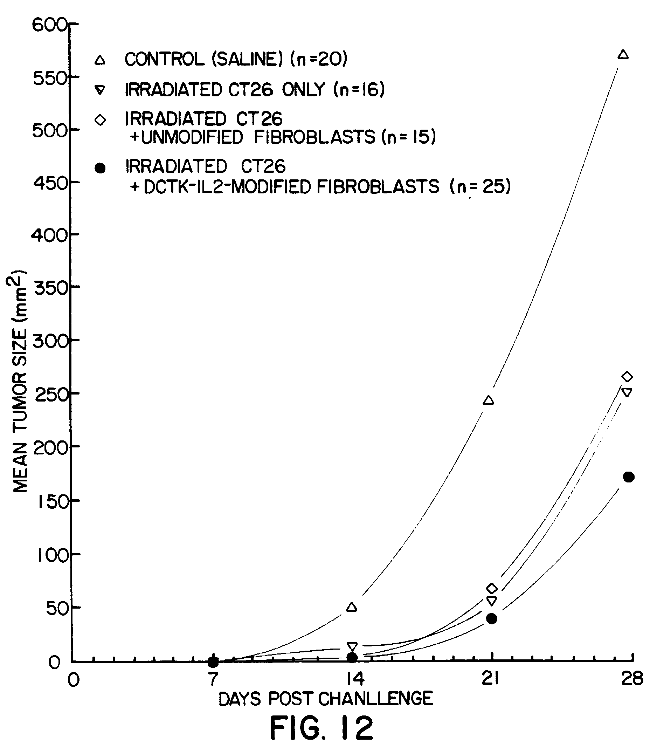

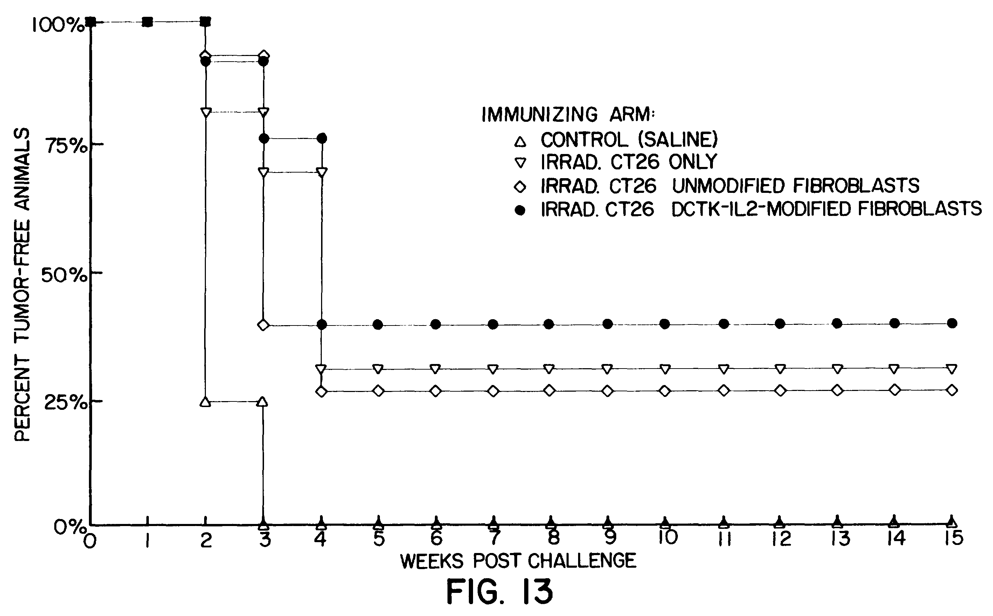

Groups of Balb/c mice were immunized with 2.5 x 10⁵ irradiated tumor cells either alone or mixed with 2 x 10⁶ transduced or unmodified fibroblasts, and challenged one week later with 5 x 10⁴ live tumor cells in the opposite flank. These results (Figures 12 and 13, and Table 14) demonstrate that immunization with irradiated tumor cells and transduced fibroblasts protect some animals against a live tumor challenge, but that the protection is only slightly better than that achieved by immunization with irradiated tumor cells alone or irradiated tumor cells mixed with unmodified fibroblasts.

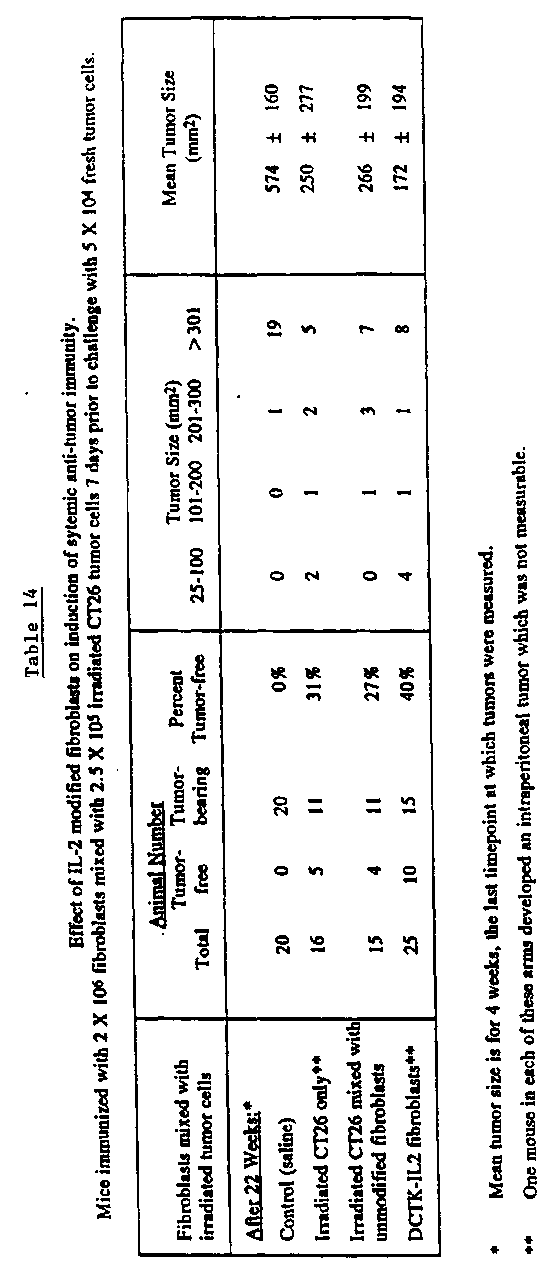

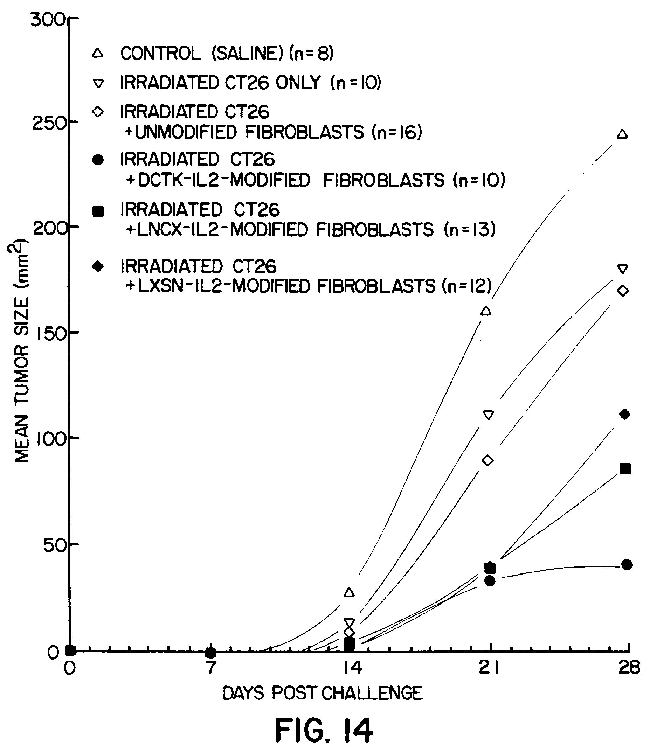

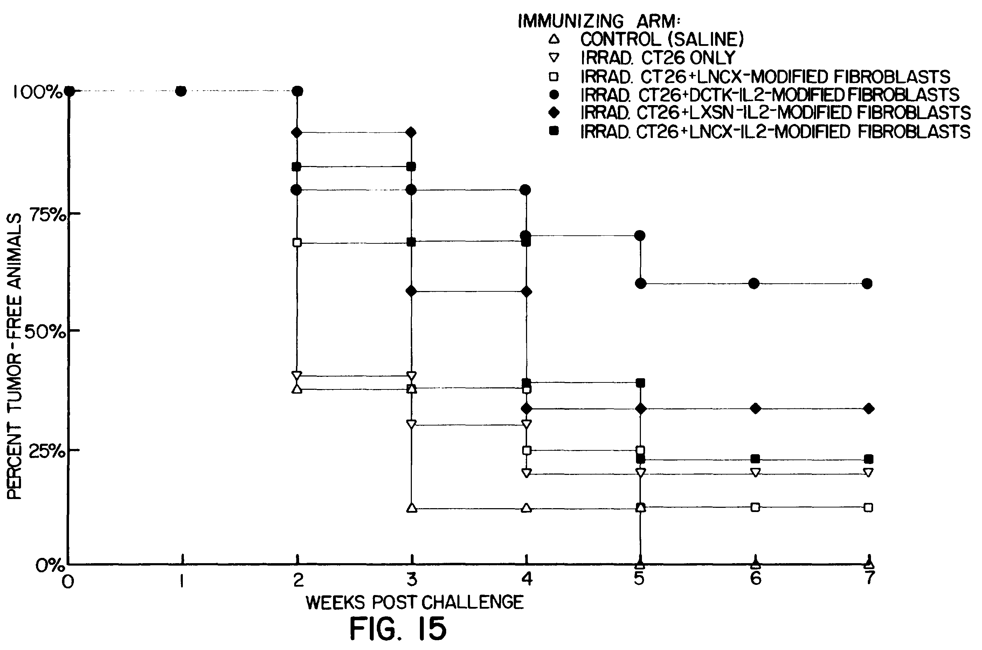

In a second protocol similar to the one described above, animals were challenged with fresh tumor cells two weeks following immunization with irradiated tumor cells mixed with fibroblasts. The results, shown in Figures 14 and 15, and in Table 15, demonstrate that DCTK-IL2 modified fibroblasts mixed with irradiated tumor cells confers superior protection to subsequent tumor challenge than irradiated tumor cells alone, irradiated tumor cells mixed with unmodified fibroblasts, or irradiated tumor cells mixed with LNCX-modified fibroblasts. After 7 weeks, seven of ten animals (70%) treated with DCTK-IL2 modified fibroblasts remain tumor free compared to only one third of the control animals. At four weeks, the mean tumor size of this group was 41 mm², compared to 180, 170, and 140 mm² for the three control groups. Animals treated with LNCX-IL2 modified fibroblasts were also protected against subsequent tumor challenge, but the results were less striking. In this group, 54% of the animals remain tumor free and the mean tumor size for the group at four weeks was 86 mm². The number of tumor free animals in the group treated with LXSN-IL2 modified fibroblasts was similar to the control groups, although the tumors were slightly delayed in their onset. A multivariate non-parametric statistical procedure (19, 20), utilized to evaluate differences in tumor onset, demonstrated that the differences for the six arms presented in Figure 15 were significant (p = 0.012). It further showed that the saline control arm and the arms that received irradiated tumor cells alone or mixed with unmodified or LNCX vector modified fibroblasts formed a statistical group. A second, distinct statistical group was formed by the three arms that received IL-2 vector modified fibroblasts mixed with irradiated tumor cells. Subsequent comparisons between the saline injected control arm and animals that received tumor cells mixed with IL2 transduced fibroblasts revealed a significant difference for all vectors (p < 0.05).

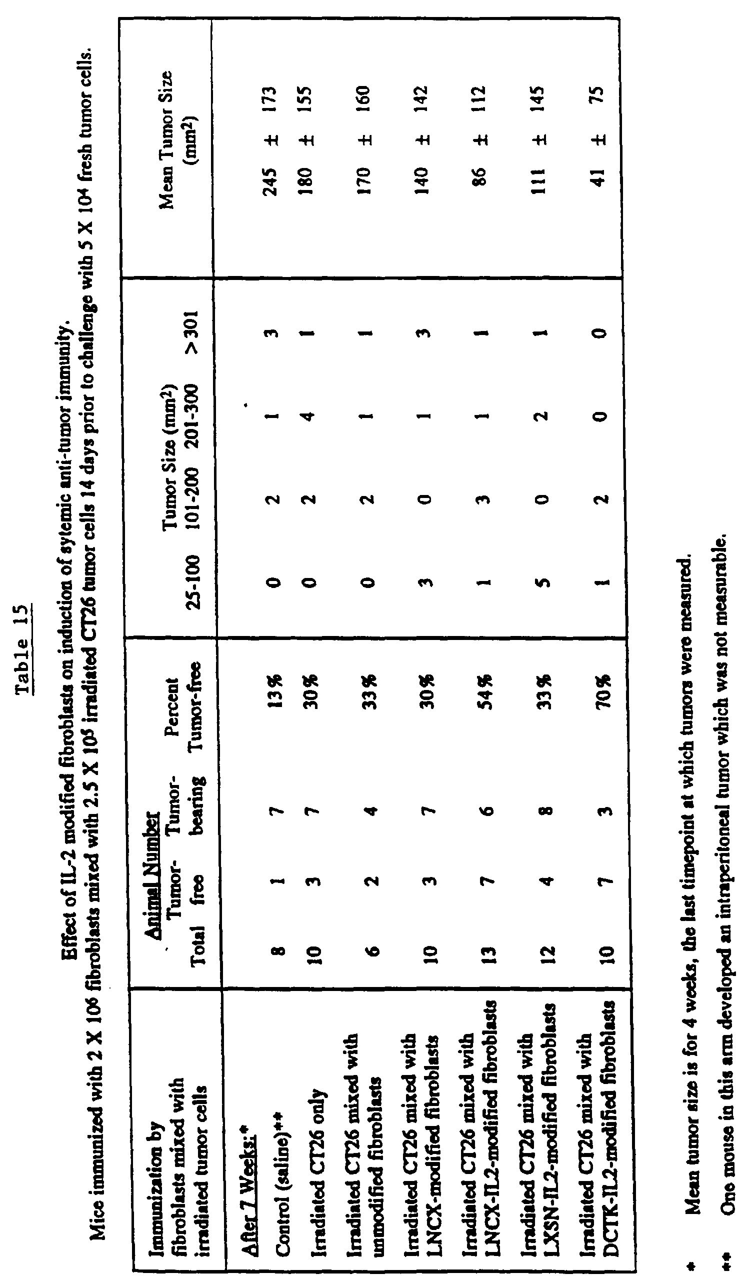

These results demonstrate the feasibility of using genetically modified fibroblasts as a means of delivering cytokine gene therapy. In all experiments, the LNCX-L2 vector proved superior in preventing tumor establishment while the DCTK-IL2 vector was better in the induction of systemic protection against subsequent tumor challenges. These contrasting effects, although somewhat surprising, can be explained by the observation that the CMV promoter is turned off in vivo five days after implantation while the TK promoter remains active for a longer period of time. The implication of this finding is that to apply this method of gene therapy successfully we have to use promoters that result in high level, sustained expression of IL-2 in vivo in the transduced fibroblasts.

The data obtained from this research effort has important implications for all cytokines that have either direct or indirect anti-tumor effects. Furthermore, this data suggests that anti-tumor efficacy is IL-2 dose dependent. Hence, construction of vectors which result in higher levels of cytokine secretion will be a significant advance toward the application of this method of gene therapy.

### References

1. Gabrilove, J.L. et al., Monogr. J. Natl. Cancer Inst. 10:73-7 (1990).
2. Kelso, A., Current Opinion in Immunology, 2:215-25 (1989).
3. Borden, E.C. et al., Cancer, 65:800-14 (1990).
4. Rosenberg, S.A. et al., Ann. Intern. Med., 108:853-864 (1988).
5. Lotze, M.T. et al., JAMA, 256:3117-3124 (1986).
6. Pizza, G. et al., Lymphokine Research, 7:45-8 (1988).
7. Sarna, G. et al., Journal of Biological Response Modifiers, 9:81-6 (1990).
8. Gandolfi, L. et al., Hepato-Gastroenterology, 36:352-6 (1989).
9. Bubenik, J. et al., Immunol. Letters, 19:279-82 (1988).
10. Bubenik et al., Immunol. Letters, 23:287-292 (1990).
11. Fearon, E.R. et al., Cell, 60:387-403 (1990).
12. Gansbacher, B. et al., J. Exp. Med., 172:1217-1224 (1990).
13. Watanabe, Y. et al., Proc. Natl. Acad. Sci., 86:9456-9460 (1989).
14. Tepper, R.I. et al., Cell, 57:503-512 (1989).
15. Kriegler, M., Gene Transfer and Expression: A Laboratory Manual, Stockton Press (1990).
16. Rosenberg, S.A. et al., N. Eng. J. Med., 370 (1990).
17. Cornetta, K. et al., Prog. Nucl. Acid Res. Mol. Biol., 36:311-22 (1989).
18. Hoover, H.C. et al., Cancer Res., 44:1671-76 (1984).
19. Sobol et al. New Eng. J. Med. 316:1111-1117 (1987).
20. Li Xu, et al., Virology, 171:331-341 (1989).

## Claims

1. Use of a formulation comprising a tumor antigen and cytokine-expressing cells genetically modified to express an immunostimulatory cytokine gene product, wherein said cytokine-expressing cells are not tumor cells, for the preparation of a medicament for stimulating an anti-cancer immune response in a patient, wherein said medicament is for immunizing said patient at a site other than an active tumor site.

2. The use of claim 1, wherein tumor cells previously isolated from said patient provide the tumor antigens.

3. The use of claim 1, wherein the cytokine gene is selected from the group consisting of interleukin-1, interleukin-2, interleukin-3, interleukin-4, interleukin-5, interleukin-6 and gamma interferon.

4. The use of claim 1, wherein the cytokine gene is interleukin-2.

5. The use of claim 1, wherein cytokine-expressing cells are generated by transferring a cytokine gene into cells by recombinant methods.

6. The use of claim 5, wherein the cytokine gene is present in an expression vector.

7. The use of claim 6, wherein said expression vector additionally contains a suicide gene.

8. The use of claim 5, wherein the cytokine-expressing cells are generated from fibroblasts and antigen-presenting cells.

9. Use of fibroblasts that express IL-2 at a level sufficient to enhance an immune response but low enough to avoid substantial systemic toxicity and that express a tumor antigen for the preparation of a medicament for stimulating an anti-cancer immune response, wherein:
a) fibroblasts being isolated from said patient;
b) said fibroblasts being cultured in vitro;
c) said fibroblasts being transduced with a retroviral expression vector containing the gene coding for IL-2 and a gene coding for a tumor antigen in a retroviral expression vector, to express said tumor antigen and to express and secrete said IL-2 by said fibroblasts; and wherein said medicament is for immunizing said patient at a site other than an active tumor site.

10. The use of claim 9, wherein said fibroblasts are further modified to express a suicide gene.

11. The use of claim 9, wherein said retroviral expression vector has a promoter causing sustained secretion of IL-2.

12. The use of claim 11, wherein said retroviral expression vector causes the secretion of about two nanograms to about 160 nanograms of IL-2 per 10⁶ cells per day.

13. A composition for stimulating an anti-cancer immune response in a patient comprising a tumor antigen and cytokine-expressing cells genetically modified to express an immunostimulatory cytokine gene product, wherein said cytokine-expressing cells are not tumor cells.

14. A composition of claim 13, wherein the cytokine gene is selected from the group consisting of interleukin-1, interleukin-2, interleukin-3, interleukin-4, interleukin-5, interleukin-6, and gamma interferon.

15. The composition of claim 13, wherein the cytokine gene is interleukin-2.

16. The composition of claim 13, wherein the cytokine gene is expressed at a level sufficient to stimulate the immune response but low enough to avoid substantial systemic toxicities.

## Patentansprüche

1. Verwendung einer Formulierung, die ein Tumorantigen und Zytokin-exprimierende Zellen umfaßt, die genetisch modifiziert sind, um ein immunstimulatorisches Zytokin-Genprodukt zu exprimieren, wobei die Zytokin-exprimierenden Zellen keine Tumorzellen sind, zur Herstellung eines Medikaments zur Stimulierung einer anti-Krebs-Immunantwort in einem Patienten, wobei das Medikament zum Immunisieren des Patienten an einer Stelle ist, die nicht die Stelle eines aktiven Tumors ist.

2. Verwendung nach Anspruch 1, bei der Tumorzellen, die zuvor aus dem Patienten isoliert wurden, die Tumorantigene liefern.

3. Verwendung nach Anspruch 1, bei der das Zytokingen aus der Gruppe bestehend aus Interleukin-1, Interleukin-2, Interleukin-3, Interleukin-4, Interleukin-5, Interleukin- 6 und Interferon-γ ausgewählt ist.

4. Verwendung nach Anspruch 1, bei der das Zytokingen Interleukin-2 ist.

5. Verwendung nach Anspruch 1, bei der die Zytokin-exprimierenden Zellen durch Transferieren eines Zytokingens in Zellen mittels rekombinanter Methoden erzeugt sind.

6. Verwendung nach Anspruch 5, bei der das Zytokingen in einem Expressionsvektor vorliegt.

7. Verwendung nach Anspruch 6, bei der der Expressionsvektor zusätzlich ein Suizidgen enthält.

8. Verwendung nach Anspruch 5, bei der die Zytokin-exprimierenden Zellen aus Fibroblasten und Antigen-präsentierenden Zellen erzeugt sind.

9. Verwendung von Fibroblasten, die IL-2 auf einem Niveau exprimieren, das zur Verstärkung einer Immunantwort ausreicht, aber der niedrig genug ist, um eine erhebliche systemische Toxizität zu vermeiden, und die ein Tumorantigen exprimieren, zur Herstellung eines Medikaments zur Stimulierung. einer anti-Krebs-Immunantwort, wobei:
a) Fibroblasten von dem Patienten isoliert werden;
b) die Fibroblasten in vitro kultiviert werden;
c) wobei die Fibroblasten mit einem retroviralen Expressionsvektor, der das für IL-2 kodierende Gen enthält, und einem für ein Tumorgen kodierenden Gen in einem retroviralen Expressionsvektor transduziert sind, um das Tumorgen zu exprimieren und um das IL-2 durch die Fibroblasten zu exprimieren und zu sezonieren;
und wobei das Medikament zum Immunisieren des Patienten an einer Stelle ist, die nicht die Stelle eines aktiven Tumors ist.

10. Verwendung nach Anspruch 9, bei der die Fibroblasten ferner modifiziert sind, um ein Suizidgen zu exprimieren.

11. Verwendung nach Anspruch 9, bei der der retrovirale Expressionsvektor einen Promotor aufweist, der eine lang anhaltende Ausschüttung von IL-2 bewirkt.

12. Verwendung nach Anspruch 11, bei der der retrovirale Expressionsvektor die Ausschüttung von etwa 2 ng bis etwa 160 ng IL-2 pro 10⁶ Zellen/Tag bewirkt.

13. Zusammensetzung zum Stimulieren einer anti-Krebs-Immunantwort in einem Patienten, die ein Tumorantigen und Zytokin-exprimierende Zellen umfaßt, die genetisch modifiziert sind, um ein immunstimulatorisches Zytokin-Genprodukt zu exprimieren, wobei die Zytokin-exprimierenden Zellen keine Tumorzellen sind.

14. Zusammensetzung nach Anspruch 13, wobei das Zytokingen aus der Gruppe bestehend aus Interleukin-1, Interleukin-2, Interleukin-3, Interleukin-4, Interleukin-5, Interleukin- 6 und Interferon-γ ausgewählt ist.

15. Zusammensetzung nach Anspruch 13, wobei das Zytokingen Interleukin-2 ist.

16. Zusammensetzung nach Anspruch 13, wobei das Zytokingen auf einem Niveau exprimiert ist, das zur Stimulierung der Immunantwort ausreicht, aber das niedrig genug ist, um erhebliche systemische Toxizitäten zu vermeiden.

## Revendications

1. Utilisation d'une formulation comprenant un antigène tumoral et des cellules exprimant des cytokines génétiquement modifiées pour exprimer un produit de gène de cytokine immuno-stimulatrice, dans laquelle lesdites cellules exprimant des cytokines ne sont pas des cellules tumorales, pour la préparation d'un médicament destiné à stimuler une réponse immunitaire anticancéreuse chez un patient, ledit médicament étant destiné à immuniser ledit patient en un site autre qu'un site de tumeur active

2. Utilisation selon la revendication 1, dans laquelle des cellules tumorales préalablement isolées à partir dudit patient fournissent les antigènes tumoraux.

3. Utilisation selon la revendication 1, dans laquelle le gène de cytokine est choisi dans l'ensemble consistant en interleukine-1, interleukine-2, interleukine-3, interleukine-4, interleukine-5, interleukine-6, et interféron gamma.

4. Utilisation selon la revendication 1, dans laquelle le gène de cytokine est l'interleukine-2.

5. Utilisation selon la revendication 1, dans laquelle les cellules exprimant des cytokines sont produites en transférant un gène de cytokine dans des cellules par des méthodes de recombinaison.

6. Utilisation selon la revendication 5, dans laquelle le gène de cytokine est présent dans un vecteur d'expression.

7. Utilisation selon la revendication 6, dans laquelle ledit vecteur d'expression contient en outre un gène suicide.

8. Utilisation selon la revendication 5, dans laquelle les cellules exprimant des cytokines sont produites à partir de fibroblastes et de cellules présentatrices d'antigènes.

9. Utilisation de fibroblastes qui expriment IL-2 à un niveau suffisant pour stimuler une réponse immunitaire, mais suffisamment bas pour éviter une toxicité systémique importante et qui expriment un antigène tumoral, pour la préparation d'un médicament destiné à stimuler une réponse immunitaire anticancéreuse, dans laquelle :
a) des fibroblastes sont isolés à partir dudit patient ;
b) lesdits fibroblastes sont cultivés in vitro ;
c) lesdits fibroblastes sont transduits par un vecteur d'expression rétroviral contenant le gène codant pour IL-2 et un gène codant pour un antigène tumoral dans un vecteur d'expression rétroviral, pour exprimer ledit antigène tumoral et pour exprimer et sécréter ladite IL-2 par lesdits fibroblastes ; et dans laquelle ledit médicament est destiné à immuniser ledit patient en un site autre qu'un site de tumeur active

10. Utilisation selon la revendication 9, dans laquelle lesdits fibroblastes sont en outre modifiés pour exprimer un gène suicide.

11. Utilisation selon la revendication 9, dans laquelle ledit vecteur d'expression rétroviral comprend un promoteur entraînant une sécrétion prolongée d'IL-2.

12. Utilisation selon la revendication 11, dans laquelle ledit vecteur d'expression rétroviral entraîne la sécrétion d'environ deux nanogrammes à environ 160 nanogrammes d'IL-2 par 10⁶ cellules par jour.

13. Composition pour la stimulation d'une réponse immunitaire anticancéreuse chez un patient, comprenant un antigène tumoral et des cellules exprimant des cytokines génétiquement modifiées pour exprimer un produit de gène de cytokine immunostimulatrice, dans laquelle lesdites cellules exprimant les cytokines ne sont pas des cellules tumorales.

14. Composition selon la revendication 13, dans laquelle le gène de cytokine est choisi dans l'ensemble consistant en interleukine-1, interleukine-2, interleukine-3, interleukine-4, interleukine-5, interleukine-6, et interféron gamma.

15. Composition selon la revendication 13, dans laquelle le gène de cytokine est l'interleukine-2.

16. Composition selon la revendication 13, dans laquelle le gène de cytokine est exprimé à un niveau suffisant pour stimuler la réponse immunitaire mais suffisamment bas pour éviter des toxicités systémiques importantes.
